# EUROPÄISCHE PATENTSCHRIFT

(11) **EP 2 686 336 B1**
(45) Veröffentlichungstag und Bekanntmachung des Hinweises auf die Patenterteilung: **06.05.2015**
(21) Anmeldenummer: 12708034.9
(22) Anmeldetag: 12.03.2012
(51) Int. Cl.: C07K 5/02, A61P 35/00, A61K 38/00, A61K 47/48

(54) **N-CARBOXYALKYL-AURISTATINE UND IHRE VERWENDUNG**
N-CARBOXYALKYL-AURISTATINS AND USE THEREOF
N-CARBOXYALKYL-AURISTATINES ET LEUR UTILISATION

(30) Priorität: 16.03.2011 EP 11158481
(43) Veröffentlichungstag der Anmeldung: 22.01.2014
(73) Patentinhaber: Seattle Genetics, Inc., Bothell, WA 98021 (US)
(72) Erfinder: LERCHEN, Hans-Georg, 51375 Leverkusen (DE); EL SHEIKH, Sherif, 45219 Essen (DE); STELTE-LUDWIG, Beatrix, 42489 Wülfrath (DE); SCHUHMACHER, Joachim, 42113 Wuppertal (DE); GNOTH, Mark, 40822 Mettmann (DE)
(74) Vertreter: Hoffmann Eitle
(86) Internationale Anmeldenummer: PCT/EP2012/054294
(87) Internationale Veröffentlichungsnummer: WO 2012/123423

(56) Entgegenhaltungen:
- WO-A1-03/008378
- WO-A1-2011/154359
- WO-A2-02/088172
- WO-A2-2005/081711

## Beschreibung

Die vorliegende Anmeldung betrifft neue, am N-Terminus mit einer Carboxyalkyl-Gruppe substituierte Derivate von Monomethylauristatin E und Monomethylauristatin F, Verfahren zur Herstellung dieser Derivate, die Verwendung dieser Derivate zur Behandlung und/oder Prävention von Krankheiten sowie die Verwendung dieser Derivate zur Herstellung von Arzneimitteln zur Behandlung und/oder Prävention von Krankheiten, insbesondere von hyperproliferativen und/oder angiogenen Erkrankungen wie beispielsweise Krebserkrankungen. Solche Behandlungen können als Monotherapie oder auch in Kombination mit anderen Arzneimitteln oder weiteren therapeutischen Maßnahmen erfolgen.

Krebserkrankungen sind die Folge unkontrollierten Zellwachstums verschiedenster Gewebe. In vielen Fällen dringen die neuen Zellen in bestehende Gewebe ein (invasives Wachstum), oder sie metastasieren in entfernte Organe. Krebserkrankungen treten in verschiedensten Organen auf und haben oft gewebespezifische Krankheitsverläufe. Daher beschreibt die Bezeichnung Krebserkrankung als Oberbegriff eine große Gruppe definierter Erkrankungen verschiedener Organe, Gewebe und Zelltypen.

Tumore früher Stadien lassen sich gegebenenfalls durch chirurgische und radiotherapeutische Maßnahmen entfernen. Metastasierte Tumore können im Regelfall durch Chemotherapeutika nur palliativ therapiert werden. Ziel hierbei ist, die optimale Kombination aus einer Verbesserung der Lebensqualität und der Verlängerung der Lebenszeit zu erreichen.

Die meisten der heutzutage parenteral applizierten Chemotherapeutika sind oft nicht zielgerichtet auf das Tumorgewebe oder die Tumorzellen, sondern durch die systemische Gabe unspezifisch im Körper verteilt, d.h. auch an Orten, an denen eine Wirkstoffexposition unerwünscht ist, wie beispielsweise in gesunden Zellen, Geweben und Organen. Dies kann zu unerwünschten Nebenwirkungen bis hin zu gravierenden allgemein-toxischen Effekten führen, die dann den therapeutisch nutzbaren Dosisbereich des Wirkstoffs oftmals stark limitieren oder ein völliges Absetzen der Medikation erfordern.

Die verbesserte und selektive Verfügbarkeit dieser Chemotherapeutika in der Tumorzelle oder dem direkt umgebenden Gewebe und die damit verbundene Wirksteigerung einerseits und Minimierung toxischer Nebeneffekte andererseits steht daher seit mehreren Jahren im Fokus bei der Entwicklung neuer Chemotherapeutika. Viele Versuche wurden bislang unternommen, effiziente Methoden für die Wirkstoffeinbringung in die Zielzelle zu entwickeln. Die Optimierung der Assoziation zwischen Wirkstoff und intrazellulärem Ziel und die Minimierung der interzellulären Wirkstoffverteilung, beispielsweise zu Nachbarzellen, stellen jedoch nach wie vor eine schwierige Aufgabe dar.

Für die zielgerichtete Adressierung von Tumorgewebe und Tumorzellen sind beispielsweise monoklonale Antikörper geeignet. Die Bedeutung solcher Antikörper für die klinische Behandlung von Krebserkrankungen hat allgemein in den letzten Jahren erheblich zugenommen, basierend auf der Wirksamkeit solcher Agentien wie Trastuzumab (Herceptin), Rituximab (Rituxan), Cetuximab (Erbitux) und Bevacizumab (Avastin), welche inzwischen für die Therapie einzelner, spezifischer Tumorerkrankungen zugelassen sind [siehe z.B. G. P. Adams und L. M. Weiner, Nat. Biotechnol. 23, 1147-1157 (2005)]. In Folge hiervon ist auch das Interesse an so genannten Immunokonjugaten deutlich gestiegen, in welchen ein internalisierender, gegen ein Tumor-assoziiertes Antigen gerichteter Antikörper auf kovalente Weise über eine Verknüpfungseinheit ("linker") mit einem cytotoxisch wirkenden Agens verbunden ist, das nach Einschleusung und anschließender Spaltung des Konjugats innerhalb der Tumorzelle freigesetzt wird und dort seine Wirkung direkt und selektiv entfalten kann. Auf diesem Wege könnte die Schädigung von normalem Gewebe im Vergleich zu einer konventionellen Chemotherapie der Krebserkrankung in signifikant engeren Grenzen gehalten werden [siehe z.B. J. M. Lambert, Curr. Opin. Pharmacol. 5, 543-549 (2005); A. M. Wu und P. D. Senter, Nat. Biotechnol. 23, 1137-1146 (2005); P. D. Senter, Curr. Opin. Chem. Biol. 13, 235-244 (2009); L. Ducry und B. Stump, Bioconjugate Chem. 21, 5-13 (2010)].

Statt Antikörpern können auch Liganden aus dem Wirkstoffbereich kleiner Moleküle verwendet werden, die selektiv an einen speziellen Zielort ("target"), wie beispielsweise an einen Rezeptor, binden [siehe z.B. E. Ruoslahti et al., Science 279, 377-380 (1998); D. Karkan et al., PLoS ONE 3 (6), e2469 (June 25, 2008)]. Bekannt sind auch Konjugate aus cytotoxischem Wirkstoff und adressierendem Ligand, die zwischen Ligand und Wirkstoff eine definierte Spaltstelle zur Freisetzung des Wirkstoffs aufweisen. Eine solche "Soll-Bruchstelle" kann beispielsweise in einer Peptidkette bestehen, die an einer bestimmten Stelle selektiv durch ein spezielles Enzym am Wirkort gespalten werden kann [siehe z.B. R. A. Firestone und L. A. Telan, US Patent Application US 2002/0147138].

Auristatin E (AE) und Monomethylauristatin E (MMAE) sind synthetische Analoga der Dolastatine, einer speziellen Gruppe von linearen Pseudopeptiden, welche ursprünglich aus marinen Quellen isoliert wurden und die zum Teil sehr potente cytotoxische Aktivität gegenüber Tumorzellen aufweisen [für eine Übersicht siehe z.B. G. R. Pettit, Prog. Chem. Org. Nat. Prod. 70, 1-79 (1997); G. R. Pettit et al., Anti-Cancer Drug Design 10, 529-544 (1995); G. R. Pettit et al., Anti-Cancer Drug Design 13, 243-277 (1998)]. 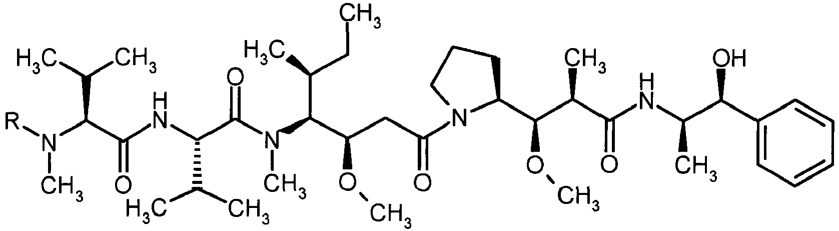 Auristatin E (AE): R = CH₃
Monomethylauristatin E (MMAE): R = H

Allerdings besitzt MMAE den Nachteil einer vergleichsweise hohen systemischen Toxizität. Zudem ist diese Verbindung bei einer Anwendung in Form von Antikörper-Wirkstoff-Konjugaten (Immunokonjugaten) nicht kompatibel mit Verknüpfungseinheiten (Linkern) zwischen Antikörper und Wirkstoff, welche keine enzymatisch spaltbare Soll-Bruchstelle aufweisen [S. O. Doronina et al., Bioconjugate Chem. 17, 114-124 (2006)].

Monomethylauristatin F (MMAF) ist ein Auristatin-Derivat mit einer C-terminalen Phenylalanin-Einheit, das nur moderate anti-proliferative Wirkung im Vergleich zu MMAE aufweist. Dies ist sehr wahrscheinlich auf die freie Carboxylgruppe zurückzuführen, die aufgrund ihrer Polarität und Ladung die Zellgängigkeit dieser Verbindung negativ beeinflusst. In diesem Zusammenhang wurde der Methylester von MMAF (MMAF-OMe) als ein neutral geladenes, zellgängiges Prodrug-Derivat beschrieben, das im Vergleich zu MMAF eine um mehrere Größenordnungen erhöhte *in vitro*-Cytotoxizität gegenüber verschiedenen Karzinoma-Zelllinien zeigt [S. O. Doronina et al., Bioconjugate Chem. 17. 114-124 (2006)]. Es ist anzunehmen, dass diese Wirkung durch MMAF selbst hervorgerufen wird, das nach Aufnahme des Prodrugs in die Zellen schnell durch intrazelluläre Ester-Hydrolyse freigesetzt wird. 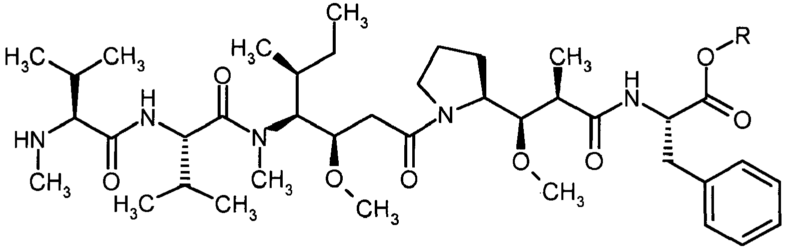 Monomethylauristatin F (MMAF): R = H
Monomethylauristatin F-methylester (MMAF-OMe): R = CH₃

Wirkstoff-Verbindungen auf Basis von einfachen Ester-Derivaten können generell jedoch dem Risiko einer chemischen Instabilität infolge einer unspezifischen, vom vorgesehenen Wirkort unabhängigen Ester-Hydrolyse unterliegen, beispielsweise durch im Blutplasma vorhandene Esterasen; dies kann die Anwendbarkeit solcher Verbindungen in der Therapie deutlich einschränken. Darüber hinaus sind Auristatin-Derivate wie MMAE und MMAF auch Substrate für Transporterproteine, welche von vielen Tumorzellen exprimiert werden, was zu einer Resistenzentwicklung gegenüber diesen Wirkstoffen führen kann.

Aufgabe der vorliegenden Erfindung war es daher, neue Auristatin-Verbindungen zu identifizieren und zur Verwendung für die Behandlung insbesondere von Krebserkrankungen bereitzustellen, die eine stärkere cytotoxische Aktivität in Ganzzell-Assays gegenüber dem nur moderat wirksamen Monomethylauristatin F (MMAF) zeigen und/oder schwächer ausgeprägte Substrat-Eigenschaften für Transporterproteine aufweisen. Solche Substanzen könnten sich in besonderem Maße als Toxophore für die Verknüpfung mit Proteinen, wie beispielsweise Antikörpern, oder auch mit niedermolekularen Liganden zu anti-proliferativ wirkenden (Immuno-)Konjugaten eignen.

Monomethylauristatin F (MMAF) sowie verschiedene Ester- und Amid-Derivate hiervon wurden in WO 2005/081711-A2 offenbart. Weitere Auristatin-Analoga mit einer C-terminalen, amidisch substituierten Phenylalanin-Einheit sind in WO 01/18032-A2 beschrieben. In WO 02/088172-A2 und WO 2007/008603-A1 werden MMAF-Analoga beansprucht, welche Seitenketten-Modifikationen des Phenylalanins betreffen, und in WO 2007/008848-A2 solche, in denen die Carboxylgruppe des Phenylalanins modifiziert ist. Weitere über den N- oder C-Terminus verknüpfte Auristatin-Konjugate sind unter anderem in WO 2004/010957-A2 und WO 2009/117531-A1 beschrieben [siehe auch S. O. Doronina et al., Bioconjugate Chem. 19, 1960-1963 (2008)].

Gegenstand der vorliegenden Erfindung sind nun Verbindungen der allgemeinen Formel (I) 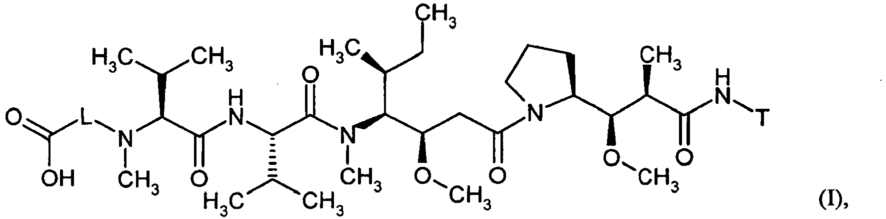 in welcher
- L: für geradkettiges (C₁-C₁₂)-Alkandiyl steht, das bis zu vierfach mit Methyl substituiert sein kann und in dem (*a*) zwei Kohlenstoffatome in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können oder (*b*) bis zu drei zueinander nicht benachbarte CH₂-Gruppen gegen -O- ausgetauscht sein können,
und
- T: für eine Gruppe der Formel 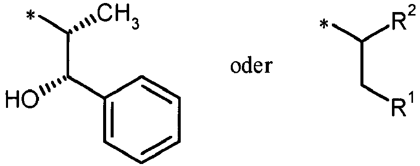 steht, worin
* die Verknüpfungsstelle mit dem Stickstoffatom kennzeichnet,
R¹ Phenyl oder 1*H*-Indol-3-yl bedeutet,
   und
R² Wasserstoff oder eine Gruppe der Formel 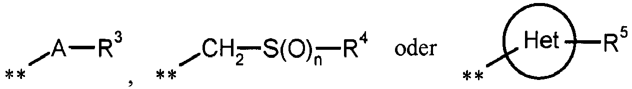 bedeutet, worin
   ** die jeweilige Verknüpfungsstelle mit dem Rest der betreffenden Gruppe T kennzeichnet,
   A geradkettiges (C₁-C₄)-Alkandlyl oder geradkettiges (C₂-C₄)-Alkendiyl darstellt,
   R³ Phenyl, das mit (C₁-C₄)-Alkoxycarbonyl oder Carboxyl substituiert sein kann, darstellt,
   n die Zahl 0, 1 oder 2 darstellt,
   R⁴ Phenyl, Benzyl oder 2-Phenylethyl, welche jeweils in der Phenylgruppe mit (C₁-C₄)-Alkoxycarbonyl oder Carboxyl substituiert sein können, darstellt,
   Het einen divalenten 5-gliedrigen Heteroaryl-Ring mit bis zu drei Ring-Heteroatomen aus der Reihe N, O und/oder S darstellt,
   und
   R⁵ (C₃-C₆)-Cycloalkyl, Phenyl oder (C₁-C₄)-Alkyl, das mit Phenyl substituiert sein kann, darstellt,
   wobei die genannten Phenylgruppen ihrerseits mit (C₁-C₄)-Alkoxycarbonyl oder Carboxyl substituiert sein können,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomeren und Diastereomeren und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfmdungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Benzolsulfonsäure, Toluolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, *N*,*N*-Diisopropylethylamin, Monoethanolamin, Diethanolamin, Trisethanolamin, Dimethylaminoethanol, Diethylaminoethanol, Procain, Dicyclohexylamin, Dibenzylamin, *N*-Methylpiperidin, *N*-Methylmorpholin, Arginin, Lysin und 1,2-Ethylendiamin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Im Rahmen der vorliegenden Erfindung haben die Substituenten, soweit nicht anders spezifiziert, die folgende Bedeutung:
(C₁-C₄)-Alkyl steht im Rahmen der Erfindung für einen geradkettigen oder verzweigten Alkylrest mit 1 bis 4 Kohlenstoffatomen. Beispielhaft und vorzugsweise seien genannt: Methyl, Ethyl, *n*-Propyl, Isopropyl, n-Butyl, Isobutyl, *sec*.-Butyl und *tert*.-Butyl.
(C₁-C₁₂)-Alkandiyl, (C₁-C₈)-Alkandiyl und (C₁-C₆)-Alkandiyl stehen im Rahmen der Erfindung für einen geradkettigen, α,ω-divalenten Alkylrest mit 1 bis 12, 1 bis 8 bzw. 1 bis 6 Kohlenstoffatomen. Bevorzugt ist eine geradkettige Alkandiyl-Gruppe mit 1 bis 8, besonders bevorzugt mit 1 bis 6 Kohlenstoffatomen. Beispielhaft und vorzugsweise seien genannt: Methylen, Ethan-1,2-diyl (1,2-Ethylen), Propan-1,3-diyl (1,3-Propylen), Butan-1,4-diyl (1,4-Butylen), Pentan-1,5-diyl (1,5-Pentylen), Hexan-1,6-diyl (1,6-Hexylen), Heptan-1,7-diyl (1,7-Hexylen), Octan-1,8-diyl (1,8-Octylen), Nonan-1,9-diyl (1,9-Nonylen), Decan-1,10-diyl (1,10-Decylen), Undecan-1,11-diyl (1,11-Undecylen) und Dodecan-1,12-diyl (1,12-Dodecylen).
(C₁-C₄)-Alkandiyl steht im Rahmen der Erfindung für einen geradkettigen, (α,ω-divalenten Alkylrest mit 1 bis 4 Kohlenstoffatomen. Beispielhaft und vorzugsweise seien genannt: Methylen, Ethan-1,2-diyl (1,2-Ethylen), Propan-1,3-diyl (1,3-Propylen) und Butan-1,4-diyl (1,4-Butylen).
(C₂-C₄)-Alkendiyl steht im Rahmen der Erfindung für einen geradkettigen, α,ω-divalenten Alkenylrest mit 2 bis 4 Kohlenstoffatomen und einer Doppelbindung. Beispielhaft und vorzugsweise seien genannt: Ethen-1,2-diyl, Propen-1,3-diyl, But-1-en-1,4-diyl und But-2-en-1,4-diyl. Die Doppelbindung kann hierbei in einer *cis*- oder einer *trans*-Konfiguration vorliegen.
(C₁-C₄)-Alkoxy steht im Rahmen der Erfindung für einen geradkettigen oder verzweigten Alkoxyrest mit 1 bis 4 Kohlenstoffatomen. Beispielhaft und vorzugsweise seien genannt: Methoxy, Ethoxy, *n-*Propoxy, Isopropoxy, n-Butoxy und *tert*.-Butoxy.
(C₁-C₄)-Alkoxycarbonyl steht im Rahmen der Erfindung für einen geradkettigen oder verzweigten Alkoxyrest mit 1 bis 4 Kohlenstoffatomen, der über eine an das Sauerstoffatom gebundene Carbonyl-Gruppe [-C(=O)-] verknüpft ist. Beispielhaft und vorzugsweise seien genannt: Methoxycarbonyl, Ethoxycarbonyl, *n*-Propoxycarbonyl, Isopropoxycarbonyl, *n*-Butoxycarbonyl und *tert.-*Butoxycarbonyl.
(C₃-C₆)-Cycloalkyl steht im Rahmen der Erfindung für eine monocyclische, gesättigte Cycloalkylgruppe mit 3 bis 6 Kohlenstoffatomen. Beispielhaft und vorzugsweise seien genannt: Cyclopropyl, Cyclobutyl, Cyclopentyl und Cyclohexyl.
5-gliedriges Heteroaryl in der Definition des Ringes Het steht für einen divalenten aromatischen Heterocyclus (Heteroaromaten) mit insgesamt 5 Ringatomen, der bis zu drei gleiche oder verschiedene Ring-Heteroatome aus der Reihe N, O und/oder S enthält und über zwei Ring-Kohlenstoffatome oder gegebenenfalls ein Ring-Stickstoffatom und ein Ring-Kohlenstoffatom verknüpft ist. Beispielhaft seien genannt: Furyl, Pyrrolyl, Thienyl, Pyrazolyl, Imidazolyl, 1,2-Oxazolyl, 1,3-Oxazolyl, 1,2-Thiazolyl, 1,3-Thiazolyl, 1,2,3-Triazolyl, 1,2,4-Triazolyl, 1,2,4-Oxadiazolyl, 1,3,4-Oxadiazolyl, 1,2,4-Thiadiazolyl und 1,3,4-Thiadiazolyl. Bevorzugt ist 5-gliedriges Heteroaryl mit zwei oder drei gleichen oder verschiedenen Ring-Heteroatomen aus der Reihe N, O und/oder S, wie insbesondere Pyrazolyl, Imidazolyl, 1,3-Oxazolyl, 1,3-Thiazolyl, 1,2,4-Oxadiazolyl und 1,3,4-Oxadiazolyl.

Im Rahmen der vorliegenden Erfindung gilt, dass für alle Reste, die mehrfach auftreten, deren Bedeutung unabhängig voneinander ist. Wenn Reste in den erfindungsgemäßen Verbindungen substituiert sind, können die Reste, soweit nicht anders spezifiziert, ein- oder mehrfach substituiert sein. Eine Substitution mit einem oder mit zwei gleichen oder verschiedenen Substituenten ist bevorzugt. Besonders bevorzugt ist die Substitution mit einem Substituenten.

### Bevorzugt im Rahmen der vorliegenden Erfindung sind Verbindungen der Formel (I), in welcher

- L: für geradkettiges (C₁-C₈)-Alkandiyl steht, in dem (a) zwei Kohlenstoffatome in 1,3- oder 1,4-Relation zueinander unter Einbezug des einen beziehungsweise der beiden zwischen ihnen liegenden Kohlenstoffatome zu einem Phenyl-Ring verbrückt sein können oder (b) bis zu zwei zueinander nicht benachbarte CH₂-Gruppen gegen -O- ausgetauscht sein können,
und
- T: für eine Gruppe der Formel 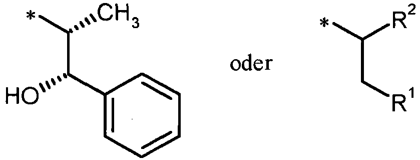 steht, worin
* die Verknüpfungsstelle mit dem Stickstoffatom kennzeichnet,
R¹ Phenyl oder 1*H*-Indol-3-yl bedeutet,
   und
R² Wasserstoff oder eine Gruppe der Formel 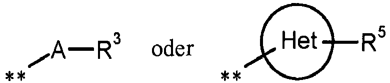 bedeutet, worin
   ** die Verknüpfungsstelle mit dem Rest der betreffenden Gruppe T kennzeichnet,
   A Ethen-1,2-diyl oder Propen-1,3-dlyl darstellt,
   R³ Phenyl, das mit (C₁-C₄)-Alkoxycarbonyl oder Carboxyl substituiert sein kann, darstellt,
   Het einen divalenten 5-gliedrigen Heteroaryl-Ring ausgewählt aus der Reihe Pyrazolyl, Imidazolyl, 1,3-Oxazolyl, 1,3-Thiazolyl, 1,2,4-Oxadiazolyl und 1,3,4-Oxadiazolyl darstellt,
      und
   R⁵ Phenyl, das mit (C₁-C₄)-Alkoxycarbonyl oder Carboxyl substituiert sein kann, darstellt,
sowie ihre Salze, Solvate und Solvate der Salze.

Besonders bevorzugt im Rahmen der vorliegenden Erfindung sind Verbindungen der Formel (I), in welcher
- L: für geradkettiges (C₁-C₆)-Alkandiyl steht,
und
- T: für eine Gruppe der Formel 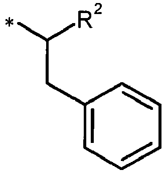 steht, worin
* die Verknüpfungsstelle mit dem Stickstoffatom kennzeichnet
   und
R² Wasserstoff oder eine Gruppe der Formel 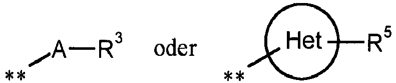 bedeutet, worin
   ** die Verknüpfungsstelle mit dem Rest der betreffenden Gruppe T kennzeichnet,
   A Ethen-1,2-diyl darstellt,
   R³ Phenyl, das mit Methoxycarbonyl oder Carboxyl substituiert sein kann, darstellt,
   Het 1,3,4-Oxadiazol-2,5-diyl darstellt,
      und
   R⁵ Phenyl, das mit Methoxycarbonyl oder Carboxyl substituiert sein kann, darstellt,
sowie ihre Salze, Solvate und Solvate der Salze.

Von besonderer Bedeutung im Rahmen der vorliegenden Erfindung sind Verbindungen der Formel (I), in welcher
- L: für Propan-1,3-diyl steht,
sowie ihre Salze, Solvate und Solvate der Salze.

Von besonderer Bedeutung im Rahmen der vorliegenden Erfindung sind auch Verbindungen der Formel (I), in welcher
- T: für eine Gruppe der Formel 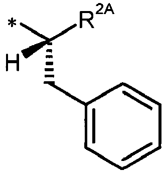 steht, worin
* die Verknüpfungsstelle mit dem Stickstoffatom kennzeichnet
und
R^{2A} die oben definierten Bedeutungen von R² hat, jedoch nicht für Wasserstoff steht,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt. Ganz besonders bevorzugt sind Kombinationen von zweien oder mehreren der oben genannten Vorzugsbereiche. Weiterer Gegenstand der vorliegenden Erfindung ist ein Verfahren zur Herstellung der erfindungsgemäßen Verbindungen der Formel (I), dadurch gekennzeichnet, dass man eine Verbindung der Formel (II) 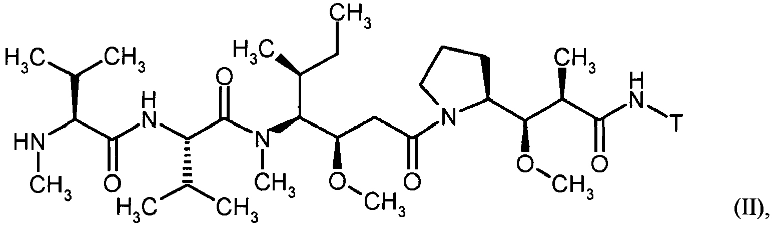 in welcher T die oben angegebenen Bedeutungen hat,
in einem inerten Lösungsmittel entweder
- [A]: durch baseninduzierte Alkylierung mit einer Verbindung der Formel (III) 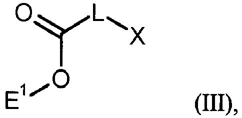 in welcher L die oben angegebene Bedeutung hat,
E¹ für Wasserstoff, (C₁-C₄)-Alkyl oder Benzyl steht,
und
X für eine Fluchtgruppe wie beispielsweise Chlorid, Bromid, Iodid, Mesylat, Triflat oder Tosylat steht,
zu einer Verbindung der Formel (IV) 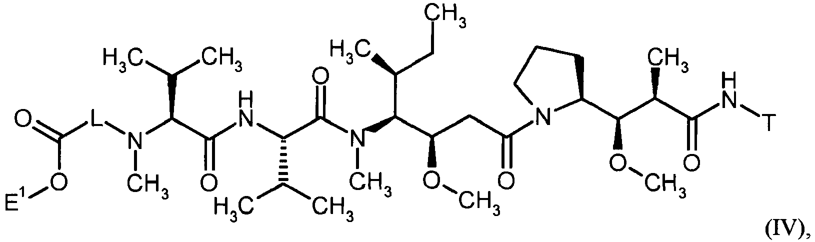 in welcher E¹, L und T die oben angegebenen Bedeutungen haben,
umsetzt und anschließend im Fall, dass E¹ für (C₁-C₄)-Alkyl oder Benzyl steht, diesen Ester-Rest nach üblichen Methoden abspaltet, so dass ebenso wie im Fall, dass E¹ in (III) für Wasserstoff steht, die erfindungsgemäße Carbonsäure der Formel (I) 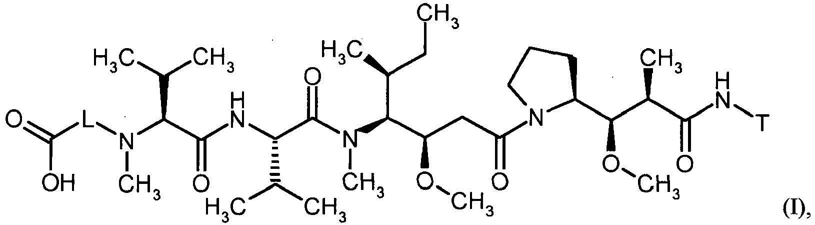 in welcher L und T die oben angegebenen Bedeutungen haben,
erhalten wird, oder
- [B]: durch Umsetzung mit einer Verbindung der Formel (V) 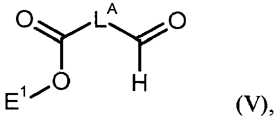 in welcher
E¹ für Wasserstoff, (C₁-C₄)-Alkyl oder Benzyl steht,
und
L^{A} die oben definierte Bedeutung von L hat, jedoch in der Alkyl-Kettenlänge um eine CH₂-Einheit verkürzt ist,
in Gegenwart eines geeigneten Reduktionsmittels in eine Verbindung der Formel (VI) 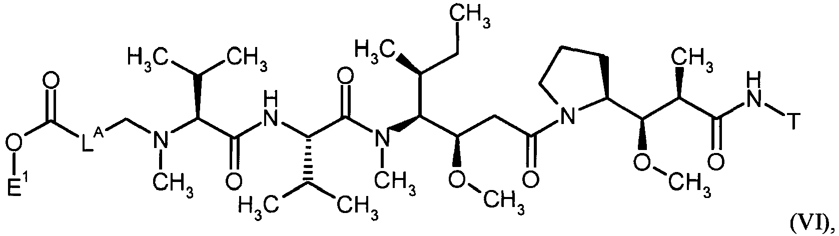 in welcher E¹, L^{A} und T die oben angegebenen Bedeutungen haben,
überführt und anschließend im Fall, dass E¹ für (C₁-C₄)-Alkyl oder Benzyl steht, diesen Ester-Rest nach üblichen Methoden abspaltet, so dass ebenso wie im Fall, dass E¹ in (V) für Wasserstoff steht, die erfindungsgemäße Carbonsäure der Formel (I-A) 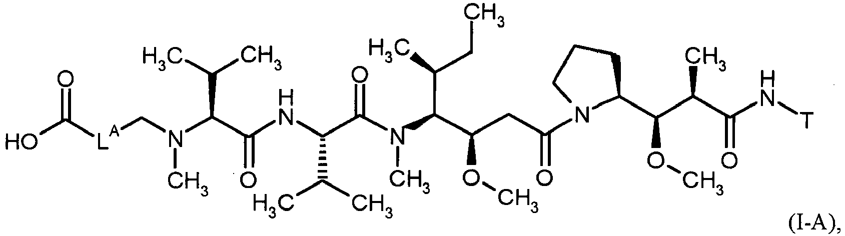 in welcher L^{A} und T die oben angegebenen Bedeutungen haben,
erhalten wird,
und die resultierenden Verbindungen der Formel (I) bzw. (I-A) gegebenenfalls in ihre Enantiomere und/oder Diastereomere trennt und/oder mit den entsprechenden (i) Lösungsmitteln und/oder (ii) Basen oder Säuren zu ihren Solvaten, Salzen und/oder Solvaten der Salze umsetzt.

Als inerte Lösungsmittel für die Umsetzung (II) + (III) → (IV) eignen sich beispielsweise Ether wie Diethylether, Diisopropylether, Methyl-*tert*.-butylether, Tetrahydrofuran, 1,4-Dioxan, 1,2-Dimethoxyethan oder Bis-(2-methoxyethyl)-ether, Kohlenwasserstoffe wie Benzol, Toluol, Xylol, Pentan, Hexan, Heptan, Cyclohexan oder Erdölfraktionen, oder dipolar-aprotische Lösungsmittel wie Aceton, Methylethylketon, Acetonitril, Dimethylsulfoxid (DMSO), *N*,*N*-Dimethylformamid (DMF), *N*,*N*-Dimethylacetamid (DMA), *N*,*N*'-Dimethylpropylenharnstoff (DMPU), *N*-Methylpyrrolidinon (NMP) oder Pyridin. Ebenso ist es möglich, Gemische solcher Lösungsmittel einzusetzen. Bevorzugt wird Aceton oder *N*,*N*-Dimethylformamid verwendet.

Geeignete Basen für diese Alkylierungsreaktion sind insbesondere Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat, oder übliche organische Amine wie Triethylamin, *N*-Methylmorpholin, *N*-Methylpiperidin, *N,N-*Diisopropylethylamin, Pyridin oder 4-*N*,*N-*Dimethylaminopyridin. Bevorzugt wird Kalium- oder Cäsiumcarbonat verwendet. Gegebenenfalls ist der Zusatz eines Alkylierungskatalysators von Vorteil, wie beispielsweise Lithiumbromid oder -iodid, Natrium- oder Kaliumiodid, Tetra-*n*-butylammoniumbromid oder -iodid oder Benzyltriethylammoniumbromid.

Die Reaktion (II) + (III) → (IV) wird im Allgemeinen in einem Temperaturbereich von -20°C bis +100°C, bevorzugt bei 0°C bis +50°C durchgeführt. Die Umsetzung kann bei normalem, bei erhöhtem oder bei erniedrigtem Druck erfolgen (z.B. von 0.5 bis 5 bar); in der Regel arbeitet man bei Normaldruck.

Die Umsetzung (II) + (V) → (VI) erfolgt in den für eine reduktive Aminierung üblichen, unter den Reaktionsbedingungen inerten Lösungsmitteln, gegebenenfalls in Gegenwart einer Säure und/ oder eines wasserentziehenden Mittels als Katalysator. Zu solchen Lösungsmitteln gehören beispielsweise Alkohole wie Methanol, Ethanol, n-Propanol, Isopropanol, n-Butanol oder *tert*.-Butanol, Ether wie Tetrahydrofuran, 1,4-Dioxan, 1,2-Dimethoxyethan oder Bis-(2-methoxyethyl)-ether, oder andere Solventien wie Dichlormethan, 1,2-Dichlorethan, *N,N-*Dimethylformamid oder auch Wasser. Ebenso ist es möglich, Gemische dieser Lösungsmittel einzusetzen. Bevorzugt wird als Lösungsmittel ein 1,4-Dioxan/Wasser-Gemisch verwendet unter Zusatz von Essigsäure oder verdünnter Salzsäure als Katalysator.

Als Reduktionsmittel eignen sich für diese Reaktion insbesondere komplexe Borhydride, wie beispielsweise Natriumborhydrid, Natriumcyanoborhydrid, Natriumtriacetoxyborhydrid oder Tetran-butylammoniumborhydrid. Bevorzugt wird Natriumcyanoborhydrid eingesetzt.

Die Umsetzung (II) + (V) → (VI) erfolgt im Allgemeinen in einem Temperaturbereich von 0°C bis +120°C, bevorzugt bei +50°C bis +100°C. Die Reaktion kann bei normalem, bei erhöhtem oder bei erniedrigtem Druck durchgeführt werden (z.B. von 0.5 bis 5 bar); in der Regel arbeitet man bei Normaldruck.

Die Abspaltung eines Ester-Restes E¹ in den Verfahrensschritten (IV) → (I) und (VI) → (I-A) [E¹ = (C₁-C₄)-Alkyl oder Benzyl] wird nach üblichen Methoden durchgeführt, indem man den Ester in einem inerten Lösungsmittel mit einer Säure oder einer Base behandelt, wobei bei letzterer Variante das zunächst entstehende Carboxylat-Salz durch nachfolgende Zugabe einer Säure in die freie Carbonsäure überführt wird. Im Falle eines *tert*.-Butylesters geschieht die Spaltung bevorzugt mittels einer Säure. Bei einem Benzylester kann die Abspaltung auch durch Hydrogenolyse in Gegenwart eines geeigneten Palladium-Katalysators, wie beispielsweise Palladium auf Aktivkohle, erfolgen.

Der aus Verbindung (III) bzw. (V) stammende Ester-Rest E¹ wird hierbei so gewählt, dass die Bedingungen seiner Abspaltung kompatibel mit der jeweiligen Gruppe T in Verbindung (IV) und (VI) sind.

Als Base für die Ester-Hydrolyse sind die üblichen anorganischen Basen geeignet. Hierzu gehören insbesondere Alkali- oder Erdalkalihydroxide wie beispielsweise Lithium-, Natrium-, Kalium- oder Bariumhydroxid, oder Alkali- oder Erdalkalicarbonate wie Natrium-, Kalium- oder Calciumcarbonat. Bevorzugt sind Lithium-, Natrium- oder Kaliumhydroxid.

Als Säure eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle eines *tert*.-Butylesters und Salzsäure bei einem Methylester.

Als inerte Lösungsmittel eignen sich für diese Reaktionen Wasser oder die für eine Esterspaltung üblichen organischen Lösungsmittel. Hierzu gehören bevorzugt niedere Alkohole wie Methanol, Ethanol, n-Propanol oder Isopropanol, Ether wie Diethylether, Tetrahydrofuran, 1,4-Dioxan oder 1,2-Dimethoxyethan, oder andere Lösungsmittel wie Dichlormethan, Aceton, Methylethylketon, *N,N-*Dimethylformamid oder Dimethylsulfoxid. Ebenso ist es möglich, Gemische dieser Lösungsmittel einzusetzen. Im Falle einer basischen Ester-Hydrolyse werden bevorzugt Gemische von Wasser mit 1,4-Dioxan, Tetrahydrofuran, Methanol, Ethanol und/oder Dimethylformamid verwendet. Im Falle der Umsetzung mit Trifluoressigsäure wird bevorzugt Dichlormethan und im Falle der Umsetzung mit Chlorwasserstoff bevorzugt Tetrahydrofuran, Diethylether, 1,4-Dioxan oder Wasser eingesetzt.

Die Esterspaltung erfolgt im Allgemeinen in einem Temperaturbereich von -20°C bis +100°C, bevorzugt bei 0°C bis +50°C.

Die Verbindungen der Formel (II) können nach üblichen Methoden der Peptidchemie beispielsweise dadurch hergestellt werden, dass man eine Verbindung der Formel (VII) 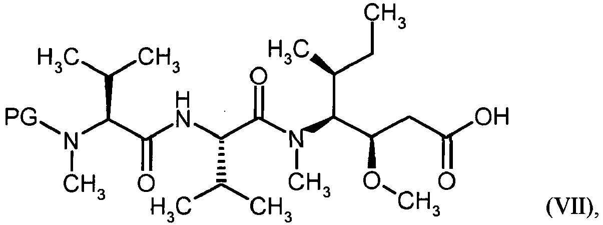 in welcher
- PG: für eine Amino-Schutzgruppe wie beispielsweise (9*H*-Fluoren-9-ylmethoxy)carbonyl, *tert.-*Butoxycarbonyl oder Benzyloxycarbonyl steht,
in einem inerten Lösungsmittel unter Aktivierung der Carboxyl-Funktion in (VII) entweder
- [C]: zunächst mit einer Verbindung der Formel (VIII) 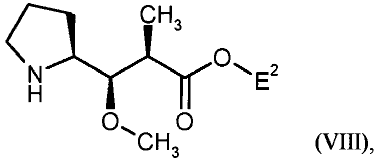 in welcher
E² für Wasserstoff, (C₁-C₄)-Alkyl oder Benzyl steht,
oder einem Salz dieser Verbindung zu einer Verbindung der Formel (IX) 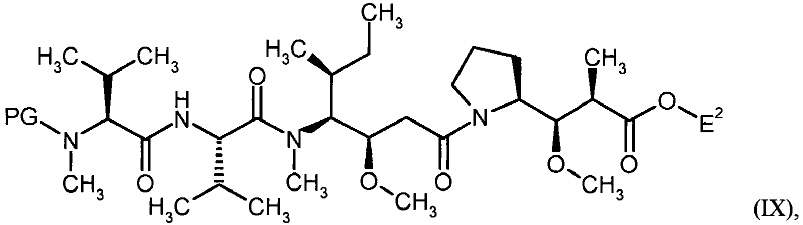 in welcher E² und PG die oben angegebenen Bedeutungen haben,
kuppelt, anschließend im Fall, dass E² für (C₁-C₄)-Alkyl oder Benzyl steht, diesen Ester-Rest nach üblichen Methoden abspaltet und die resultierende Carbonsäure der Formel (X) 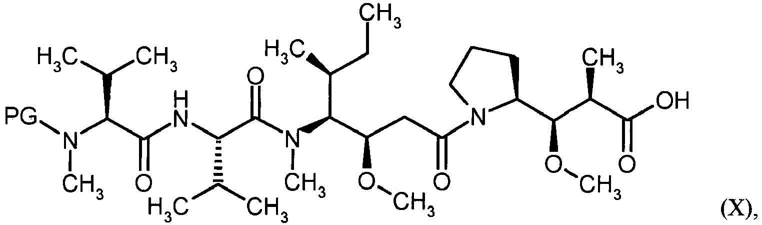 in welcher PG die oben angegebene Bedeutung hat,
dann in einem inerten Lösungsmittel unter Aktivierung der Carboxyl-Funktion mit einer Verbindung der Formel (XI)

H₂N-T (XI),

in welcher T die oben angegebenen Bedeutungen hat,
oder einem Salz dieser Verbindung zu einer Verbindung der Formel (XII) 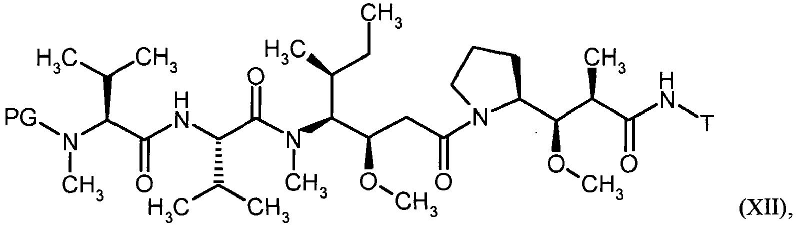 in welcher PG und T die oben angegebenen Bedeutungen haben,
kuppelt oder
- [D]: mit einer Verbindung der Formel (XIII) 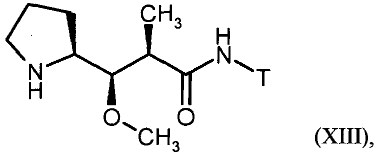 in welcher T die oben angegebenen Bedeutungen hat,
oder einem Salz dieser Verbindung gleichfalls zu der Verbindung der Formel (XII) 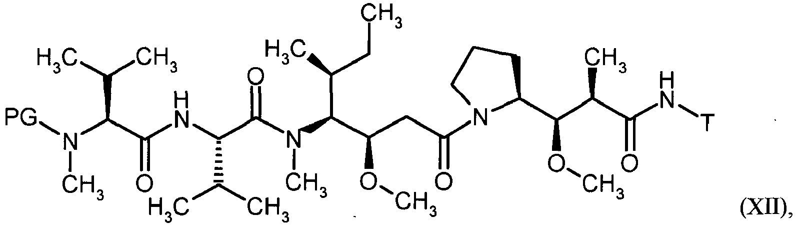 in welcher PG und T die oben angegebenen Bedeutungen haben,
kuppelt
und die Verbindung der Formel (XII) dann auf übliche Weise zu einer Verbindung der Formel (II) 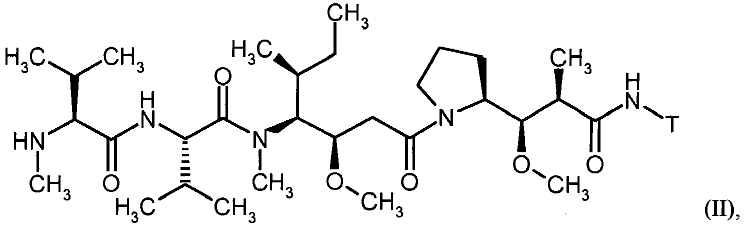 in welcher T die oben angegebenen Bedeutungen hat,
entschützt.

Die oben beschriebenen Kupplungsreaktionen (Amid-Bildung aus jeweiliger Amin- und Carbonsäure-Komponente) werden nach allgemein üblichen Methoden der Peptidchemie durchgeführt [siehe z.B. M. Bodanszky, Principles of Peptide Synthesis, Springer-Verlag, Berlin, 1993; M. Bodanszky und A. Bodanszky, The Practice of Peptide Synthesis, Springer-Verlag, Berlin, 1984; H.-D. Jakubke und H. Jeschkeit, Aminosäuren, Peptide, Proteine, Verlag Chemie, Weinheim, 1982].

Inerte Lösungsmittel für die Kupplungsreaktionen (VII) + (VIII) → (IX), (X) + (XI) → (XII) und (VII) + (XIII) → (XII) sind beispielsweise Ether wie Diethylether, Diisopropylether, *tert*.-Butyl-methylether, Tetrahydrofuran, 1,4-Dioxan, 1,2-Dimethoxyethan oder Bis-(2-methoxyethyl)-ether, Kohlenwasserstoffe wie Benzol, Toluol, Xylol, Pentan, Hexan, Heptan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder dipolar-aprotische Lösungsmittel wie Aceton, Methylethylketon, Acetonitril, Ethylacetat, Pyridin, Dimethylsulfoxid (DMSO), *N*,*N*-Dimethylformamid (DMF), *N*,*N*-Dimethylacetamid (DMA), *N*,*N*'-Dimethylpropylenharnstoff (DMPU) oder *N-*Methylpyrrolidinon (NMP). Ebenso ist es möglich, Gemische solcher Lösungsmittel zu verwenden. Bevorzugt wird *N*,*N*-Dimethylformamid eingesetzt.

Als Aktivierungs-/Kondensationsmittel für diese Kupplungen eignen sich beispielsweise Carbodiimide wie *N*,*N*'-Diethyl-, *N*,*N*'-Dipropyl-, *N*,*N*'-Diisopropyl-, *N*,*N*'-Dicyclohexylcarbodiimid (DCC) oder *N*-(3-Dimethylaminopropyl)-*N*'-ethylcarbodiimid-Hydrochlorid (EDC), Phosgen-Derivate wie *N*,*N*'-Carbonyldiimidazol (CDI) oder Isobutylchlorformiat, 1,2-Oxazolium-Verbindungen wie 2-Ethyl-5-phenyl-1,2-oxazolium-3-sulfat oder 2-*tert*.-Butyl-5-methylisoxazolium-perchlorat, Acylamino-Verbindungen wie 2-Ethoxy-1-ethoxycarbonyl-1,2-dihydrochinolin, α-Chlorenamine wie 1-Chlor-2-methyl-1-dimethylamino-1-propen, Phosphor-Verbindungen wie Propanphosphonsäureanhydrid, Cyanophosphonsäurediethylester, Bis-(2-oxo-3-oxazolidinyl)-phosphorylchlorid, Benzotriazol-1-yloxy-tris(dimethylamino)phosphonium-hexafluorophosphat oder Benzotriazol-1-yloxy-tris-(pyrrolidino)phosphonium-hexafluorophosphat (PyBOP), oder Uronium-Verbindungen wie *O*-(Benzotriazol-1-yl)-*N*,*N*,*N*',*N*'-tetramethyluronium-tetrafluoroborat (TBTU), *O*-(Benzotriazol-1-yl)-*N*,*N*,*N*',*N*'-tetramethyluronium-hexafluorophosphat (HBTU), 2-(2-Oxo-1-(2*H*)-pyridyl)-1,1,3,3-tetramethyluronium-tetrafluoroborat (TPTU), *O*-(7-Azabenzotriazol-1-yl)-*N*,*N*,*N*',*N*'-tetramethyluronium-hexafluorophosphat (HATU) oder *O*-(1*H*-6-Chlorbenzotriazol-1-yl)-1,1,3,3-tetramethyluronium-tetrafluoroborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat, oder tertiäre Aminbasen wie Triethylamin, *N*-Methylmorpholin, *N*-Methylpiperidin, *N,N-*Diisopropylethylamin, Pyridin oder 4-*N*,*N*-Dimethylaminopyridin.

Im Rahmen der vorliegenden Erfindung wird als Aktivierungs-/Kondensationsmittel für solche Kupplungsreaktionen bevorzugt *N*-(3-Dimethylaminopropyl)-*N*'-ethylcarbodiimid-Hydrochlorid (EDC) in Kombination mit 1-Hydroxybenzotriazol (HOBt) und *N*,*N*-Diisopropylethylamin, oder *O-*(7-Azabenzotriazol-1-yl)-*N*,*N*,*N*',*N*'-tetramethyluronium-hexafluorophosphat (HATU) gleichfalls in Verbindung mit *N*,*N*-Diisopropylethylamin verwendet.

Die Kupplungsreaktionen (VII) + (VIII) → (IX), (X) + (XI) → (XII) und (VII) + (XIII) → (XII) werden in der Regel in einem Temperaturbereich von -20°C bis +60°C, bevorzugt bei 0°C bis +40°C durchgeführt. Die Umsetzungen können bei normalem, bei erhöhtem oder bei erniedrigtem Druck erfolgen (z.B. von 0.5 bis 5 bar); im Allgemeinen arbeitet man bei Normaldruck.

Gegebenenfalls vorhandene funktionelle Gruppen - wie insbesondere Amino-, Hydroxy- und Carboxylgruppen - können bei den zuvor beschriebenen Verfahrensschritten, falls zweckmäßig oder erforderlich, auch in temporär geschützter Form vorliegen. Die Einführung und Entfernung solcher Schutzgruppen erfolgt hierbei nach üblichen, aus der Peptidchemie bekannten Methoden [siehe z.B. T.W. Greene und P.G.M. Wuts, Protective Groups in Organic Synthesis, Wiley, New York, 1999; M. Bodanszky und A. Bodanszky, The Practice of Peptide Synthesis, Springer-Verlag, Berlin, 1984]. Bei Vorhandensein mehrerer geschützter Gruppen kann deren Wiederfreisetzung gegebenenfalls simultan in einer Eintopf-Reaktion oder auch in separaten Reaktionsschritten vorgenommen werden.

Als Amino-Schutzgruppe wird bevorzugt *tert*.-Butoxycarbonyl (Boc), Benzyloxycarbonyl (Z) oder (9H-Fluoren-9-ylmethoxy)carbonyl (Fmoc) verwendet; für eine Hydroxy- oder Carboxyl-Funktion wird vorzugsweise *tert*.-Butyl oder Benzyl als Schutzgruppe eingesetzt. Die Abspaltung einer *tert.-*Butyl- oder *tert*.-Butoxycarbonyl-Gruppe geschieht üblicherweise durch Behandlung mit einer starken Säure, wie Chlorwasserstoff, Bromwasserstoff oder Trifluoressigsäure, in einem inerten Lösungsmittel wie Diethylether, 1,4-Dioxan, Dichlormethan oder Essigsäure; gegebenenfalls kann diese Reaktion auch ohne Zusatz eines inerten Lösungsmittels durchgeführt werden. Im Falle von Benzyl oder Benzyloxycarbonyl als Schutzgruppe werden diese bevorzugt durch Hydrogenolyse in Gegenwart eines geeigneten Palladium-Katalysators, wie beispielsweise Palladium auf Aktivkohle, entfernt. Die (9*H*-Fluoren-9-ylmethoxy)carbonyl-Gruppe wird im Allgemeinen mit Hilfe einer sekundären Aminbase wie Diethylamin oder Piperidin abgespalten.

Ein Ester-Rest E² in Verbindung (VIII) [E² = (C₁-C₄)-Alkyl oder Benzyl] wird hierbei so gewählt, dass die Bedingungen seiner Abspaltung kompatibel mit der jeweils eingesetzten Schutzgruppe PG aus Verbindung (VII) sind.

Die Verbindungen der Formel (VII) können auf analoge Weise beispielsweise dadurch hergestellt werden, dass man zunächst *N*-(Benzyloxycarbonyl)-L-Valin der Formel (XIV) 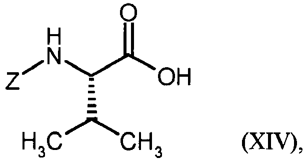 in welcher Z für die Benzyloxycarbonyl-Schutzgruppe steht,
mit Hilfe eines Kondensationsmittels mit einer Verbindung der Formel (XV) 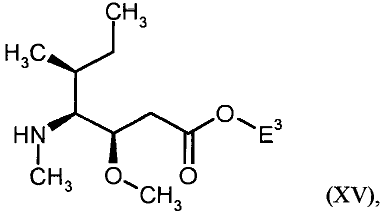 in welcher E³ für (C₁-C₄)-Alkyl steht,
oder einem Salz dieser Verbindung zu einer Verbindung der Formel (XVI) 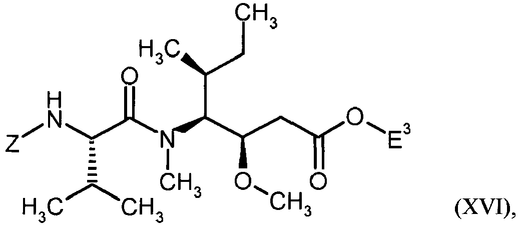 in welcher E³ und Z die oben angegebenen Bedeutungen haben,
kuppelt, diese nach hydrogenolytischer Entfernung der Z-Schutzgruppe dann in Gegenwart eines Kondensationsmittels mit *N*-geschütztem *N*-Methyl-L-valin der Formel (XVII) 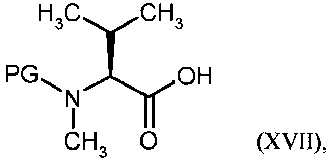 in welcher
- PG: für eine Amino-Schutzgruppe wie beispielsweise (9*H*-Fluoren-9-ylmethoxy)carbonyl, *tert.-*Butoxycarbonyl oder Benzyloxycarbonyl steht,
zu einer Verbindung der Formel (XVIII) 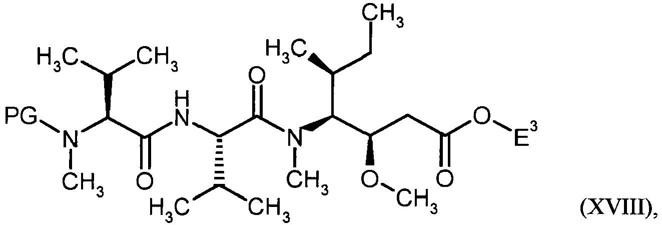 in welcher E³ und PG die oben angegebenen Bedeutungen haben,
kuppelt und abschließend die Ester-Gruppierung -C(O)O-E³ in (XVIII) nach üblichen Methoden in die freie Carbonsäure (VII) überführt.

Die Kupplungen (XIV) + (XV) → (XVI) und Z-entschütztes (XVI) + (XVII) → (XVIII) werden unter analogen Reaktionsbedingungen durchgeführt wie zuvor bei den in Verfahren [C] und [D] dargestellten Kupplungsschritten beschrieben.

Die Hydrolyse der Ester-Gruppe -C(O)O-E³ im Reaktionsschritt (XVIII) → (VII) erfolgt auf analoge Weise wie zuvor im Rahmen der Verfahrenssequenzen [A] und [B] für den Ester-Rest E¹ beschrieben. Die Alkylgruppe E³ in Verbindung (XV) wird hierbei so gewählt, dass die Bedingungen ihrer Abspaltung kompatibel mit der jeweils eingesetzten Schutzgruppe PG aus Verbindung (XVII) sind.

Die Verbindungen der Formel (XIII) ihrerseits sind durch Kupplung der oben beschriebenen Verbindung (XI) mit der Verbindung (XIX) 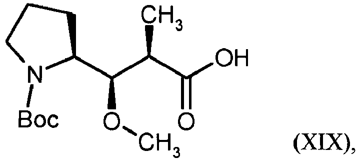 in welcher Boc für die *tert*.-Butoxycarbonyl-Schutzgruppe steht,
zu einer Verbindung der Formel (XX) 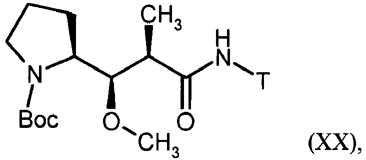 in welcher Boc und T die oben angegebenen Bedeutungen haben,
und nachfolgende Abspaltung der Boc-Schutzgruppe zugänglich.

Die Kupplungsreaktion (XI) + (XIX) → (XX) wird wiederum unter analogen Bedingungen durchgeführt wie zuvor bei den in Verfahren [C] und [D] dargestellten Kupplungsschritten beschrieben.

Die Verbindungen der Formeln (III), (V), (VIII), (XI), (XIV), (XV), (XVII) und (XIX) einschließlich, wo zutreffend, chiraler oder diastereomerer Formen hiervon sind kommerziell erhältlich oder als solche in der Literatur beschrieben, oder sie können auf für den Fachmann offenkundigem Wege in Analogie zu in der Literatur publizierten Methoden hergestellt werden. Zahlreiche ausführliche Vorschriften sowie Literaturangaben zur Herstellung der Ausgangsmaterialien befinden sich auch im Experimentellen Teil im Abschnitt zur Herstellung der Ausgangsverbindungen und Intermediate.

Sollten entsprechende isomerenreine Ausgangsmaterialien nicht zur Verfügung stehen, so kann eine Trennung der erfindungsgemäßen Verbindungen in die korrespondierenden Enantiomere und/ oder Diastereomere zweckmäßigerweise auch bereits auf der Stufe der Verbindungen (II), (IV), (VI), (XI), (XII), (XIII) und (XX) erfolgen, welche dann in separierter Form gemäß den zuvor beschriebenen Reaktionsschritten weiter umgesetzt werden. Eine solche Auftrennung der Stereoisomeren läßt sich nach üblichen, dem Fachmann bekannten Methoden durchführen. Bevorzugt werden chromatographische Verfahren an achiralen bzw. chiralen Trennphasen angewandt; im Falle von freien Carbonsäuren als Zwischenprodukten kann alternativ auch eine Trennung über diastereomere Salze mit Hilfe chiraler Basen erfolgen.

Die Herstellung der erfindungsgemäßen Verbindungen kann durch die folgenden Reaktionsschemata beispielhaft veranschaulicht werden:

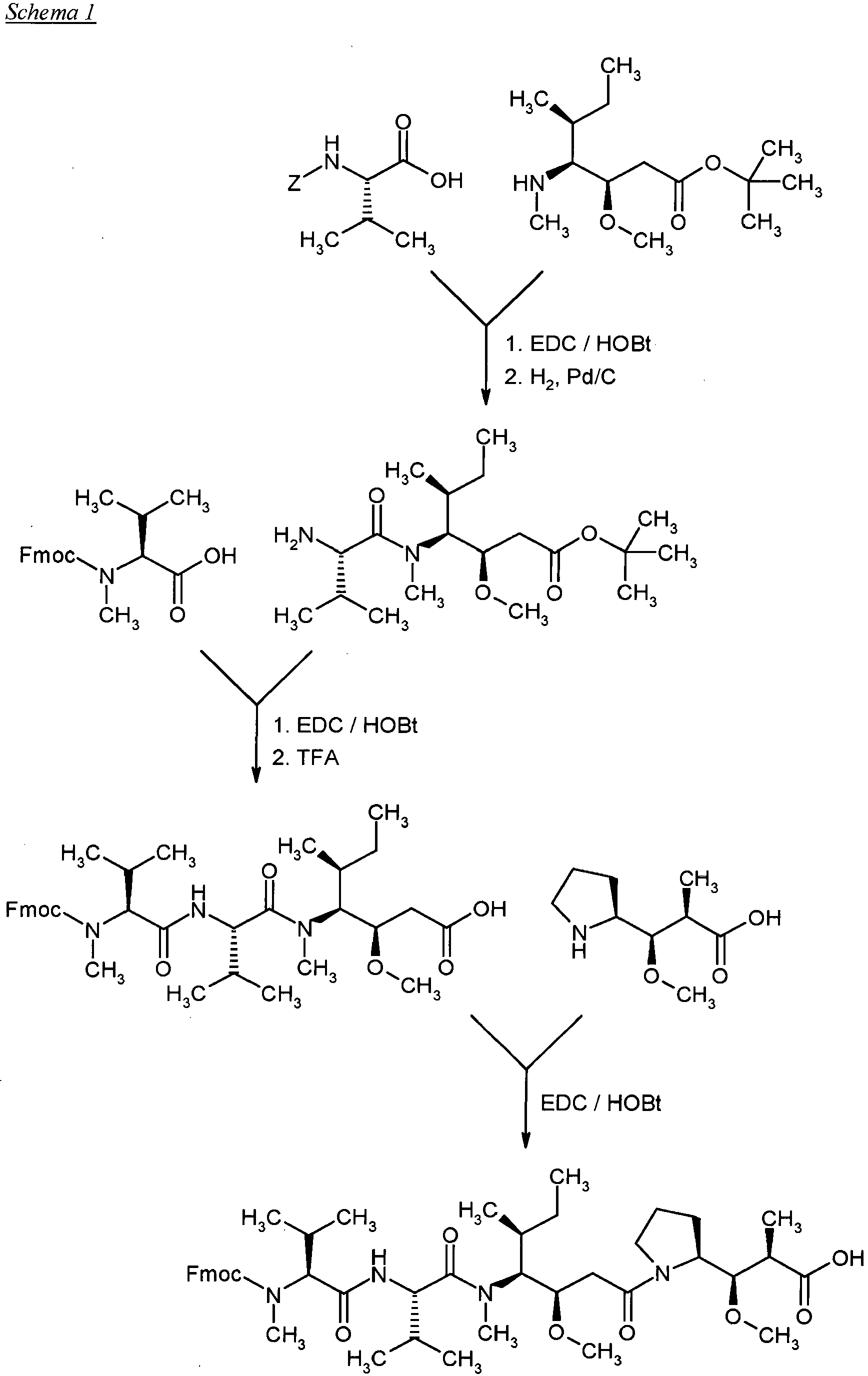

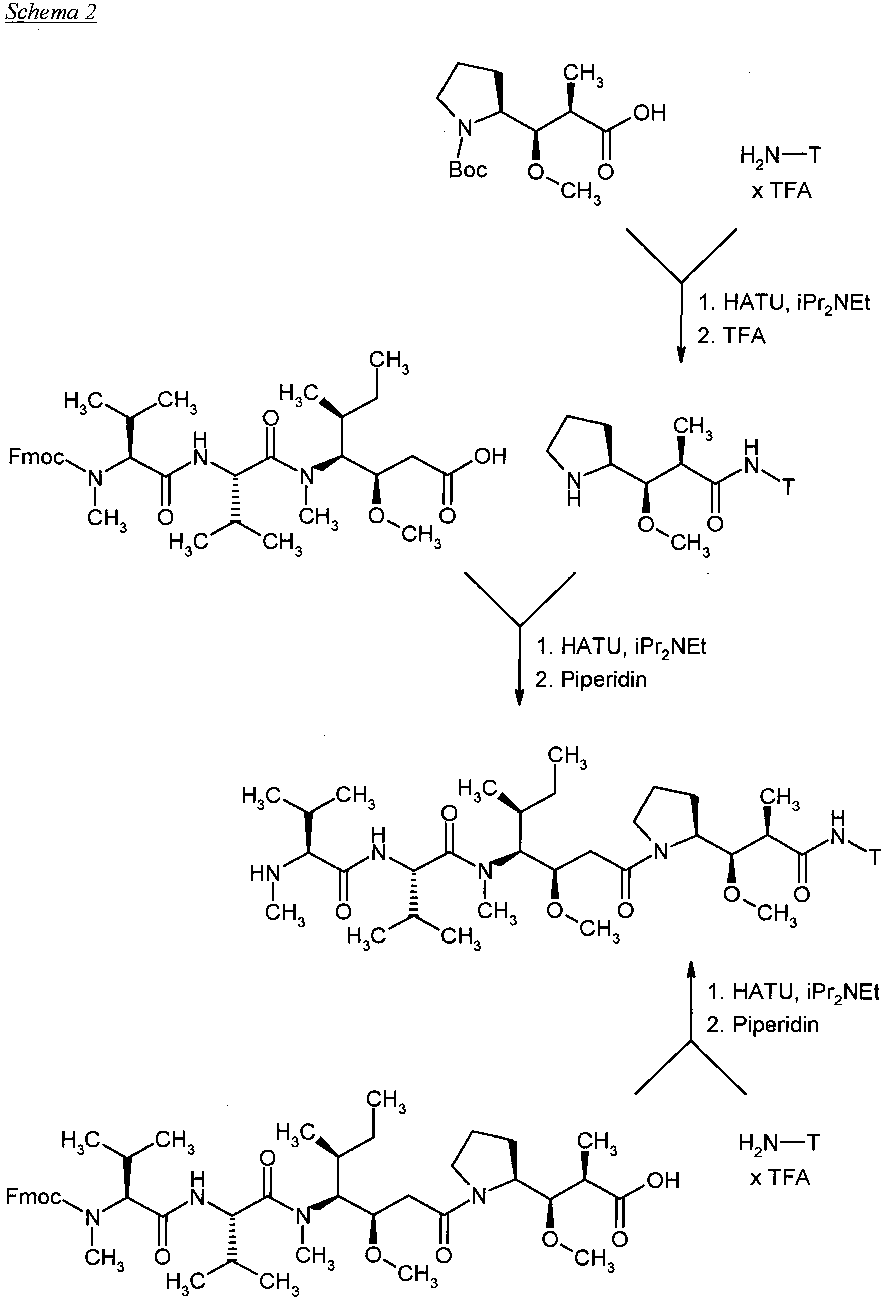

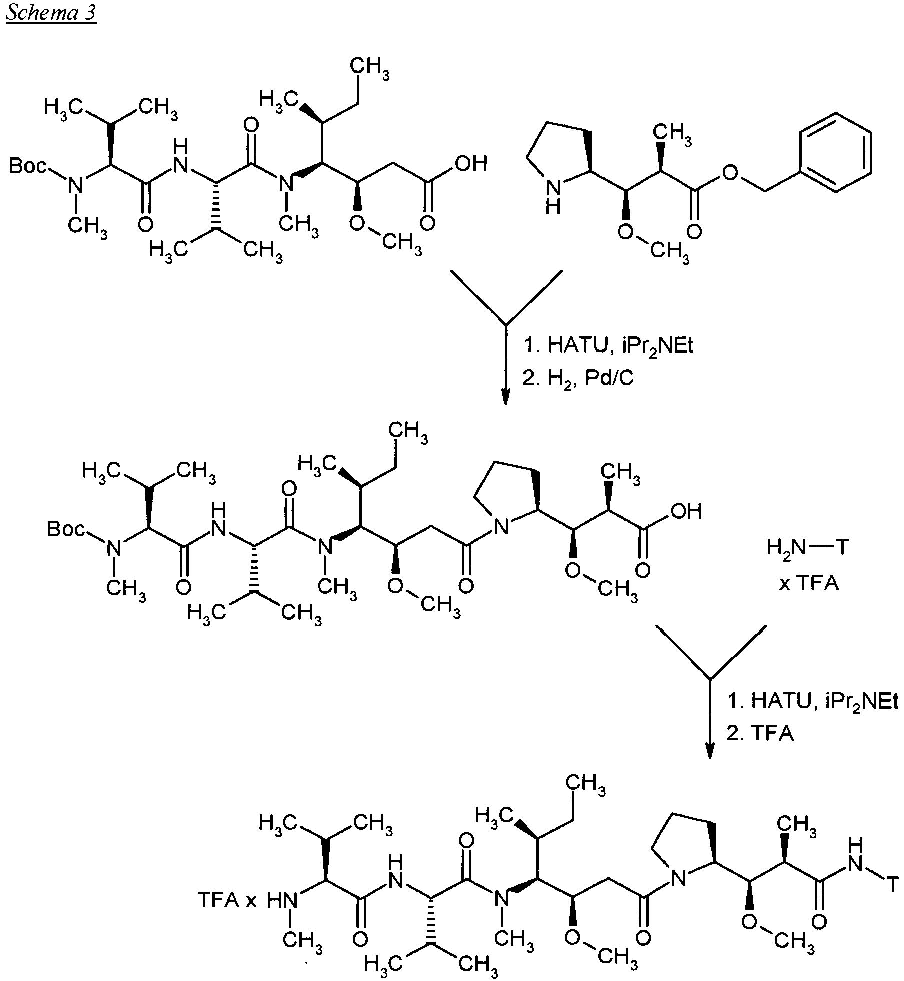

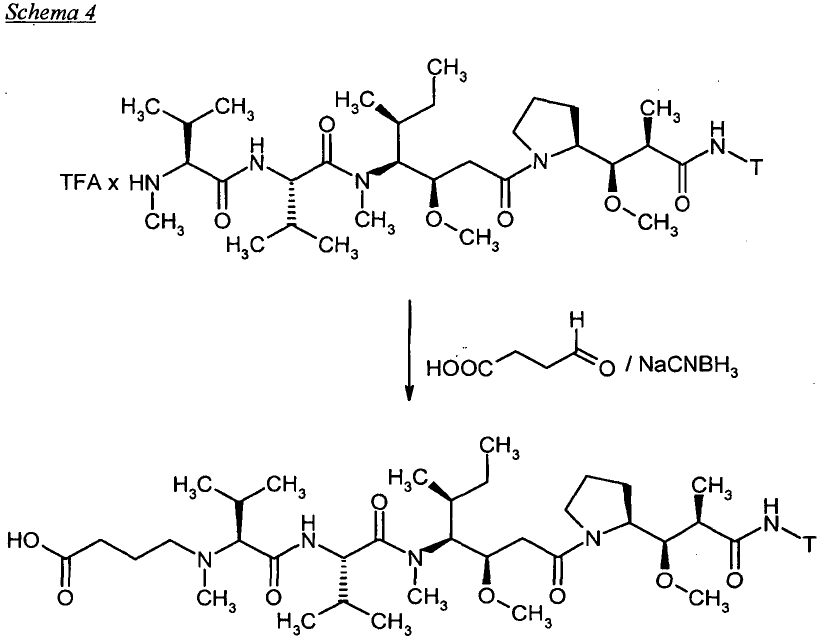

Die erfindungsgemäßen Verbindungen besitzen wertvolle pharmakologische Eigenschaften und können zur Vorbeugung und Behandlung von Erkrankungen bei Menschen und Tieren verwendet werden.

Im Vergleich zu anderen, aus dem Stand der Technik bekannten Auristatin-Derivaten hat die in den Verbindungen der vorliegenden Erfindung enthaltene N-terminale Carboxyalkyl-Gruppierung [HOOC-L- in Formel (I)] nicht die bloße Funktion eines Linkers für die potentielle Verknüpfung mit Antikörperproteinen oder anderen Liganden, sondern stellt vielmehr ein konstitutives Strukturelement für das überraschend vorteilhafte Eigenschaftsprofil dieser Verbindungen dar.

Die erfindungsgemäßen Verbindungen zeigen im Vergleich beispielsweise zu Monomethylauristatin F (MMAF) eine stärkere cytotoxische Aktivität oder weisen ein vermindertes Potential auf, gleichzeitig auch Substrate für zelluläre Transporterproteine zu sein.

Die erfindungsgemäßen Verbindungen sind daher in besonderem Maße zur Verwendung für die Behandlung von hyperproliferativen Erkrankungen beim Menschen und bei Säugetieren allgemein geeignet. Die Verbindungen können einerseits die Zellproliferation und Zellteilung hemmen, blockieren, verringern oder senken und andererseits die Apoptose verstärken. Zu den hyperproliferativen Erkrankungen, zu deren Behandlung die erfindungsgemäßen Verbindungen eingesetzt werden können, zählt insbesondere die Gruppe der Krebs- und Tumorerkrankungen. Hierunter werden im Rahmen der vorliegenden Erfindung insbesondere die folgenden Erkrankungen verstanden, ohne jedoch auf sie beschränkt zu sein: Brustkarzinome und Brusttumore (Mammakarzinome einschließlich ductaler und lobulärer Formen, auch *in situ*), Atemwegstumore (kleinzelliges und nicht-kleinzelliges Karzinom, Bronchialkarzinome), Hirntumore (z.B. des Hirnstamms und des Hypothalamus, Astrocytoma, Ependymoma, Glioblastoma, Gliome, Medulloblastoma, Meningiome sowie neuro-ektodermale und pineale Tumore), Tumore der Verdauungsorgane (Speiseröhren-, Magen-, Gallenblasen-, Dünndarm-, Dickdarm-, Rektum- und Analkarzinome), Lebertumore (u.a. hepatozelluläres Karzinom, Cholangiokarzinom und gemischthepatozelluläres Cholangiokarzinom), Tumore des Kopf- und Halsbereiches (Larynx-, Hypopharynx-, Nasopharynx-, Oropharynx-, Lippen- und Mundhöhlenkarzinome, orale Melanome), Hauttumore (Basaliome, Spinaliome, Plattenepithelkarzinome, Kaposi-Sarkom, maligne Melanome, nicht-melanomartiger Hautkrebs, Merkelzell-Hautkrebs, Mastzelltumore), Tumore des Stütz- und Bindegewebes (u.a. Weichteilsarkome, Osteosarkome, maligne fibröse Histiozytome, Chondrosarkome, Fibrosarkome, Hämangiosarkome, Leiomyosarkome, Liposarkome, Lymphosarkome und Rhabdomyosarkome), Tumore der Augen (u.a. intraokuläres Melanom und Retinoblastom), Tumore der endokrinen und exokrinen Drüsen (z.B. der thyroiden und parathyroiden Drüsen, Bauchspeicheldrüsen- und Speicheldrüsenkarzinome, Adenokarzinome), Tumore des Harntrakts (Blasen-, Penis-, Nieren-, Nierenbecken- und Harnleitertumore) sowie Tumore der reproduktiven Organe (Endometrium-, Zervix-, Ovarial-, Vaginal-, Vulva- und Uteruskarzinome der Frau sowie Prostata-und Hodenlcarzinome des Mannes). Dazu gehören auch proliferative Erkrankungen des Blutes, des Lymphsystems und des Rückenmarks, in solider Form und als zirkulierende Zellen, wie Leukämien, Lymphome und myeloproliferative Erkrankungen, z.B. akute myeloide, akute lymphoblastische, chronisch-lymphozytische, chronisch-myelogene und Haarzell-Leukämie, sowie AIDS-korrelierte Lymphome, Hodgkin-Lymphome, Non-Hodgkin-Lymphome, kutane T-Zell-Lymphome, Burkitt-Lymphome und Lymphome im zentralen Nervensystem.

Diese gut charakterisierten Krankheiten des Menschen können mit vergleichbarer Ätiologie auch in anderen Säugetieren vorkommen und dort ebenfalls mit den Verbindungen der vorliegenden Erfindung behandelt werden.

Die Behandlung der zuvor genannten Krebserkrankungen mittels der erfindungsgemäßen Verbindungen umfasst sowohl eine Behandlung der soliden Tumore als auch eine Behandlung metastasierter oder zirkulierender Formen hiervon.

Der Begriff "Behandlung" oder "behandeln" wird im Rahmen dieser Erfindung konventionell verwendet und bedeutet die Versorgung, Pflege und Betreuung eines Patienten mit dem Ziel, eine Krankheit oder gesundheitliche Abweichung zu bekämpfen, zu verringern, abzuschwächen oder zu erleichtern und die Lebensbedingungen zu verbessern, die durch diese Krankheit beeinträchtigt werden, wie beispielsweise bei einer Krebserkrankung.

Weiterer Gegenstand der vorliegenden Erfindung ist somit die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein antiproliferatives Konjugat, in dem eine erfindungsgemäßen Verbindungen mit Proteinen, wie beispielsweise Antikörper, verbunden ist. Die erfindungsgemäßen Verbindungen können allein oder bei Bedarf in Kombination mit einer oder mehreren anderen pharmakologisch wirksamen Substanzen eingesetzt werden, solange diese Kombination nicht zu unerwünschten und inakzeptablen Nebenwirkungen führt. Weiterer Gegenstand der vorliegenden Erfindung sind daher Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen.

Beispielsweise können die Verbindungen der vorliegenden Erfindung mit bekannten anti-hyperproliferativen, zytostatischen oder zytotoxischen Substanzen zur Behandlung von Krebserkrankungen kombiniert werden. Als geeignete Kombinationswirkstoffe seien beispielhaft genannt:
Aldesleukin, Alendronsäure, Alfaferon, Alitretinoin, Allopurinol, Aloprim, Aloxi, Altretamin, Aminoglutethimid, Amifostin, Amrubicin, Amsacrin, Anastrozol, Anzmet, Aranesp, Arglabin, Arsentrioxid, Aromasin, 5-Azacytidin, Azathioprin, BCG oder tice-BCG, Bestatin, Betamethason-Acetat, Betamethason-Natriumphosphat, Bexaroten, Bleomycin-Sulfat, Broxuridin, Bortezomib, Busulfan, Calcitonin, Campath, Capecitabin, Carboplatin, Casodex, Cefeson, Celmoleukin, Cerubi din, Chlorambucil, Cisplatin, Cladribin, Clodronsäure, Cyclophosphamid, Cytarabin, Dacarbazin, Dactinomycin, DaunoXome, Decadron, Decadron-Phosphat, Delestrogen, Denileukin Diftitox, Depomedrol, Deslorelin, Dexrazoxan, Diethylstilbestrol, Diflucan, Docetaxel, Doxifluridin, Doxorubicin, Dronabinol, DW-166HC, Eligard, Elitek, Ellence, Emend, Epirubicin, Epoetin-alfa, Epogen, Eptaplatin, Ergamisol, Estrace, Estradiol, Estramustin-Natriumphosphat, Ethinylestradiol, Ethyol, Etidronsäure, Etopophos, Etoposid, Fadrozol, Farston, Filgrastim, Finasterid, Fligrastim, Floxuridin, Fluconazol, Fludarabin, 5-Fluordeoxyuridin-Monophosphat, 5-Fluoruracil (5-FU), Fluoxymesteron, Flutamid, Formestan, Fosteabin, Fotemustin, Fulvestrant, Gammagard, Gemcitabin, Gemtuzumab, Gleevec, Gliadel, Goserelin, Granisetron-Hydrochlorid, Histrelin, Hycamtin, Hydrocorton, erythro-Hydroxynonyladenin, Hydroxyharnstoff, Ibritumomab Tiuxetan, Idarubicin, Ifosfamid, Interferon-alpha, Interferon-alpha-2, Interferon-alpha-2α, Interferon-alpha-2β, Interferon-alpha-n1, Interferon-alpha-n3, Interferon-beta, Interferon-gamma-1α, Interleukin-2, Intron A, Iressa, Irinotecan, Kytril, Lentinan-Sulfat, Letrozol, Leucovorin, Leuprolid, Leuprolid-Acetat, Levamisol, Levofolinsäure-Calciumsalz, Levothroid, Levoxyl, Lomustin, Lonidamin, Marinol, Mechlorethamin, Mecobalamin, Medroxyprogesteron-Acetat, Megestrol-Acetat, Melphalan, Menest, 6-Mercaptopurin, Mesna, Methotrexat, Metvix, Miltefosin, Minocyclin, Mitomycin C, Mitotan, Mitoxantron, Modrenal, Myocet, Nedaplatin, Neulasta, Neumega, Neupogen, Nilutamid, Nolvadex, NSC-631570, OCT-43, Octreotid, Ondansetron-Hydrochlorid, Orapred, Oxaliplatin, Paclitaxel, Pediapred, Pegaspargase, Pegasys, Pentostatin, Picibanil, Pilocarpin-Hydrochlorid, Pirarubicin, Plicamycin, Porfimer-Natrium, Prednimustin, Prednisolon, Prednison, Premarin, Procarbazin, Procrit, Raltitrexed, Rebif, Rhenium-186-Etidronat, Rituximab, Roferon-A, Romurtid, Salagen, Sandostatin, Sargramostim, Semustin, Sizofiran, Sobuzoxan, Solu-Medrol, Streptozocin, Strontium-89-chlorid, Synthroid, Tamoxifen, Tamsulosin, Tasonermin, Tastolacton, Taxoter, Teceleukin, Temozolomid, Teniposid, Testosteron-Propionat, Testred, Thioguanin, Thiotepa, Thyrotropin, Tiludronsäure, Topotecan, Toremifen, Tositumomab, Tastuzumab, Teosulfan, Tretinoin, Trexall, Trimethylmelamin, Trimetrexat, Triptorelin-Acetat, Triptorelin-Pamoat, UFT, Uridin, Valrubicin, Vesnarinon, Vinblastin, Vincristin, Vindesin, Vinorelbin, Virulizin, Zinecard, Zinostatin-Stimalamer, Zofran; ABI-007, Acolbifen, Actimmun, Affinitak, Aminopterin, Arzoxifen, Asoprisnil, Atamestan, Atrasentan, Avastin, BAY 43-9006 (Sorafenib), CCI-779, CDC-501, Celebrex, Cetuximab, Crisnatol, Cyproteron-Acetat, Decitabin, DN-101, Doxorubicin-MTC, dSLIM, Dutasterid, Edotecarin, Eflornithin, Exatecan, Fenretinid, Histamin-Dihydrochlorid, Histrelin-Hydrogel-Implant, Holmium-166-DOTMP, Ibandronsäure, Interferon-gamma, Intron-PEG, Ixabepilon, Keyhole Limpet-Hemocyanin, L-651582, Lanreotid, Lasofoxifen, Libra, Lonafarnib, Miproxifen, Minodronat, MS-209, liposomales MTP-PE, MX-6, Nafarelin, Nemorubicin, Neovastat, Nolatrexed, Oblimersen, Onko-TCS, Osidem, Paclitaxel-Polyglutamat, Pamidronat-Dinatrium, PN-401, QS-21, Quazepam, R-1549, Raloxifen, Ranpirnas, 13-*cis*-Retinsäure, Satraplatin, Seocalcitol, T-138067, Tarceva, Taxoprexin, Thymosin-alpha-1, Tiazofurin, Tipifarnib, Tirapazamin, TLK-286, Toremifen, TransMID-107R, Valspodar, Vapreotid, Vatalanib, Verteporfin, Vinflunin, Z-100, Zoledronsäure, sowie Kombinationen hiervon.

In einer bevorzugten Ausführungsform können die Verbindungen der vorliegenden Erfindung mit anti-hyperproliferativen Agentien kombiniert werden, welche beispielhaft - ohne dass diese Aufzählung abschließend wäre - sein können:
Aminoglutethimid, L-Asparaginase, Azathioprin, 5-Azacytidin, Bleomycin, Busulfan, Carboplatin, Carmustin, Chlorambucil, Cisplatin, Colaspase, Cyclophosphamid, Cytarabin, Dacarbazin, Dactinomycin, Daunorubicin, Diethylstilbestrol, 2',2'-Difluordeoxycytidin, Docetaxel, Doxorubicin (Adriamycin), Epirubicin, Epothilon und seine Derivate, erythro-Hydroxynonyladenin, Ethinylestradiol, Etoposid, Fludarabin-Phosphat, 5-Fluordeoxyuridin, 5-Fluordeoxyuridin-Monophosphat, 5-Fluoruracil, Fluoxymesteron, Flutamid, Hexamethylmelamin, Hydroxyharnstoff, Hydroxyprogesteron-Caproat, Idarubicin, Ifosfamid, Interferon, Irinotecan, Leucovorin, Lomustin, Mechlorethamin, Medroxyprogesteron-Acetat, Megestrol-Acetat, Melphalan, 6-Mercaptopurin, Mesna, Methotrexat, Mitomycin C, Mitotan, Mitoxantron, Paclitaxel, Pentostatin, *N*-Phosphonoacetyl-L-aspartat (PALA), Plicamycin, Prednisolon, Prednison, Procarbazin, Raloxifen, Semustin, Streptozocin, Tamoxifen, Teniposid, Testosteron-Propionat, Thioguanin, Thiotepa, Topotecan, Trimethylmelamin, Uridin, Vinblastin, Vincristin, Vindesin und Vinorelbin.

In viel versprechender Weise lassen sich die erfindungsgemäßen Verbindungen auch mit biologischen Therapeutika wie Antikörpern (z.B. Avastin, Rituxan, Erbitux, Herceptin) kombinieren. Die erfindungsgemäßen Verbindungen können auch in Kombination mit gegen die Angiogenese gerichteten Therapien positive Effekte erzielen, wie zum Beispiel mit Avastin, Axitinib, Recentin, Regorafenib, Sorafenib oder Sunitinib. Kombinationen mit Inhibitoren des Proteasoms und von mTOR sowie Kombinationen mit Antihormonen und steroidalen metabolischen Enzyminhibitoren sind wegen ihres günstigen Nebenwirkungsprofils ebenfalls besonders geeignet.

Generell können mit der Kombination von Verbindungen der vorliegenden Erfindung mit anderen, zytostatisch oder zytotoxisch wirksamen Agentien folgende Ziele verfolgt werden:
- eine verbesserte Wirksamkeit bei der Verlangsamung des Wachstums eines Tumors, bei der Reduktion seiner Größe oder sogar bei seiner völligen Eliminierung im Vergleich zu einer Behandlung mit einem einzelnen Wirkstoff;
- die Möglichkeit, die verwendeten Chemotherapeutika in geringerer Dosierung als bei der Monotherapie einzusetzen;
- die Möglichkeit einer verträglicheren Therapie mit weniger Nebeneffekten im Vergleich zur Einzelgabe;
- die Möglichkeit zur Behandlung eines breiteren Spektrums von Tumorerkrankungen;
- das Erreichen einer höheren Ansprechrate auf die Therapie;
- eine längere Überlebenszeit der Patienten im Vergleich zur heutigen Standardtherapie.

Darüber hinaus können die erfindungsgemäßen Verbindungen auch in Kombination mit einer Strahlentherapie und/oder einer chirurgischen Intervention eingesetzt werden.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie beispielsweise oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- abs.: absolut
- Ac: Acetyl
- aq.: wässrig, wässrige Lösung
- Boc: *tert*.-Butoxycarbonyl
- br.: breit (bei NMR)
- Bsp.: Beispiel
- *ca.*: *circa,* ungefähr
- CI: chemische Ionisation (bei MS)
- d: Dublett (bei NMR)
- d: Tag(e)
- DC: Dünnschichtchromatographie
- DCI: direkte chemische Ionisation (bei MS)
- dd: Dublett von Dublett (bei NMR)
- DMAP: 4-*N*,*N*-Dimethylaminopyridin
- DME: 1,2-Dimethoxyethan
- DMF: *N*,*N*-Dimethylformamid
- DMSO: Dimethylsulfoxid
- DPBS: Dulbecco's Phosphat-gepufferte Salz-Lösung
- dt: Dublett von Triplett (bei NMR)
- d. Th.: der Theorie (bei chemischer Ausbeute)
- EDC: *N*'-(3-Dimethylaminopropyl)-*N*-ethylcarbodiimid-Hydrochlorid
- EI: Elektronenstoß-Ionisation (bei MS)
- eq.: Äquivalent(e)
- ESI: Elektrospray-Ionisation (bei MS)
- FCS: fötales Kälberserum
- Fmoc: (9*H*-Fluoren-9-ylmethoxy)carbonyl
- GC-MS: Gaschromatographie-gekoppelte Massenspektrometrie
- ges.: gesättigt
- GTP: Guanosin-5'-triphosphat
- h: Stunde(n)
- HATU: *O*-(7-Azabenzotriazol-1-yl)-*N*,*N*,*N*',*N*'-tetramethyluroniumhexafluorophosphat
- HEPES: 4-(2-Hydroxyethyl)piperazin-1-ethansulfonsäure
- HOAc: Essigsäure
- HOBt: 1-Hydroxy-1*H*-benzotriazol-Hydrat
- HOSu: *N*-Hydroxysuccinimid
- HPLC: Hochdruck-, Hochleistungsflüssigchromatographie
- HR-MS: hochaufgelöste Massenspektrometrie
- konz.: konzentriert
- LC-MS: Flüssigchromatographie-gekoppelte Massenspektrometrie
- m: Multiplett (bei NMR)
- min: Minute(n)
- MS: Massenspektrometrie
- MTBE: Methyl-*tert*.-butylether
- MTT: 3-(4,5-Dimethylthiazol-2-yl)-2,5-diphenyl-2H-tetrazoliumbromid
- NMM: *N*-Methylmorpholin
- NMP: *N*-Methyl-2-pyrrolidinon
- NMR: Kernresonanzspektrometrie
- PBS: Phosphat-gepufferte Salz-Lösung
- Pd/C: Palladium auf Aktivkohle
- quant.: quantitativ (bei Ausbeute)
- quart: Quartett (bei NMR)
- quint: Quintett (bei NMR)
- R_{f}: Retentionsindex (bei DC)
- RT: Raumtemperatur
- Rₜ: Retentionszeit (bei HPLC)
- s: Singulett (bei NMR)
- t: Triplett (bei NMR)
- *tert.*: tertiär
- TFA: Trifluoressigsäure
- THF: Tetrahydrofuran
- UV: Ultraviolett-Spektrometrie
- v/v: Volumen zu Volumen-Verhältnis (einer Lösung)
- Z: Benzyloxycarbonyl
- zus.: zusammen

### HPLC-, LC-MS- und GC-MS-Methoden:

### Methode 1 (LC-MS):

Instrument: Waters Acquity SQD UPLC System; Säule: Waters Acquity UPLC HSS T3 1.8µ 50 mm x 1 mm; Eluent A: 11 Wasser + 0.25 ml 99%-ige Ameisensäure, Eluent B: 11 Acetonitril + 0.25 ml 99%-ige Ameisensäure; Gradient: 0.0 min 90% A → 1.2 min 5% A → 2.0 min 5% A; Fluss: 0.40 ml/min; Ofen: 50°C; UV-Detektion: 210-400 nm.

### Methode 2 (LC-MS):

Instrument: Micromass QuattroPremier mit Waters UPLC Acquity; Säule: Thermo Hypersil GOLD 1.9µ 50 mm x 1 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Methode 3 (LC-MS):

Instrument: Micromass Quattro Micro MS mit HPLC Agilent Serie 1100; Säule: Thermo Hypersil GOLD 3µ 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 4 (LC-MS):

Gerätetyp MS: Micromass ZQ; Gerätetyp HPLC: HP 1100 Series; UV DAD; Säule: Phenomenex Gemini 3µ 30 mm x 3.00 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 5 (HPLC):

Gerät: HP 1090 Serie II; Säule: Merck Chromolith SpeedROD RP-18e, 50 mm x 4.6 mm; Vorsäule: Merck Chromolith Guard Cartridge Kit RP-18e, 5 mm x 4.6 mm; Injektionsvolumen: 5 µl; Eluent A: 70% HClO₄ in Wasser (4 ml/Liter), Eluent B: Acetonitril; Gradient: 0.00 min 20% B → 0.50 min 20% B → 3.00 min 90% B → 3.50 min 90% B → 3.51 min 20% B → 4.00 min 20% B; Fluss: 5 ml/min; Säulentemperatur: 40°C.

### Methode 6 (HPLC):

Gerät: Waters 2695 mit DAD 996; Säule: Merck Chromolith SpeedROD RP-18e, 50 mm x 4.6 mm; Vorsäule: Merck Chromolith Guard Cartridge Kit RP-18e, 5 mm x 4.6 mm; Eluent A: 70% HClO₄ in Wasser (4 ml/Liter), Eluent B: Acetonitril; Gradient: 0.00 min 5% B → 0.50 min 5% B → 3.00 min 95% B → 4.00 min 95% B; Fluss: 5 ml/min.

### Methode 7 (LC-MS):

Gerätetyp MS: Waters ZQ; Gerätetyp HPLC: Agilent 1100 Series; UV DAD; Säule: Thermo Hypersil GOLD 3µ 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 100% A → 3.0 min 10% A → 4.0 min 10% A → 4.1 min 100% A (Fluss 2.5 ml/min); Ofen: 55°C; Fluss: 2 ml/min; UV-Detektion: 210 nm.

### Methode 8 (LC-MS):

Gerätetyp MS: Waters ZQ; Gerätetyp HPLC: Agilent 1100 Series; UV DAD; Säule: Thermo Hypersil GOLD 3µ 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 100% A → 2.0 min 60% A → 2.3 min 40% A → 3.0 min 20% A → 4.0 min 10% A → 4.2 min 100% A (Fluss 2.5 ml/min); Ofen: 55°C; Fluss: 2 ml/min; UV-Detektion: 210 nm.

### Methode 9 (LC-MS):

Instrument: Waters Acquity SQD UPLC System; Säule: Waters Acquity UPLC HSS T3 1.8µ 50 mm x 1 mm; Eluent A: 11 Wasser + 0.25 ml 99%-ige Ameisensäure, Eluent B: 11 Acetonitril + 0.25 ml 99%-ige Ameisensäure; Gradient: 0.0 min 95% A → 6.0 min 5% A → 7.5 min 5% A; Ofen: 50°C; Fluss: 0.35 ml/min; UV-Detektion: 210-400 nm.

### Methode 10 (HPLC):

Gerät: Agilent 1200 Series; Säule: Agilent Eclipse XDB-C18 5µ 4.6 mm x 150 mm; Vorsäule: Phenomenex KrudKatcher Disposable Pre-Column; Injektionsvolumen: 5 µl; Eluent A: 11 Wasser + 0.01% Trifluoressigsäure; Eluent B: 11 Acetonitril + 0.01% Trifluoressigsäure; Gradient: 0.00 min 10% B → 1.00 min 10% B → 1.50 min 90% B → 5.5 min 10% B; Fluss: 2 ml/min; Säulentemperatur: 30°C.

### Methode 11 (LC-MS):

Instrument: Waters Acquity SQD UPLC System; Säule: Waters Acquity UPLC HSS T3 1.8µ 30 mm x 2 mm; Eluent A: 11 Wasser + 0.25 ml 99%-ige Ameisensäure, Eluent B: 11 Acetonitril + 0.25 ml 99%-ige Ameisensäure; Gradient: 0.0 min 90% A → 1.2 min 5% A → 2.0 min 5% A; Fluss: 0.60 ml/min; Ofen: 50°C; UV-Detektion: 208-400 nm.

### Methode 12 (GC-MS):

Instrument: Micromass GCT, GC 6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### Methode 13 (HR-MS):

Instrument: Thermo Scientific LTQ Orbitrap XL; FTMS ESI positiv.

Für alle Reaktanden oder Reagenzien, deren Herstellung im Folgenden nicht explizit beschrieben ist, gilt, dass sie von allgemein zugänglichen Quellen kommerziell bezogen wurden. Für alle übrigen Reaktanden oder Reagenzien, deren Herstellung im Folgenden ebenfalls nicht beschrieben ist und die nicht kommerziell erhältlich waren oder von Quellen bezogen wurden, die nicht allgemein zugänglich sind, ist ein Verweis auf die veröffentlichte Literatur angegeben, in der ihre Herstellung beschrieben ist.

### Ausgangsverbindunzen und Intermediate:

### Ausgangsverbindung 1

### (2R,3R)-3-[(2S)-1-(tert.-Butoxycarbonyl)pyrrolidin-2-yl]-3-methoxy-2-methylpropansäure (Boc-Dolaproin) Dicyclohexylamin-Salz

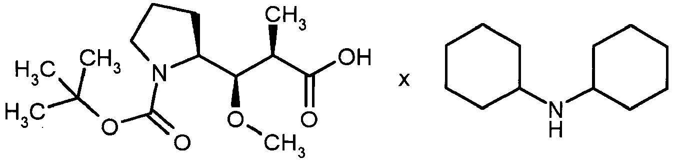

Die Titelverbindung kann auf verschiedenen Wegen nach Literaturvorschriften hergestellt werden, siehe z.B. Pettit et al., Synthesis 1996, 719; Shioiri et al., Tetrahedron Lett. 1991, 32, 931; Shioiri et al., Tetrahedron 1993, 49, 1913; Koga et al., Tetrahedron Lett. 1991, 32, 2395; Vidal et al., Tetrahedron 2004, 60, 9715; Poncet et al., Tetrahedron 1994, 50, 5345. Sie wurde hier entsprechend der Vorschrift von Shioiri et al. (Tetrahedron Lett. 1991, 32, 931) synthetisiert.

### Ausgangsverbindung 2

### tert.-Butyl-(3R,4S,5S)-3-methoxy-5-methyl-4-(methylamino)heptanoat-Hydrochlorid (Dolaisoleucin-OtBu x HCl)

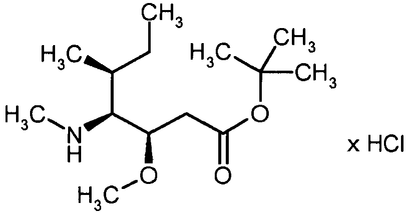

Die Titelverbindung kann auf verschiedenen Wegen nach Literaturvorschriften hergestellt werden, siehe z.B. Pettit et al., J. Org. Chem. 1994, 59, 1796; Koga et al., Tetrahedron Lett. 1991, 32, 2395; Shioiri et al., Tetrahedron Lett. 1991, 32, 931; Shioiri et al., Tetrahedron 1993, 49, 1913. Sie wurde hier entsprechend der Vorschrift von Koga et al. (Tetrahedron Lett. 1991, 32, 2395) synthetisiert.

### Intermediat 1

### tert.-Butyl-(3R,4S,5S)-4-[{N-[(benzyloxy)carbonyl]-L-valyl}(methyl)amino]-3-methoxy-5-methylheptanoat

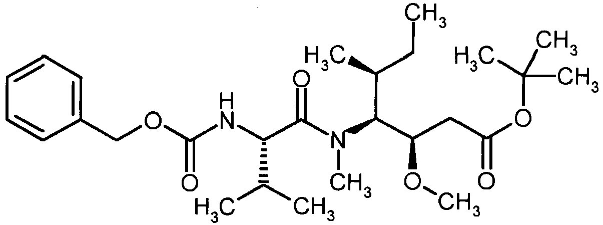

425 mg (1.7 mmol) *N*-[(Benzyloxy)carbonyl]-L-valin wurden in 50 ml DMF gelöst und nacheinander mit 500 mg (1.7 mmol) *tert*.-Butyl-(3*R*,4*S*,5*S*)-3-methoxy-5-methyl-4-(methylamino)-heptanoat-Hydrochlorid (Ausgangsverbindung 2), 356 mg (1.9 mmol) 1-(3-Dimethylaminopropyl)-3-ethylcarbodiimid-Hydrochlorid, 285 mg (1.9 mmol) 1-Hydroxy-1*H*-benzotriazol-Hydrat sowie 655 mg (5.1 mmol) *N*,*N*-Diisopropylethylamin versetzt. Die Mischung wurde 20 h bei RT gerührt. Anschließend wurden nochmals 142 mg (0.5 mmol) *N*-[(Benzyloxy)carbonyl]-L-valin, 119 mg (0.6 mmol) 1-(3-Dimethylaminopropyl)-3-ethylcarbodiimid-Hydrochlorid, 95 mg (0.6 mmol) 1-Hydroxy-1*H*-benzotriazol-Hydrat sowie 218 mg (1.7 mmol) *N*,*N*-Diisopropylethylamin zugesetzt und die Mischung 90 min lang mit Ultraschall behandelt. Der Ansatz wurde dann in eine Mischung aus halbgesättigter wässriger Ammoniumchlorid-Lösung und Ethylacetat gegossen. Die organische Phase wurde abgetrennt, nacheinander mit gesättigter Natriumhydrogencarbonat-Lösung und gesättigter Natriumchlorid-Lösung gewaschen, über Magnesiumsulfat getrocknet, filtriert und eingeengt. Der Rückstand wurde durch präparative HPLC gereinigt. Es wurden 329 mg (40% d. Th.) der Titelverbindung als farbloses Öl erhalten.

HPLC (Methode 5): Rₜ = 2.5 min;

LC-MS (Methode 1): Rₜ = 1.45 min; MS (ESIpos): m/z = 493 (M+H)⁺.

### Intermediat 2

### tert.-Butyl-(3R,4S,5S)-3-methoxy-5-methyl-4-[methyl(L-valyl)amino]heptanoat

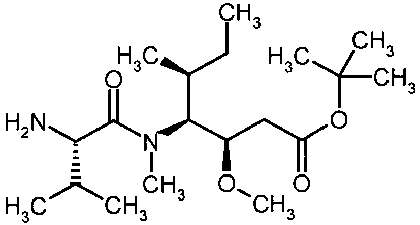

500 mg (1 mmol) *tert*.-Butyl-(3*R*,4*S*,5*S*)-4-[{*N*-[(benzyloxy)carbonyl]-L-valyl}(methyl)amino]-3-methoxy-5-methylheptanoat (Intermediat 1) wurden in 50 ml Methanol gelöst und nach Zugabe von 100 mg 10%-igem Palladium auf Aktivkohle 1 h lang bei RT unter Normaldruck hydriert. Der Katalysator wurde danach abfiltriert und das Lösungsmittel im Vakuum entfernt. Man erhielt 370 mg (quant.) der Titelverbindung als nahezu farbloses Öl.

HPLC (Methode 5): Rₜ = 1.59 min;

LC-MS (Methode 1): Rₜ = 0.74 min; MS (ESIpos): m/z = 359 (M+H)⁺.

### Intermediat 3

### N-[(9H-Fluoren-9-ylmethoxy)carbonyl]-N-methyl-L-valyl-N-[(3R,4S,5S)-1-tert.-butoxy-3-methoxy-5-methyl-1-oxoheptan-4-yl]-N-methyl-L-valinamid

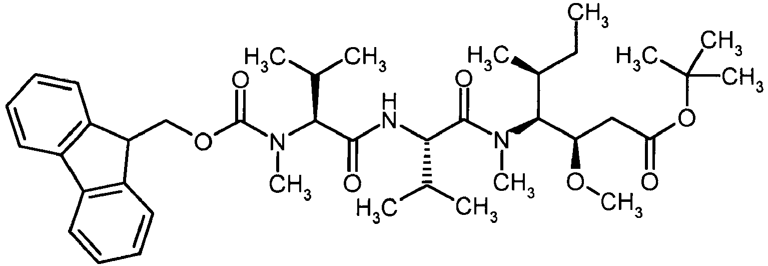

396 mg (1.1 mmol) *N*-[(9*H*-Fluoren-9-ylmethoxy)carbonyl]-*N*-methyl-L-valin wurden in 20 ml DMF gelöst und nacheinander mit 365 mg (1 mmol) *tert*.-Butyl-(3*R*,4*S*,5*S*)-3-methoxy-5-methyl-4-[methyl(L-valyl)amino]heptanoat (Intermediat 2), 234 mg (1.2 mmol) 1-(3-Dimethylaminopropyl)-3-ethylcarbodiimid-Hydrochlorid sowie 187 mg (1.2 mmol) 1-Hydroxy-1*H*-benzotriazol-Hydrat versetzt. Die Mischung wurde über Nacht bei RT gerührt. Der Ansatz wurde danach in eine Mischung aus halbgesättigter wässriger Ammoniumchlorid-Lösung und Ethylacetat gegossen. Die organische Phase wurde abgetrennt, nacheinander mit gesättigter Natriumhydrogencarbonat-Lösung und gesättigter Natriumchlorid-Lösung gewaschen, über Magnesiumsulfat getrocknet, filtriert und eingeengt. Der Rückstand wurde direkt, ohne weitere Reinigung, in der nächsten Stufe eingesetzt. Ausbeute: 660 mg (68% d. Th.)

HPLC (Methode 5): Rₜ = 3.0 min;

LC-MS (Methode 1): Rₜ = 1.61 min; MS (ESIpos): m/z = 694 (M+H)⁺.

### Intermediat 4

### N-[(9H-Fluoren-9-ylmethoxy)carbonyl]-N-methyl-L-valyl-N-[(2R,3S,4S)-1-carboxy-2-methoxy-4-methylhexan-3-yl]-N-methyl-L-valinamid

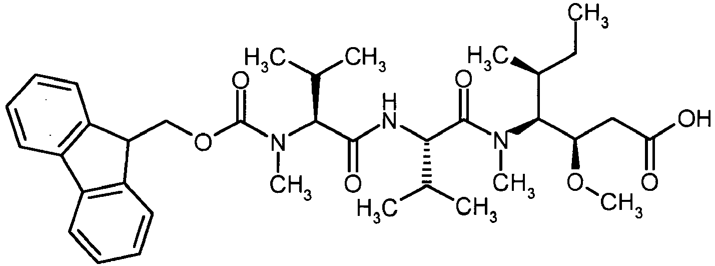

650 mg (0.94 mmol) *N*-[(9*H*-Fluoren-9-ylmethoxy)carbonyl]-*N*-methyl-L-valyl-*N*-[(3*R*,4*S*,5*S*)-1-*tert*.-butoxy-3-methoxy-5-methyl-1-oxoheptan-4-yl]-*N*-methyl-L-valinamid (Intermediat 3) wurden in 5 ml Dichlormethan aufgenommen, mit 5 ml Trifluoressigsäure versetzt und über Nacht bei RT gerührt. Anschließend wurde im Vakuum eingeengt und der verbliebene Rückstand durch präparative HPLC gereinigt. Es wurden 430 mg (72% d. Th.) der Titelverbindung als farbloser Schaum erhalten.

HPLC (Methode 5): Rₜ = 2.4 min;

LC-MS (Methode 2): Rₜ = 1.51 min; MS (ESIpos): m/z = 638 (M+H)⁺.

### Intermediat 5

### N-(tert.-Butoxycarbonyl)-N-methyl-L-valyl-N-[(2R,3S,4S)-1-carboxy-2-methoxy-4-methylhexan-3-yl]-N-methyl-L-valinamid

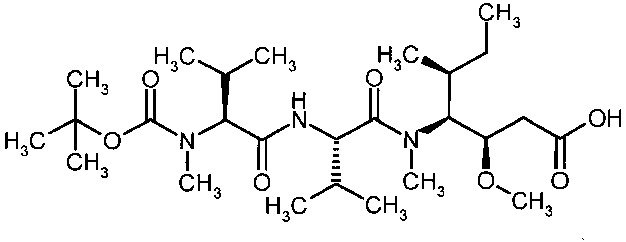

51 mg (0.08 mmol) *N*-[(9*H*-Fluoren-9-ylmethoxy)carbonyl]-*N*-methyl-L-valyl-*N*-[(2*R*,3*S*,4*S*)-1-carb-oxy-2-methoxy-4-methylhexan-3-yl]-*N*-methyl-L-valinamid (Intermediat 4) wurden in 10 ml DMF gelöst und mit 0.5 ml Piperidin versetzt. Nach 10 min Rühren bei RT wurde der Ansatz im Vakuum eingeengt und der Rückstand mit Diethylether verrührt. Die unlöslichen Bestandteile wurden abfiltriert und mehrfach mit Diethylether gewaschen. Dann wurde der Filter-Rückstand in 5 ml Dioxan/Wasser (1:1) aufgenommen und die Lösung mit 1 N Natronlauge auf pH 11 eingestellt. Unter Ultraschallbehandlung wurden in mehreren Portionen insgesamt 349 mg (1.6 mmol) Di-*tert.-*butyldicarbonat zugegeben, wobei der pH-Wert der Lösung bei 11 gehalten wurde. Nach Beendigung der Reaktion wurde das Dioxan abgedampft und die wässrige Lösung mit Zitronensäure auf einen pH-Wert von 2-3 eingestellt. Man extrahierte zweimal mit je 50 ml Ethylacetat. Die organischen Phasen wurden vereinigt, über Magnesiumsulfat getrocknet und im Vakuum eingeengt. Der Rückstand wurde in Diethylether aufgenommen und das Produkt mit Pentan ausgefällt. Durch Dekantieren wurde das Lösungsmittel abgetrennt. Der Rückstand wurde noch mehrmals mit Pentan digeriert und schließlich im Hochvakuum getrocknet. Es wurden so 31 mg (93% d. Th.) der Titelverbindung erhalten.

HPLC (Methode 6): Rₜ = 2.2 min;

LC-MS (Methode 2): Rₜ = 1.32 min; MS (ESIpos): m/z = 516 (M+H)⁺.

### Intermediat 6

### Benzyl-(2R,3R)-3-methoxy-2-methyl-3-[(2S)-pyrrolidin-2-yl]propanoat Trifluoressigsäure-Salz

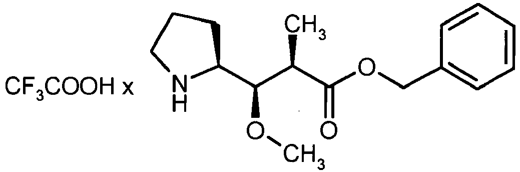

Zunächst wurde (2*R*,3*R*)-3-[(2*S*)-1-(*tert*.-Butoxycarbonyl)pyrrolidin-2-yl]-3-methoxy-2-methylpropansäure aus 1.82 g (3.88 mmol) des Dicyclohexylamin-Salzes (Ausgangsverbindung 1) durch Aufnehmen in 150 ml Ethylacetat und Ausschütteln mit 100 ml 0.5%-iger wässriger Schwefelsäure freigesetzt. Die organische Phase wurde über Magnesiumsulfat getrocknet, filtriert und eingeengt. Der Rückstand wurde in 10 ml Dioxan und 10 ml Wasser aufgenommen, mit 1517 mg (4.66 mmol) Cäsiumcarbonat versetzt und 5 min im Ultraschallbad behandelt. Anschließend wurde im Vakuum eingeengt und der Rückstand einmal mit DMF co-destilliert. Der Rückstand wurde dann in 15 ml DMF aufgenommen und mit 1990 mg (11.64 mmol) Benzylbromid versetzt. Die Mischung wurde 15 min im Ultraschallbad behandelt und anschließend im Vakuum eingeengt. Der Rückstand wurde zwischen Ethylacetat und Wasser verteilt. Die organische Phase wurde abgetrennt, mit gesättigter Natriumchlorid-Lösung gewaschen und danach eingeengt. Der Rückstand wurde schließlich durch präparative HPLC gereinigt. Man erhielt auf diese Weise 1170 mg (80% d. Th.) des Boc-geschützten Intermediats *tert*.-Butyl-(2*S*)-2-[(1*R*,2*R*)-3-(benzyloxy)-1-methoxy-2-methyl-3-oxopropyl]pyrrolidin-1-carboxylat.

Sofort anschließend wurden diese 1170 mg des Intermediats in 15 ml Dichlormethan aufgenommen und mit 5 ml Trifluoressigsäure versetzt. Nach 15 min Rühren bei RT wurde der Ansatz im Vakuum eingeengt und der Rückstand aus Dioxan lyophilisiert. Nach Trocknen im Hochvakuum verblieben 1333 mg (84% d. Th.) der Titelverbindung als gelbes Öl.

HPLC (Methode 5): Rₜ = 1.5 min;

LC-MS (Methode 1): Rₜ = 0.59 min; MS (ESIpos): m/z = 278 (M+H)⁺.

### Intermediat 7

### N-(tert.-Butoxycarbonyl)-N-methyl-L-valyl-N-[(3R,4S,5S)-1-{(2S)-2-[(1R,2R)-2-carboxy-1-methoxypropyl]pyrrolidin-1-yl}-3-methoxy-5-methyl-1-oxoheptan-4-yl]-N-methyl-L-valinamid

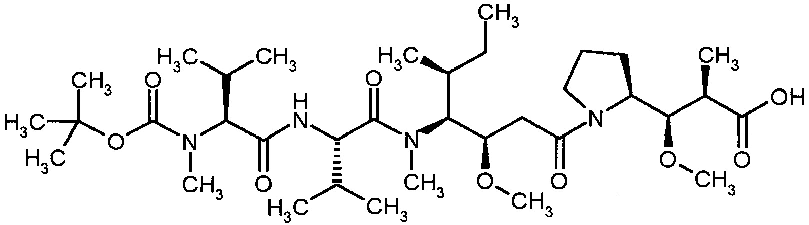

1200 mg (2.33 mmol) *N*-(*tert*.-Butoxycarbonyl)-*N*-methyl-L-valyl-*N*-[(2*R*,3*S*,4*S*)-1-carboxy-2-methoxy-4-methylhexan-3-yl]-*N*-methyl-L-valinamid (Intermediat 5) wurden mit 910.8 mg (2.33 mmol) Benzyl-(2*R*,3*R*)-3-methoxy-2-methyl-3-[(2*S*)-pyrrolidin-2-yl]propanoat Trifluoressigsäure-Salz (Intermediat 6), 1327 mg (3.49 mmol) *O*-(7-Azabenzotriazol-1-yl)-*N*,*N*,*N*',*N*'-tetramethyluroniumhexafluorophosphat und 2027 µl *N*,*N*-Diisopropylethylamin in 50 ml DMF zusammengegeben und 5 min bei RT gerührt. Danach wurde das Lösungsmittel im Vakuum entfernt. Der verbliebene Rückstand wurde in Ethylacetat aufgenommen und nacheinander mit 5%-iger wässriger Zitronensäure-Lösung und gesättigter Natriumhydrogencarbonat-Lösung ausgeschüttelt. Die organische Phase wurde abgetrennt und eingeengt. Der Rückstand wurde mittels präparativer HPLC gereinigt. Die Produktfraktionen wurden vereinigt, eingeengt und der Rückstand im Hochvakuum getrocknet. Es wurden so 1000 mg (55% d. Th.) des Benzylester-Intermediats *N*-(*tert*.-Butoxycarbonyl)-*N*-methyl-L-valyl-*N*-[(3*R*,4*S*,5*S*)-1-{(2*S*)-2-[(1*R*,2*R*)-3-(benzyloxy)-1-methoxy-2-methyl-3-oxopropyl]pyrrolidin-1-yl}-3-methoxy-5-methyl-1-oxoheptan-4-yl]-*N*-methyl-L-valinamid als ein Harz erhalten.

LC-MS (Methode 1): Rₜ = 1.56 min; MS (ESIpos): m/z = 775 (M+H)⁺.

Die gesamte erhaltene Menge dieses Intermediats wurde in 25 ml einer Mischung aus Methanol und Dichlormethan (20:1) aufgenommen und die Benzylester-Gruppe durch Hydrierung unter Normaldruck mit 10% Palladium auf Aktivkohle als Katalysator entfernt. Nach 30 min Rühren bei RT wurde der Katalysator abfiltriert und das Filtrat im Vakuum eingeengt. Man erhielt 803 mg (91% d. Th.) der Titelverbindung als weißen Feststoff.

HPLC (Methode 5): Rₜ = 2.1 min;

LC-MS (Methode 1): Rₜ = 1.24 min; MS (ESIpos): m/z = 685 (M+H)⁺.

### Intermediat 8

### N^{α}-(tert.-Butoxycarbonyl)-N-methoxy-N-methyl-L-phenylalaninamid

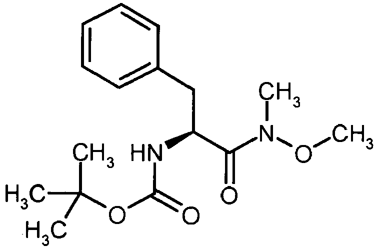

1000 mg (3.77 mmol) *N*-(*tert*.-Butoxycarbonyl)-L-phenylalanin wurden in 10 ml Dichlormethan vorgelegt und mit 733 mg (4.52 mmol) 1,1'-Carbonyldiimidazol versetzt. Der Ansatz wurde 15 min bis zur Beendigung der Gasentwicklung gerührt. Anschließend wurde das Gemisch mit 441 mg (4.52 mmol) *N*,*O*-Dimethylhydroxylamin-Hydrochlorid sowie 657 µl (3.77 mmol) *N*,*N*-Diisopropylethylamin versetzt und 1 h bei RT gerührt. Danach wurde der Ansatz mit Dichlormethan verdünnt und nacheinander mit destilliertem Wasser, 0.5 N Salzsäure und gesättigter Natriumchlorid-Lösung gewaschen. Die organische Phase wurde abgetrennt, und die vereinigten wässrigen Phasen wurden mit Ethylacetat rückextrahiert. Die vereinigten organischen Phasen wurden über Magnesiumsulfat getrocknet und im Vakuum eingeengt. Man erhielt 1090 mg (93% d. Th.) der Titelverbindung.

LC-MS (Methode 1): Rₜ = 1.02 min; MS (ESIpos): m/z = 309 (M+H)⁺.

### Intermediat 9

### tert.-Butyl-[(2S)-1-oxo-3-phenylpropan-2-yl]carbamat

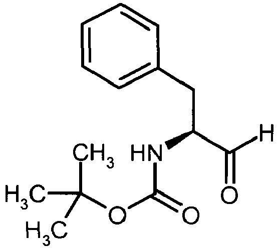

1090 mg (3.5 mmol) *N*^{α}-(*tert*.-Butoxycarbonyl)-*N*-methoxy-*N*-methyl-L-phenylalaninamid wurden in 20 ml 2-Methyltetrahydrofuran gelöst und auf 0°C gekühlt. Dann wurden langsam 4.2 ml (4.2 mmol) einer 1 M Lithiumaluminiumhydrid-Lösung in THF zugegeben und die Reaktionsmischung 30 min bei 0°C gerührt. Anschließend wurde vorsichtig 5%-ige wässrige KaliumhydrogensulfatLösung zugesetzt. Der Ansatz wurde mit Wasser verdünnt und mit MTBE extrahiert. Die organische Phase wurde über Magnesiumsulfat getrocknet und im Vakuum eingeengt. Man erhielt so 820 mg (94% d. Th.) der Titelverbindung.

GC-MS (Methode 12): Rₜ = 5.61 min; MS (ESIpos): m/z = 220 (M-29)⁺

¹H-NMR (500 MHz, DMSO-d₆): δ [ppm] = 1.15-1.42 (m, 9H), 7.11-7.39 (m, 5H), 9.52 (s, 1H) [weitere Signale unter Lösungsmittel-Peaks verborgen].

### Intermediat 10

### (2S,3Z)-1,5-Diphenylpent-3-en-2-amin Trifluoressigsäure-Salz

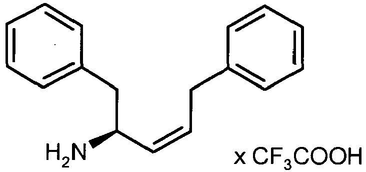

Unter Argon wurden zu einer Suspension von 986 mg (2.2 mmol) Triphenyl(2-phenylethyl)phosphoniumbromid [herstellbar z.B. nach R.W. Hartmann, M. Reichert, Archiv der Pharmazie 333, 145 (2000); K.C. Nicolaou et al., European J. Chem. 1, 467 (1995)] in 125 ml THF bei -78°C 842 µl (2.1 mmol) 2.5 M *n*-Butyllithium-Lösung in Hexan gegeben und die Mischung anschließend 1 h bei 0°C gerührt. Das Reaktionsgemisch wurde danach wieder auf -78°C abgekühlt, und eine Lösung von 500 mg (2.0 mmol) *tert*.-Butyl-[(2*S*)-1-oxo-3-phenylpropan-2-yl]carbamat in 5 ml trockenem THF wurde hinzugegeben. Der Ansatz wurde auf 0°C erwärmt und 3 h bei dieser Temperatur nachgerührt. Die Reaktion wurde dann durch Zugabe von gesättigter wässriger Ammoniumchlorid-Lösung abgebrochen. Das Gemisch wurde mit MTBE verdünnt, die Phasen wurden getrennt, und die organische Phase wurde über Magnesiumsulfat getrocknet und im Vakuum eingeengt. Der Rückstand wurde über eine Kieselgelsäule mit dem Laufmittel Cyclohexan/Ethylacetat 5:1 gereinigt. Nach Einengen der entsprechenden Fraktionen wurden 173 mg (25.6% d. Th.) des Boc-geschützten Intermediats *tert*.-Butyl-[(2*S*,3*Z*)-1,5-diphenylpent-3-en-2-yl]carbamat erhalten.

¹H-NMR (400 MHz, DMSO-d₆): δ [ppm] = 1.16-1.46 (m, 9H), 2.62 (dd, *J* = 13.20 Hz, 7.34 Hz, 1H), 2.73-3.18 (m, 1H), 4.56 (t, *J* = 7.46 Hz, 1H), 5.27-5.57 (m, 1H), 6.98-7.32 (m, 10H) [weitere Signale unter Lösungsmittel-Peaks verborgen].

173 mg (512 µmol) des Intermediats *tert*.-Butyl-[(2*S*,3*Z*)-1,5-diphenylpent-3-en-2-yl]carbamat wurden in 16 ml Dichlormethan vorgelegt, mit 4 ml Trifluoressigsäure versetzt und 30 min bei RT stehen gelassen. Anschließend wurde das Reaktionsgemisch eingeengt und der Rückstand im Vakuum getrocknet. Es wurden 180 mg (99% d. Th.) der Titelverbindung erhalten.

LC-MS (Methode 1): Rₜ = 0.74 min; MS (ESIpos): m/z = 238 (M+H)⁺

¹H-NMR (400 MHz, DMSO-d₆): δ [ppm] = 2.64-2.83 (m, 1H), 2.88-3.02 (m, 1H), 3.05-3.22 (m, 2H), 4.00-4.55 (m, 1H), 5.16-5.46 (m, 1H), 5.48-5.78 (m, 1H), 6.60-6.89 (m, 2H), 7.14 (s, 3H), 7.22-7.36 (m, 5H), 7.89-8.27 (m, 2H).

### Intermediat 11

### (2S)-1-(Benzylsulfonyl)-3-phenylpropan-2-amin

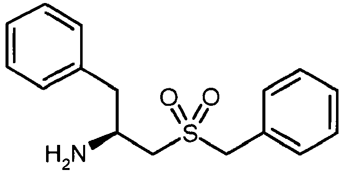

200 mg (1.13 mmol) (4*S*)-4-Benzyl-1,3-oxazolidin-2-on wurden in 3 ml *tert*.-Butanol vorgelegt und mit 280 mg (2.26 mmol) Benzylmercaptan versetzt. Das Gemisch wurde anschließend 2 Tage unter Rückfluss erhitzt. Danach wurde der Ansatz am Rotationsverdampfer eingeengt und das erhaltene Intermediat (2*S*)-1-(Benzylsulfanyl)-3-phenylpropan-2-amin ohne Aufarbeitung direkt weiter umgesetzt.

HPLC (Methode 10): Rₜ = 2.63 min;

LC-MS (Methode 1): Rₜ = 0.67 min; MS (ESIpos): m/z = 258 (M+H)⁺.

Das oben erhaltene rohe Intermediat wurde in einer Lösung von 2 ml 30%-igem Wasserstoffperoxid und 5 ml Ameisensäure gelöst und 12 h bei RT gerührt. Dann wurde das Reaktionsgemisch auf gesättigte wässrige Natriumsulfat-Lösung gegeben und dreimal mit Ethylacetat extrahiert. Die organische Phase wurde über Magnesiumsulfat getrocknet und im Vakuum eingeengt. Das erhaltene Rohprodukt wurde mittels präparativer HPLC gereinigt. Es wurden so 343 mg (61% d. Th.) der Titelverbindung erhalten.

HPLC (Methode 10): Rₜ = 2.40 min;

LC-MS (Methode 1): Rₜ = 0.65 min; MS (ESIpos): m/z = 290 (M+H)⁺.

### Intermediat 12

### (2S,3Z)-1,4-Diphenylbut-3-en-2-amin

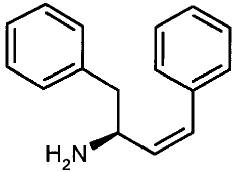

552.7 mg (9.85 mmol) Kaliumhydroxid wurden in Methanol gelöst, auf 1.1 g neutrales Aluminiumoxid aufgezogen und dann im Hochvakuum getrocknet. Zu einer Lösung von 240 mg (0.82 mmol) (2S)-1-(Benzylsulfonyl)-3-phenylpropan-2-amin und 1.56 g des so präparierten Kaliumhydroxid auf Aluminiumoxid in 6.2 ml *n*-Butanol wurden bei 5-10°C 307 µl (3.3 mmol) Dibrom-difluormethan getropft. Die Reaktionsmischung wurde 2 h bei RT gerührt, dann über Celite filtriert und der Rückstand gut mit Dichlormethan nachgewaschen. Das Filtrat wurde eingeengt und der resultierende Rückstand im Vakuum getrocknet. Das so erhaltene Rohprodukt wurde mittels präparativer HPLC gereinigt. Es wurden 98 mg (35% d. Th.) der Titelverbindung mit einem *E*/*Z-*Diastereomerenverhältnis von 4:1 erhalten.

HPLC (Methode 10): Rₜ = 2.46 min;

LC-MS (Methode 1): Rₜ = 0.75 min; MS (ESIpos): m/z = 224 (M+H)⁺.

Das oben erhaltene *E*/*Z*-Diastereomerengemisch wurde in 2 ml Ethanol und 0.2 ml *N,N-*Diisopropylethylamin gelöst und über HPLC an chiraler Phase aufgetrennt [Säule: Daicel Chiralpak AD-H, 5 µm, 250 mm x 20 mm; Eluent: Hexan/(Ethanol + 0.2% Diethylamin) 50:50 v/v; UV-Detektion: 220 nm; Temperatur: 30°C]. Die entsprechenden Fraktionen wurden am Rotationsverdampfer eingeengt und der Rückstand im Vakuum getrocknet. Es wurden 10 mg der Titelverbindung als reines Z-Isomer erhalten.

¹H-NMR (400 MHz, DMSO-d₆): δ [ppm] = 2.71 (d, *J* = 6.60 Hz, 2H), 3.73-3.95 (m, 1H), 5.42-5.67 (m, 1H), 6.21-6.50 (m, 1H), 7.08-7.38 (m, 10H) [weitere Signale unter Lösungsmittel-Peaks verborgen].

### Intermediat 13

### (2S,3E)-1,4-Diphenylbut-3-en-2-amin

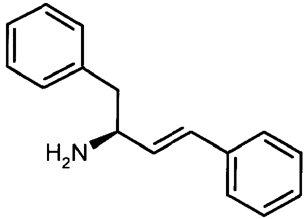

Die Titelverbindung (reines E-Isomer) wurde im Zuge der bei Intermediat 12 beschriebenen chromatographischen Diastereomerentrennung an chiraler Phase in einer Ausbeute von 45 mg erhalten.

¹H-NMR (400 MHz, DMSO-d₆): δ [ppm] = 2.62-2.83 (m, 2H), 3.52-3.71 (m, 1H), 6.18-6.30 (m, 1H), 6.34-6.46 (m, 1H), 6.98-7.57 (m, 10H) [weitere Signale unter Lösungsmittel-Peaks verborgen].

### Intermediat 14

### (1S)-2-Phenyl-1-(5-phenyl-1,3,4-oxadiazol-2-yl)ethanamin Trifluoressigsäure-Salz

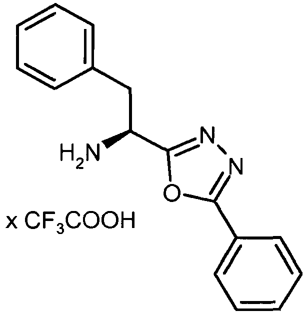

200 mg (0.75 mmol) *N*-(*tert*.-Butoxycarbonyl)-L-phenylalanin wurden bei 0°C in 5.5 ml Dichlormethan vorgelegt und mit 128 mg (0.79 mmol) 1,1'-Carbonyldiimidazol versetzt. Nach 30 min wurden 103 mg (0.75 mmol) Benzoylhydrazid zugegeben. Nach weiteren 45 min bei 0°C wurden schließlich 500 mg (1.5 mmol) Tetrabromkohlenstoff und 395 mg (1.5 mmol) Triphenylphosphin hinzugefügt. Der Ansatz wurde zunächst 2 h bei 0°C und dann über Nacht bei RT nachgerührt. Das Gemisch wurde anschließend am Rotationsverdampfer eingeengt und der Rückstand im Hochvakuum getrocknet. Das so erhaltene Rohprodukt wurde mittels präparativer HPLC gereinigt. Es wurden 217 mg (78% d. Th.) des Boc-geschützten Intermediats *tert*.-Butyl-[(1*S*)-2-phenyl-1-(5-phenyl-1,3,4-oxadiazol-2-yl)ethyl]carbamat erhalten.

HPLC (Methode 10): Rₜ = 3.01 min;

LC-MS (Methode 1): Rₜ = 1.15 min; MS (ESIpos): m/z = 366 (M+H)⁺.

217 mg (0.59 mmol) dieses Intermediats wurden in 3 ml Dichlormethan aufgenommen, mit 0.6 ml Trifluoressigsäure versetzt und 30 min bei RT gerührt. Anschließend wurde im Vakuum eingeengt. Der verbliebene Rückstand wurde im Vakuum weiter getrocknet und dann aus Dioxan lyophilisiert. Auf diese Weise wurden 214 mg (90% d. Th.) der Titelverbindung als weißer Feststoff erhalten.

HPLC (Methode 10): Rₜ = 2.43 min;

LC-MS (Methode 11): Rₜ = 0.62 min; MS (ESIpos): m/z = 266 (M+H)⁺.

### Intermediat 15

### (1R)-2-Phenyl-1-(5-phenyl-1,3,4-oxadiazol-2-yl)ethanamin Trifluoressigsäure-Salz

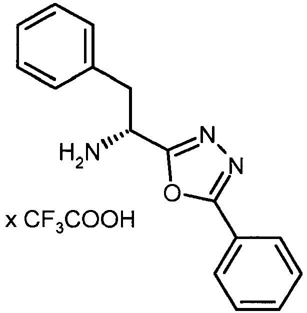

200 mg (0.75 mmol) *N*-(*tert*.-Butoxycarbonyl)-D-phenylalanin wurden bei 0°C in 5.5 ml Dichlormethan vorgelegt und mit 128.3 mg (0.79 mmol) 1,1'-Carbonyldiimidazol versetzt. Nach 30 min wurden 103 mg (0.75 mmol) Benzoylhydrazid zugegeben. Nach weiteren 45 min bei 0°C wurden schließlich 500 mg (1.5 mmol) Tetrabromkohlenstoff und 395 mg (1.5 mmol) Triphenylphosphin hinzugefügt. Der Ansatz wurde zunächst 2 h bei 0°C und dann über Nacht bei RT nachgerührt. Das Gemisch wurde anschließend am Rotationsverdampfer eingeengt und der Rückstand im Hochvakuum getrocknet. Das so erhaltene Rohprodukt wurde mittels präparativer HPLC gereinigt. Es wurden 219 mg (80% d. Th.) des Boc-geschützten Intermediats *tert*.-Butyl-[(1R)-2-phenyl-1-(5-phenyl-1,3,4-oxadiazol-2-yl)ethyl]carbamat erhalten.

HPLC (Methode 10): Rₜ = 3.01 min;

LC-MS (Methode 2): Rₜ = 1.36 min; MS (ESIpos): m/z = 366 (M+H)⁺.

219 mg (0.6 mmol) dieses Intermediats wurden in 3 ml Dichlormethan aufgenommen, mit 0.6 ml Trifluoressigsäure versetzt und 30 min bei RT gerührt. Anschließend wurde im Vakuum eingeengt. Der verbliebene Rückstand wurde im Vakuum weiter getrocknet und dann aus Dioxan lyophilisiert. Auf diese Weise wurden 196 mg (86% d. Th.) der Titelverbindung als weißer Feststoff erhalten.

HPLC (Methode 10): Rₜ = 2.41 min.

### Intermediat 16

### Methyl-4-[(1E,3S)-3-amino-4-phenylbut-1-en-1-yl]benzoat Trifluoressigsäure-Salz

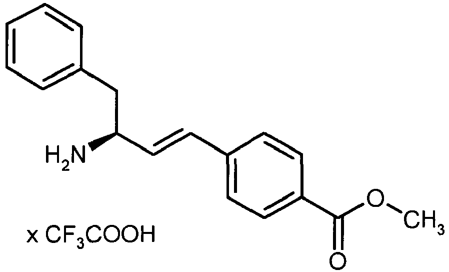

0.9 mg (4 µmol) Palladiumacetat wurden in 5 ml DMF vorgelegt und nacheinander mit 20.8 mg (97 µmol) Methyl-4-brombenzoat, 20 mg (81 µmol) (*S*)-*tert*.-Butyl 1-phenylbut-3-en-2-ylcarbamat, 1.1 mg (8 µmol) Phenylharnstoff sowie 11.2 mg (81 µmol) Kaliumcarbonat versetzt. Die Reaktionsmischung wurde anschließend 15 min bei 160°C in einer Mikrowellenapparatur (Emrys^{™} Optimizer) gerührt. Das Gemisch wurde danach filtriert und das Filtrat über präparative HPLC (Eluent: Methanol/Wasser-Gradient mit 0.1% TFA) in seine Komponenten aufgetrennt. Man erhielt so 21.3 mg (68% d. Th.) der Titelverbindung.

HPLC (Methode 10): Rₜ = 3.23 min;

LC-MS (Methode 11): Rₜ = 1.32 min; MS (ESIpos): m/z = 382 (M+H)⁺.

### Intermediat 17

### N-Methyl-L-valyl-N-[(3R,4S,5S)-1-{(2S)-2-[(1R,2R)-3-{[(2S,3Z)-1,5-diphenylpent-3-en-2-yl]-amino}-1-methoxy-2-methyl-3-oxopropyl]pyrrolidin-1-yl}-3-methoxy-5-methyl-1-oxoheptan-4-yl]-N-methyl-L-valinamid Trifluoressigsäure-Salz

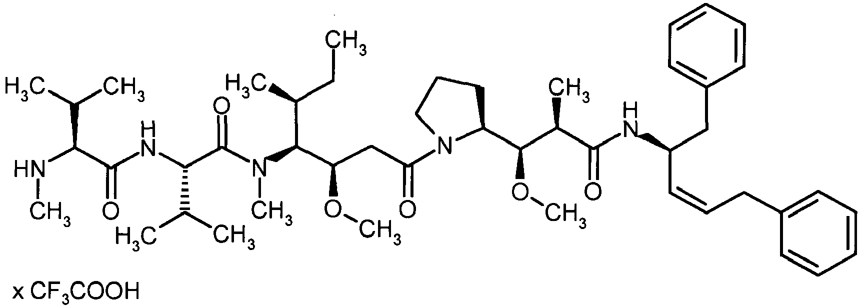

15 mg (22 µmol) *N*-(*tert*.-Butoxycarbonyl)-*N*-methyl-L-valyl-*N*-[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 7) wurden in 750 µl DMF vorgelegt und mit 11.44 µl (66 µmol) *N,N-*Diisopropylethylamin sowie 10 mg (26 µmol) HATU versetzt. Der Ansatz wurde 30 min bei RT gerührt. Anschließend wurden 8.5 mg (24 µmol) (2S,3Z)-1,5-Diphenylpent-3-en-2-amin Trifluoressigsäure-Salz (Intermediat 10) zugegeben und der Ansatz über Nacht bei RT gerührt. Das Reaktionsgemisch wurde danach direkt durch präparative HPLC in seine Komponenten aufgetrennt. Es wurden 18.1 mg (91% d. Th.) des Boc-geschützten Intermediats *N*-(*tert*.-Butoxycarbonyl)-*N*-methyl-L-valyl*-N-*[(3R,4S,5S)-1-{(2S)-2-[(1R,2R)-3-{[(2S,3Z)-1,5-diphenylpent-3-en-2-yl]amino}-1-methoxy-2-methyl-3-oxopropyl]pyrrolidin-1-yl}-3-methoxy-5-ethyl-1-oxoheptan-4-yl]-*N*-methyl-L-valinamid in Form eines weißen Feststoffs erhalten.

HPLC (Methode 10): Rₜ = 4.74 min;

LC-MS (Methode 11): Rₜ = 1.58 min; MS (ESIpos): m/z = 905 (M+H)⁺.

16 mg (18 µmol) dieses Intermediats wurden in 1 ml Dichlormethan aufgenommen, mit 0.2 ml Trifluoressigsäure versetzt und 30 min bei RT gerührt. Anschließend wurde im Vakuum eingeengt. Der verbliebene Rückstand wurde im Vakuum weiter getrocknet und dann aus Dioxan lyophilisiert. Auf diese Weise wurden 15.8 mg (97% d. Th.) der Titelverbindung erhalten.

HPLC (Methode 10): Rₜ = 2.66 min;

LC-MS (Methode 1): Rₜ = 1.03 min; MS (ESIpos): m/z = 805 (M+H)⁺.

### Intermediat 18

### N-Methyl-L-valyl-N-[(3R,4S,5S)-1-{(2S)-2-[(1R,2R)-3-{[(2S,3Z)-1,4-diphenylbut-3-en-2-yl]amino}-1-methoxy-2-methyl-3-oxopropyl]pyrrolidin-1-yl}-3-methoxy-5-methyl-1-oxoheptan-4-yl]-N-methyl-L-valinamid Trifluoressigsäure-Salz

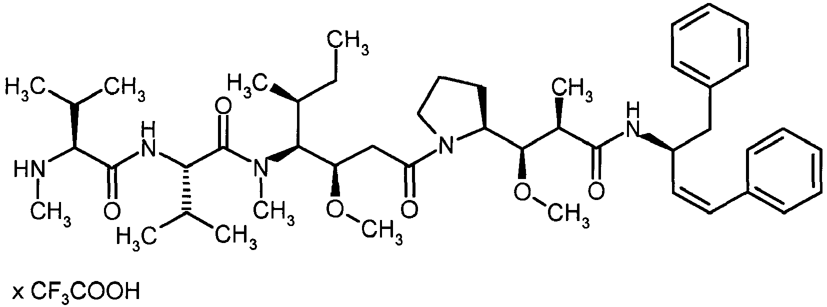

Zunächst wurde *N-*(*tert*.-Butoxycarbonyl)-*N*-methyl-L-valyl-*N*-[(3*R*,4*S*,5*S*)-1-{(2*S*)-2-[(1*R*,2*R*)-3-{[(2*S*,3*Z*)-1,4-diphenylbut-3-en-2-yl]amino}-1-methoxy-2-methyl-3-oxopropyl]pyrrolidin-1-yl}-3-methoxy-5-methyl-1-oxoheptan-4-yl]-*N*-methyl-L-valinamid in Analogie zur Synthese von Intermediat 17 durch Umsetzung von 20 mg (29 µmol) *N*-(*tert*.-Butoxycarbonyl)-*N*-methyl-L-valyl-*N-*[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 7) mit 7.1 mg (32 µmol) (2*S*,3*Z*)-1,4-Di-phenylbut-3-en-2-amin (Intermediat 12) hergestellt.

Ausbeute: 9.2 mg (35% d. Th.)

HPLC (Methode 10): Rₜ = 4.52 min;

LC-MS (Methode 1): Rₜ = 1.54 min; MS (ESIpos): m/z = 891 (M+H)⁺.

Durch nachfolgende Abspaltung der Boc-Schutzgruppe mit Trifluoressigsäure erhielt man dann 9.5 mg (99% d. Th.) der Titelverbindung.

HPLC (Methode 10): Rₜ = 2.58 min;

LC-MS (Methode 1): Rₜ = 0.97 min; MS (ESIpos): m/z = 791 (M+H)⁺.

### Intermediat 19

### N-Methyl-L-valyl-N-[(3R,4S,5S)-1-{(2S)-2-[(1R,2R)-3-{[(2S,3E)-1,4-diphenylbut-3-en-2-yl]-amino}-1-methoxy-2-methyl-3-oxopropyl]pyrrolidin-1-yl}-3-methoxy-5-methyl-1-oxoheptan-4-yl]-N-methyl-L-valinamid Trifluoressigsäure-Salz

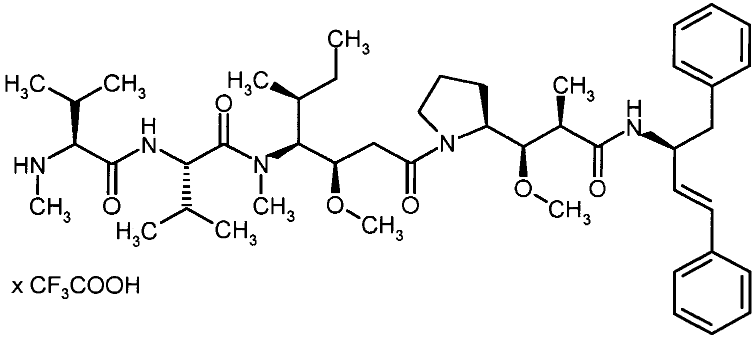

Zunächst wurde *N*-(*tert*.-Butoxycarbonyl)-*N*-methyl-L-valyl-*N*-[(3*R*,4*S*,5*S*)-1-{(2*S*)-2-[(1*R*,2*R*)-3-{[(2*S*,3*E*)-1,4-diphenylbut-3-en-2-yl]amino}-1-methoxy-2-methyl-3-oxopropyl]pyrrolidin-1-yl}-3-methoxy-5-methyl-1-oxoheptan-4-yl]-*N*-methyl-L-valinamid in Analogie zur Synthese von Intermediat 17 durch Umsetzung von 20 mg (29 µmol) *N*-(*tert*.-Butoxycarbonyl)-*N*-methyl-L-valyl-*N-*[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N-*methyl-L-valinamid (Intermediat 7) mit 7.1 mg (32 µmol) (2*S*,3*E*)-1,4-Di-phenylbut-3-en-2-amin (Intermediat 13) hergestellt.

Ausbeute: 15.1 mg (58% d. Th.)

HPLC (Methode 10): Rₜ = 4.2 min;

LC-MS (Methode 1): Rₜ = 1.51 min; MS (ESIpos): m/z = 891 (M+H)⁺.

Durch nachfolgende Abspaltung der Boc-Schutzgruppe mit Trifluoressigsäure erhielt man dann 15.7 mg (99% d. Th.) der Titelverbindung.

HPLC (Methode 10): Rₜ = 2.62 min;

LC-MS (Methode 1): Rₜ = 0.97 min; MS (ESIpos): m/z = 791 (M+H)⁺.

### Intermediat 20

### N-Methyl-L-valyl-N-[(3R,4S,5S)-1-{(2S)-2-[(1R,2R)-3-{[(2S)-1-(benzylsulfonyl)-3-phenylpropan-2-yl]amino]-1-methoxy-2-methyl-3-oxopropyl]pyrrolidin-1-yl}-3-methoxy-5-methyl-1-oxoheptan-4-yl]-N-methyl-L-valinamid Trifluoressigsäure-Salz

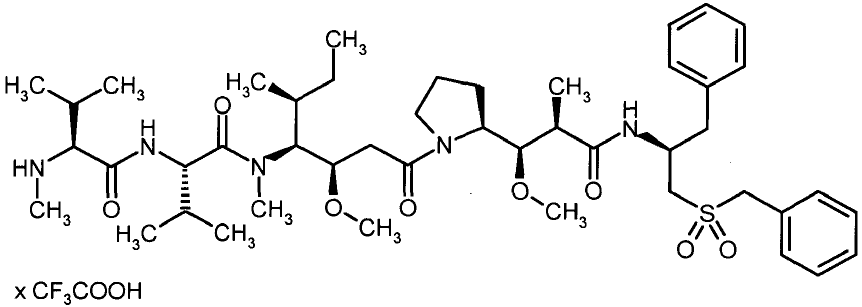

Zunächst wurde *N*-(*tert*.-Butoxycarbonyl)-*N*-methyl-L-valyl-*N*-[(3*R*,4*S*,5*S*)-1-{(2*S*)-[(1*R*,2*R*)-3-{[(2*S*)-1-(benzylsulfonyl)-3-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid in Analogie zur Synthese von Intermediat 17 durch Umsetzung von 20 mg (29 µmol) *N*-(*tert*.-Butoxycarbonyl)-*N*-methyl-L-valyl-*N*-[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 7) mit 9.3 mg (20 µmol) (2S)-1-(Benzylsulfonyl)-3-phenylpropan-2-amin (Intermediat 11) hergestellt.

Ausbeute: 19.2 mg (68% d. Th.)

HPLC (Methode 10): Rₜ = 3.5 min;

LC-MS (Methode 1): Rₜ = 1.41 min; MS (ESIpos): m/z = 957 (M+H)⁺.

Durch nachfolgende Abspaltung der Boc-Schutzgruppe mit Trifluoressigsäure erhielt man dann 19.3 mg (99% d. Th.) der Titelverbindung.

HPLC (Methode 10): Rₜ = 2.52 min;

LC-MS (Methode 1): Rₜ = 0.86 min; MS (ESIpos): m/z = 857 (M+H)⁺.

### Intermediat 21

### N-Methyl-L-valyl-N-[(3R,4S,5S)-3-methoxy-1-{(2S)-2-[(1R,2R)-1-methoxy-2-methyl-3-oxo-3-{[(1S)-2-phenyl-1-(5-phenyl-1,3,4-oxadiazol-2-yl)ethyl]amino}propyl]pyrrolidin-1-yl}-5-methyl-1-oxoheptan-4-yl]-N-methyl-L-valinamid Trifluoressigsäure-Salz

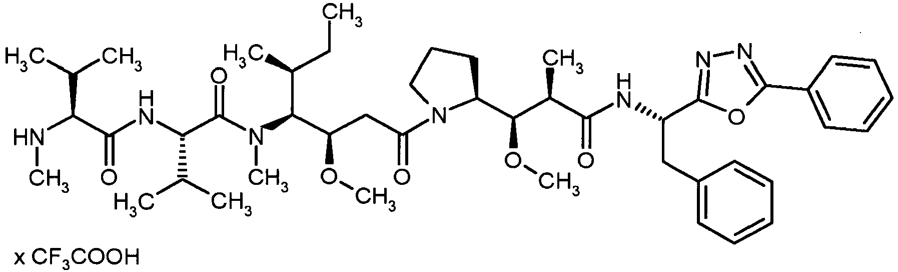

Zunächst wurde *N*-(*tert*.-Butoxycarbonyl)-N-methyl-L-valyl-*N*-[(3*R*,4*S*,5*S*)-3-methoxy-1-{(2*S*)-2-[(1*R*,2*R*)-1-methoxy-2-methyl-3-oxo-3-{[(1*S*)-2-phenyl-1-(5-phenyl-1,3,4-oxadiazol-2-yl)ethyl]-amino}propyl]pyrrolidin-1-yl}-5-methyl-1-oxoheptan-4-yl]-*N*-methyl-L-valinamid in Analogie zur Synthese von Intermediat 17 durch Umsetzung von 20 mg (29 µmol) *N*-(*tert*.-Butoxycarbonyl)-*N-*methyl-L-valyl-*N*-[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 7) mit 12.2 mg (32 µmol) (1*S*)-2-Phenyl-1-(5-phenyl-1,3,4-oxadiazol-2-yl)ethanamin Trifluoressigsäure-Salz (Intermediat 14) hergestellt.

Ausbeute: 22 mg (81% d. Th.)

HPLC (Methode 10): Rₜ = 3.74 min;

LC-MS (Methode 1): Rₜ = 1.45 min; MS (ESIpos): m/z = 933 (M+H)⁺.

Durch nachfolgende Abspaltung der Boc-Schutzgruppe mit Trifluoressigsäure wurden dann 22.4 mg (98% d. Th.) der Titelverbindung erhalten.

HPLC (Methode 10): Rₜ = 2.52 min;

LC-MS (Methode 11): Rt = 0.85 min; MS (ESIpos): m/z = 833 (M+H)⁺.

### Intermediat 22

### N-Methyl-L-valyl-N-[(3R,4S,5S)-3-methoxy-1-{(2S)-2-[(1R,2R)-1-methoxy-2-methyl-3-oxo-3-{[(1R)-2-phenyl-1-(5-phenyl-1,3,4-oxadiazol-2-yl)ethyl]amino}propyl]pyrrolidin-1-yl}-5-methyl-1-oxoheptan-4-yl]-N-methyl-L-valinamid Trifluoressigsäure-Salz

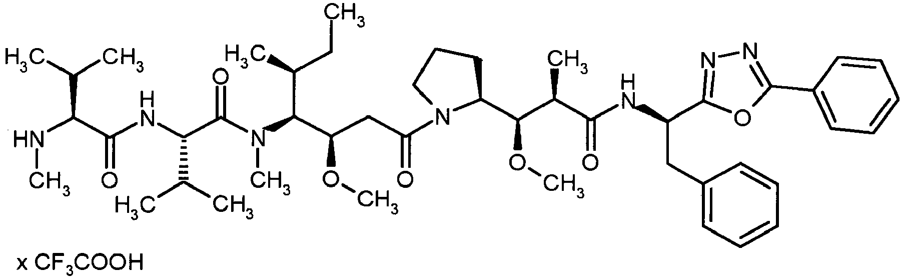

Zunächst wurde *N*-(*tert*.-Butoxycarbonyl)-*N*-methyl-L-valyl-*N*-[(3*R*,4*S*,5*S*)-3-methoxy-1-{(2*S*)-2-[(1*R*,2*R*)-1-methoxy-2-methyl-3-oxo-3-{[(1*R*)-2-phenyl-1-(5-phenyl-1,3,4-oxadiazol-2-yl)ethyl]-amino}propyl]pyrrolidin-1-yl}-5-methyl-1-oxoheptan-4-yl]-*N*-methyl-L-valinamid in Analogie zur Synthese von Intermediat 17 durch Umsetzung von 20 mg (29 µmol) *N*-(*tert*.-Butoxycarbonyl)-*N-*methyl-L-valyl-*N*-[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 7) mit 12.2 mg (32 µmol) (1*R*)-2-Phenyl-1-(5-phenyl-1,3,4-oxadiazol-2-yl)ethanamin Trifluoressigsäure-Salz (Intermediat 15) hergestellt.

Ausbeute: 17 mg (64% d. Th.)

HPLC (Methode 10): Rₜ = 3.74 min;

LC-MS (Methode 1): Rₜ = 1.45 min; MS (ESIpos): m/z = 933 (M+H)⁺.

Durch nachfolgende Abspaltung der Boc-Schutzgruppe mit Trifluoressigsäure erhielt man dann 17.1 mg (99% d. Th.) der Titelverbindung.

HPLC (Methode 10): Rₜ = 2.55 min;

LC-MS (Methode 11): Rₜ = 0.85 min; MS (ESIpos): m/z = 833 (M+H)⁺.

### Intermediat 23

### N-Methyl-L-valyl-N-[(3R,4S,5S)-3-methoxy-1-{(2S)-2-[(1R,2R)-1-methoxy-3-({(2S,3E)-4-[4-(methoxycarbonyl)phenyl]-1-phenylbut-3-en-2-yl}amino)-2-methyl-3-oxopropyl]pyrrolidin-1-yl}-5-methyl-1-oxoheptan-4-yl]-N-methyl-L-valinamid Trifluoressigsäure-Salz

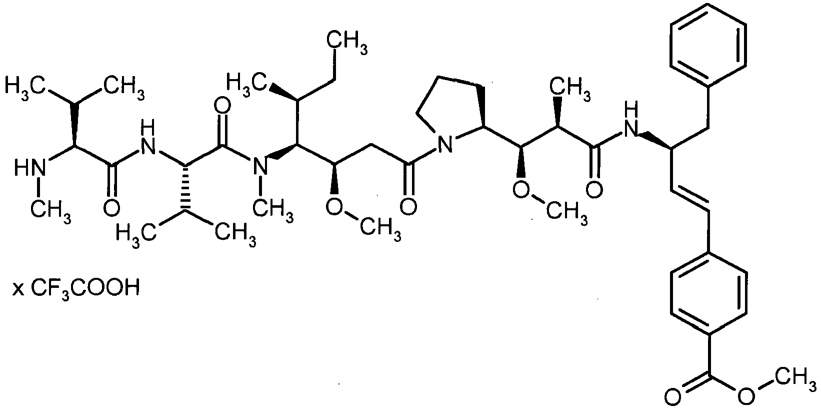

Zunächst wurde *N*-(*tert*.-Butoxycarbonyl)-*N*-methyl-L-valyl-*N*-[(3*R*,4*S*,5*S*)-3-methoxy-1-{(2*S*)-2-[(1*R*,2*R*)-1-methoxy-3-({(2*S*,3*E*)-4-[4-(methoxycarbonyl)phenyl]-1-phenylbut-3-en-2-yl}amino)-2-methyl-3-oxopropyl]pyrrolidin-1-yl}-5-methyl-1-oxoheptan-4-yl]-*N*-methyl-L-valinamid in Analogie zur Synthese von Intermediat 17 durch Umsetzung von 20 mg (29 µmol) *N*-(*tert*.-Butoxycarbonyl)-*N*-methyl-L-valyl-*N*-[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 7) mit 12.7 mg (32 µmol) Methyl-4-[(1*E*,3*S*)-3-amino-4-phenylbut-1-en-1-yl]benzoat Trifluoressigsäure-Salz (Intermediat 16) hergestellt.

Ausbeute: 8.8 mg (32% d. Th.)

LC-MS (Methode 1): Rₜ = 1.53 min; MS (ESIpos): m/z = 949 (M+H)⁺.

Durch nachfolgende Abspaltung der Boc-Schutzgruppe mit Trifluoressigsäure erhielt man dann 8 mg (90% d. Th.) der Titelverbindung.

LC-MS (Methode 1): Rt = 1.00 min; MS (ESIpos): m/z = 849 (M+H)⁺.

### Intermediat 24

### N-Methyl-L-valyl-N-[(3R,4S,5S)-1-{(2S)-2-[(1R,2R)-3-{[2-(1H-indol-3-yl)ethyl]amino}-1-methoxy-2-methyl-3-oxopropyl]pyrrolidin-1-yl}-3-methoxy-5-methyl-1-oxoheptan-4-yl]-N-methyl-L-valinamid Trifluoressigsäure-Salz

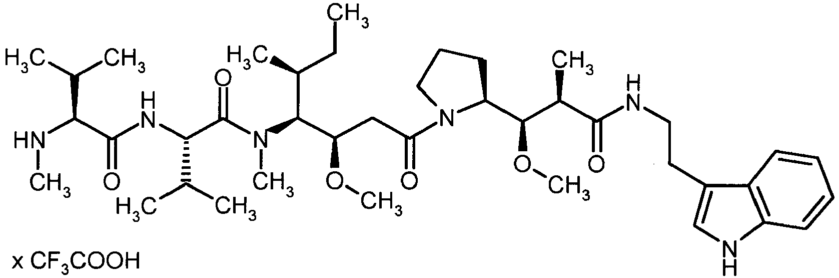

Zu einer Lösung von 100 mg (146 µmol) *N*-(*tert*.-Butoxycarbonyl)-*N*-methyl-L-valyl-*N*-[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 7) in 30 ml DMF wurden bei RT 76 µl (438 µmol) *N,N-*Diisopropylethylamin, 83 mg (219 µmol) HATU sowie 26 mg (161 µmol) 2-(1*H*-Indol-3-yl)ethanamin gegeben. Die Mischung wurde 15 min lang bei RT gerührt. Anschließend wurde das Reaktionsgemisch im Vakuum eingeengt und der Rückstand über präparative HPLC in seine Komponenten aufgetrennt. Es wurden 101 mg (83% d. Th.) des Boc-geschützten Intermediats *N-*(*tert*.-Butoxycarbonyl)-*N*-methyl-L-valyl-*N*-[(3*R*,4*S*,5*S*)-1-{(2*S*)-2-[(1*R*,2*R*)-3-{[2-(1*H*-indol-3-yl)-ethyl]amino}-1-methoxy-2-methyl-3-oxopropyl]Pyrrolidin-1-yl}-3-methoxy-5-methyl-1-oxoheptan-4-yl]-*N*-methyl-L-valinamid erhalten.

LC-MS (Methode 1): Rₜ = 1.32 min; m/z = 828 (M+H)⁺.

101 mg (122 µmol) dieses Intermediats wurden in 15 ml Dichlormethan aufgenommen, mit 1 ml Trifluoressigsäure versetzt und 30 min bei RT gerührt. Anschließend wurde im Vakuum eingeengt und der verbliebene Rückstand aus Wasser/Acetonitril lyophilisiert. Auf diese Weise wurden 108 mg der Titelverbindung in quantitativer Ausbeute als farbloser Schaum erhalten.

LC-MS (Methode 1): Rₜ = 0.84 min; MS (ESIpos): m/z = 728 (M+H)⁺.

### Intermediat 25

### N-Methyl-L-valyl-N-{(3R,4S,5S)-3-methoxy-1-[(2S)-2-{(1R,2R)-1-methoxy-2-methyl-3-oxo-3-[(2-phenylethyl)amino]propyl}pyrrolidin-]-yl]-5-methyl-1-oxoheptan-4-yl}-N-methyl-L-valinamid Trifluoressigsäure-Salz

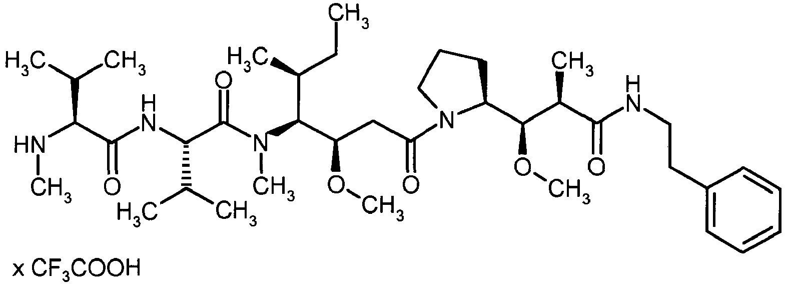

Die Titelverbindung wurde in Analogie zur Synthese von Intermediat 24 über zwei Stufen ausgehend von 60 mg (88 µmol) des Intermediats 7 durch Kupplung mit 10 mg (88 µmol) 2-Phenylethanamin und anschließende Boc-Abspaltung mit Trifluoressigsäure hergestellt. Es wurden 34 mg (97% d. Th.) der Titelverbindung erhalten.

HPLC (Methode 5): Rₜ = 2.71 min;

LC-MS (Methode 1): Rₜ = 0.80 min; MS (ESIpos): m/z = 689 (M+H)⁺.

### Ausführungsbeispiele:

### Beispiel 1

### N-(3-Carboxypropyl)-N-methyl-L-valyl-N-[(3R,4S,5S)-1-{(2S)-2-[(1R,2R)-3-{[(2S,3Z)-1,5-diphenylpent-3-en-2-yl]amino}-1-methoxy-2-methyl-3-oxopropyl]pyrrolidin-1-yl}-3-methoxy-5-methyl-1-oxoheptan-4-yl]-N-methyl-L-valinamid

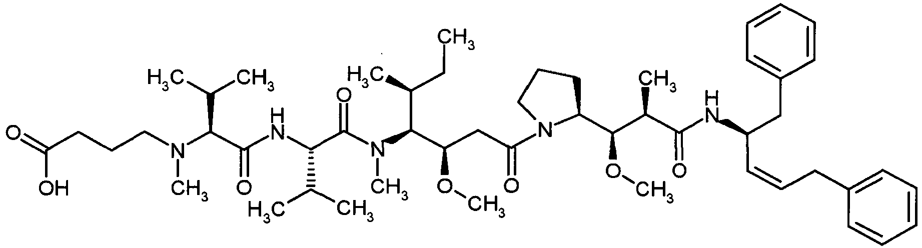

14.5 mg (16 µmol) *N*-Methyl-L-valyl-*N*-[(3*R*,4*S*,5*S*)-1-{(2*S*)-2-[(1*R*,2*R*)-3-{[(2*S*,3*S*)-1,5-diphenyl-pent-3-en-2-yl]amino}-1-methoxy-2-methyl-3-oxopropyl]pyrrolidin-1-yl}-3-methoxy-5-methyl-1-oxoheptan-4-yl]-*N*-methyl-L-valinamid Trifluoressigsäure-Salz (Intermediat 17) wurden in 1 ml Dioxan/Wasser (1:1) gelöst und mit 20.4 µl (32 µmol) einer 15%-igen wässrigen Lösung von 4-Oxobutansäure versetzt. Der Ansatz wurde anschließend 1 h bei 100°C gerührt. Nach Abkühlen auf RT wurden 1.1 mg (17 µmol) Natriumcyanoborhydrid zugegeben und die Mischung durch Zugabe von etwa 150 µl 0.1 N Salzsäure auf einen pH-Wert von 3 eingestellt. Der Ansatz wurde danach weitere 2 h bei 100°C gerührt. Nach Abkühlen wurden erneut 21 µl (32 µmol) der 15%-igen 4-Oxobutansäure-Lösung zugesetzt und der Ansatz wiederum 1 h bei 100°C gerührt. Dann wurden weitere 1.1 mg (17 µmol) Natriumcyanoborhydrid zugegeben und anschließend mit etwa 300 µl 0.1 N Salzsäure der pH-Wert wieder auf 3 eingestellt. Der Ansatz wurde danach erneut 2 h bei 100°C gerührt. Bei immer noch nicht vollständiger Umsetzung wurde diese Prozedur noch ein weiteres Mal wiederholt. Der Ansatz wurde schließlich eingeengt und das Rohprodukt mittels präparativer HPLC gereinigt und aus Dioxan lyophilisiert. Es wurden so 13.1 mg (93% d. Th.) der Titelverbindung in Form eines weißen Feststoffs erhalten.

HPLC (Methode 10): Rₜ = 2.63 min;

LC-MS (Methode 1): R, = 1.01 min; MS (ESIpos): m/z = 891 (M+H)⁺.

### Beispiel 2

### N-(3-Carboxypropyl)-N-methyl-L-valyl-N-[(3R,4S,5S)-1-{(2S)-2-[(1R,2R)-3-{[(2S,3Z)-1,4-diphenylbut-3-en-2-yl]amino}-1-methoxy-2-methyl-3-oxopropyl]pyrrolidin-1-yl}-3-methoxy-5-methyl-1-oxoheptan-4-yl]-N-methyl-L-valinamid

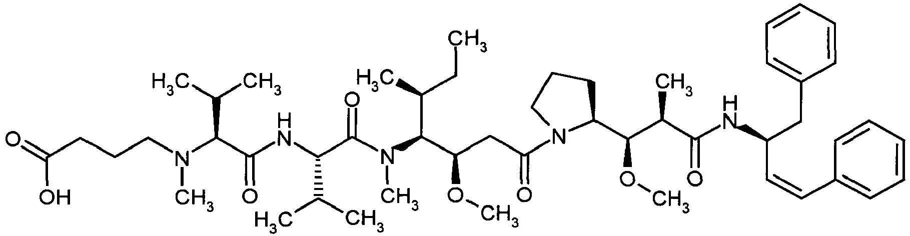

9 mg (10 µmol) *N*-Methyl-L-valyl-N*-*[(3*R*,4*S*,5*S*)-1-{(2*S*)-2-[(1*R*,2*R*)-3-{[(2*S*,3*Z*)-1,4-diphenylbut-3-en-2-yl]amino}-1-methoxy-2-methyl-3-oxopropyl]pyrrolidin-1-yl}-3-methoxy-5-methyl-1-oxo-heptan-4-yl]-*N*-methyl-L-valinamid Trifluoressigsäure-Salz (Intermediat 18) wurden in 0.6 ml Dioxan/Wasser (1:1) gelöst und analog zur Herstellung von Beispiel 1 mit 15%-iger wässriger Lösung von 4-Oxobutansäure in Gegenwart von Natriumcyanoborhydrid umgesetzt. Nach Lyophilisation aus Dioxan wurden 5.6 mg (64% d. Th.) der Titelverbindung in Form eines weißen Feststoffs erhalten.

HPLC (Methode 10): Rₜ = 2.61 min;

LC-MS (Methode 11): Rₜ = 0.94 min; MS (ESIpos): m/z = 877 (M+H)⁺;

HR-MS (Methode 13): m/z = 876.5.

### Beispiel 3

### N-(3-Carboxypropyl)-N-methyl-L-valyl-N-[(3R,4S,5S)-1-{(2S)-2-[(1R,2R)-3-{[(2S,3E)-1,4-diphenylbut-3-en-2-yl]amino}-1-methoxy-2-methyl-3-oxopropyl]pyrrolidin-1-yl}-3-methoxy-5-methyl-1-oxoheptan-4-yl]-N-methyl-L-valinamid

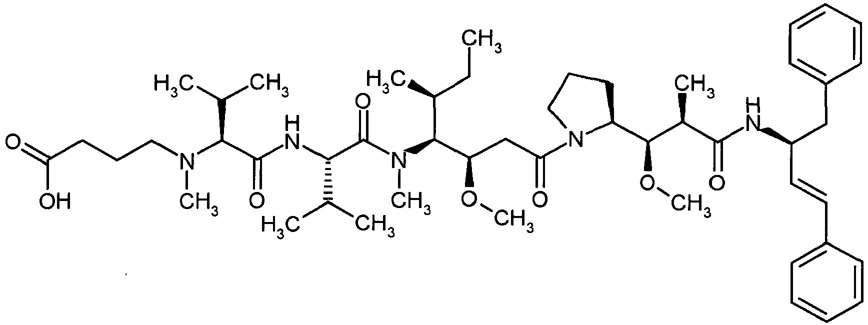

15.5 mg (10 µmol) *N*-Methyl-L-valyl-*N*-[(3*R*,4*S*,5*S*)-1-{(2*S*)-2-[(1*R*,2*R*)-3-{[(2*S*,3*E*)-1,4-diphenyl-but-3-en-2-yl]amino}-1-methoxy-2-methyl-3-oxopropyl]pyrrolidin-1-yl}-3-methoxy-5-methyl-1-oxo-heptan-4-yl]-*N*-methyl-L-valinamid Trifluoressigsäure-Salz (Intermediat 19) wurden in 1.0 ml Dioxan/Wasser (1:1) gelöst und analog zur Herstellung von Beispiel 1 mit 15%-iger wässriger Lösung von 4-Oxobutansäure in Gegenwart von Natriumcyanoborhydrid umgesetzt. Nach Lyophilisation aus Dioxan wurden 10.3 mg (68% d. Th.) der Titelverbindung in Form eines weißen Feststoffs erhalten.

HPLC (Methode 10): Rₜ = 2.59 min;

LC-MS (Methode 11): Rₜ = 0.94 min; MS (ESIpos): m/z = 877 (M+H)⁺;

HR-MS (Methode 13): m/z = 876.6;

¹H-NMR (500 MHz, Dichlormethan-d₂): δ [ppm] = 0.72-1.21 (m, 18H), 1.23-1.47 (m, 3H), 1.51-2.22 (m, 8H), 2.25-2.54 (m, 5H), 2.65-2.86 (m, 2H), 2.90-3.47 (m, 16H), 3.53-4.46 (m, 6H), 4.71-5.27 (m, 4H), 5.46-5.72 (m, 1H), 6.10-6.36 (m, 1H), 6.44-6.67 (m, 2H), 7.03-7.67 (m, 10H), 9.13 (br. s, 1H).

### Beispiel 4

### N-(3-Carboxypropyl)-N-methyl-L-valyl-N-[(3R,4S,5S)-1-{(2S)-2-[(1R,2R)-3-{[(2S)-1-(benzyl-sulfonyl)-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

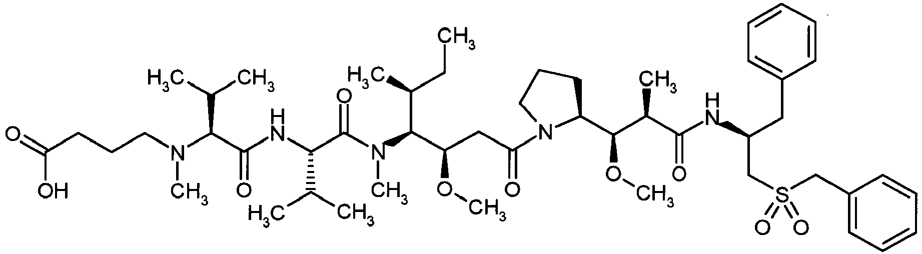

19.3 mg (20 µmol) *N*-Methyl-L-valyl-*N*-[(3*R*,4*S*,5*S*)-1-{(2*S*)-2-[(1*R*,2*R*)-3-{[(2*S*)-1-(benzylsulfonyl)-3-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid Trifluoressigsäure-Salz (Intermediat 20) wurden in 1.2 ml Dioxan/Wasser (1:1) gelöst und analog zur Herstellung von Beispiel 1 mit 15%-iger wässriger Lösung von 4-Oxobutansäure in Gegenwart von Natriumcyanoborhydrid umgesetzt. Nach Lyophilisation aus Dioxan wurden 8.6 mg (45% d. Th.) der Titelverbindung in Form eines weißen Feststoffs erhalten.

LC-MS (Methode 11): Rₜ = 0.85 min; MS (ESIpos): m/z = 943 (M+H)⁺;

HR-MS (Methode 13): m/z = 942.6;

¹H-NMR (500 MHz, Dichlormethan-d₂): δ [ppm] = 0.72-1.23 (m, 18H), 1.26-1.56 (m, 2H), 1.60-1.94 (m, 4H), 1.95-2.17 (m, 3H), 2.22-2.54 (m, 5H), 2.69-2.87 (m, 2H), 2.90-3.27 (m, 11H), 3.31-3.53 (m, 8H), 3.58-4.20 (m, 7H), 4.25-4.54 (m, 3H), 4.59-5.15 (m, 4H), 6.22 (br. s, 1H), 6.97-8.00 (m, 10H), 9.13 (br. s, 1H).

### Beispiel 5

### N-(3-Carboxypropyl)-N-methyl-L-valyl-N-[(3R,4S,5S)-3-methoxy-1-{(2S)-2-[(1R,2R)-1-methoxy-2-methyl-3-oxo-3-{[(1S)-2-phenyl-1-(5-phenyl-1,3,4-oxadiazol-2-yl)ethyl]amino}propyl]pyrrolidin-1-yl}-5-methyl-1-oxoheptan-4-yl]-N-methy1-L-valinamid

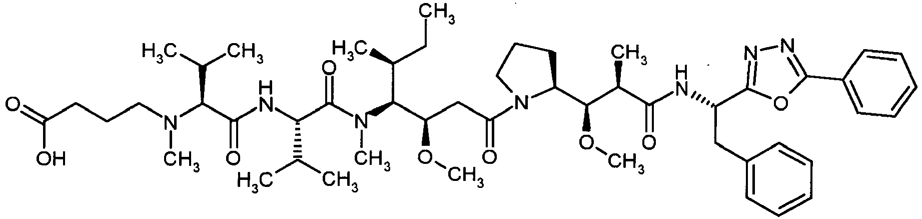

22.4 mg (24 µmol) *N*-Methyl-L-valyl-*N*-[(3*R*,4*S*,5*S*)-3-methoxy-1-{(2*S*)-2-[(1*R*,2*R*)-1-methoxy-2-methyl-3-oxo-3-{[(1*S*)-2-phenyl-1-(5-phenyl-1,3,4-oxadiazol-2-yl)ethyl]amino}propyl]pyrrolidin-1-yl}-5-methyl-1-oxoheptan-4-yl]-*N*-methyl-L-valinamid Trifluoressigsäure-Salz (Intermediat 21) wurden in 1.4 ml Dioxan/Wasser (1:1) gelöst und analog zur Herstellung von Beispiel 1 mit 15%-iger wässriger Lösung von 4-Oxobutansäure in Gegenwart von Natriumcyanoborhydrid umgesetzt. Nach Lyophilisation aus Dioxan wurden 8.2 mg (38% d. Th.) der Titelverbindung in Form eines weißen Feststoffs erhalten.

HPLC (Methode 10): Rₜ = 2.54 min;

LC-MS (Methode 1): Rₜ = 0.94 min; MS (ESIpos): m/z = 919 (M+H)⁺;

HR-MS (Methode 13): m/z = 918.6;

¹H-NMR (500 MHz, Dichlormethan-d₂): δ [ppm] = 0.58-1.21 (m, 20H), 1.25-1.52 (m, 2H), 1.62-2.19 (m, 8H), 2.28-2.50 (m, 5H), 2.64-2.84 (m, 2H), 2.89-3.16 (m, 6H), 3.19-3.52 (m, 10H), 3.59-4.00 (m, 4H), 4.02-4.40 (m, 3H), 4.66-5.13 (m, 3H), 5.61 (d, 1H), 7.32 (d, 5H), 7.49-7.69 (m, 3H), 7.93-8.16 (m, 2H), 9.07 (br. s, 1 H).

### Beispiel 6

### N-(3-Carboxypropyl)-N-methyl-L-valyl-N-[(3R,4S,5S)-3-methoxy-1-{(2S)-2-[(1R,2R)-1-methoxy-2-methyl-3-oxo-3-{[(1R)-2-phenyl-1-(5-phenyl-1,3,4-oxadiazol-2-yl)ethyl]amino}propyl]pyrrolidin-1-yl}-5-methyl-1-oxoheptan-4-yl]-N-methyl-L-valinamid

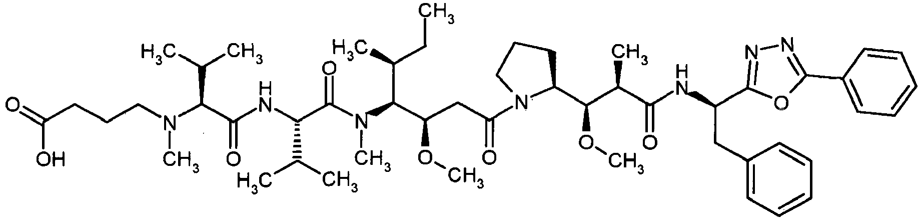

17.1 mg (18 µmol) *N*-Methyl-L-valyl-*N*-[(3*R*,4*S*,5*S*)-3-methoxy-1-{(2*S*)-2-[(1*R*,2*R*)-1-methoxy-2-methyl-3-oxo-3-{[(1*R*)-2-phenyl-1-(5-phenyl-1,3,4-oxadiazol-2-yl)ethyl]amino}propyl]pyrrolidin-1-yl}-5-methyl-1-oxoheptan-4-yl]-*N*-methyl-L-valinamid Trifluoressigsäure-Salz (Intermediat 22) wurden in 1.1 ml Dioxan/Wasser (1:1) gelöst und analog zur Herstellung von Beispiel 1 mit 15%-iger wässriger Lösung von 4-Oxobutansäure in Gegenwart von Natriumcyanoborhydrid umgesetzt. Nach Lyophilisation aus Dioxan wurden 14.8 mg (89% d. Th.) der Titelverbindung in Form eines weißen Feststoffs erhalten.

HPLC (Methode 10): Rₜ = 2.54 min;

LC-MS (Methode 1): Rₜ = 0.92 min; MS (ESIpos): m/z = 919 (M+H)⁺.

### Beispiel 7

### N-(3-Carboxypropyl)-N-methyl-L-valyl-N-[(3R,4S,5S)-1-{(2S)-2-[(1R,2R)-3-{[2-(1H-indol-3-yl)-ethyl]amino}-1-methoxy-2-methyl-3-oxopropyl]pyrrolidin-1-yl}-3-methoxy-5-methyl-1-oxoheptan-4-yl]-N-methyl-L-valinamid

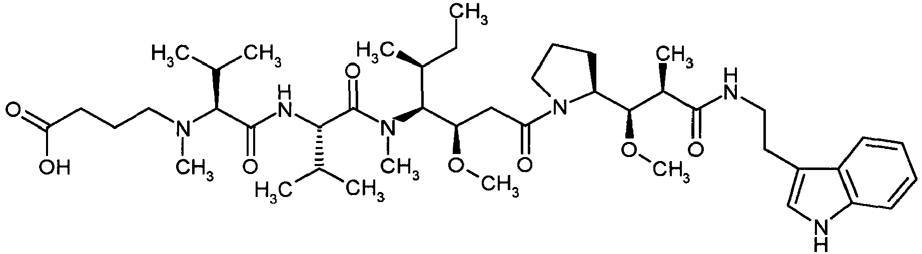

Die Titelverbindung wurde in Analogie zur Synthese von Beispiel 1 durch Umsetzung von 100 mg (119 µmol) *N*-Methyl-L-valyl-*N*-[(3*R*,4*S*,5*S*)-1-{(2*S*)-2-[(1*R*,2*R*)-3-{[2-(1*H*-indol-3-yl)ethyl]-amino}-1-methoxy-2-methyl-3-oxopropyl]pyrrolidin-1-yl}-3-methoxy-5-methyl-1-oxoheptan-4-yl]-*N*-methyl-L-valinamid Trifluoressigsäure-Salz (Intermediat 24) mit 15%-iger wässriger Lösung von 4-Oxobutansäure in Gegenwart von Natriumcyanoborhydrid hergestellt.

Ausbeute: 50 mg (49% d. Th.)

LC-MS (Methode 1): Rₜ = 0.87 min; MS (ESIpos): m/z = 814 (M+H)⁺.

### Beispiel 8

### N-(3-Carboxypropyl)-N-methyl-L-valyl-N-{(3R,4S,5S)-3-methoxy-1-[(2S)-2-{(1R,2R)-1-methoxy-2-methyl-3-oxo-3-[(2-phenylethyl)amino]propyl}pyrrolidin-1-yl]-5-methyl-1-oxoheptan-4-yl}-N-methyl-L-valinamid

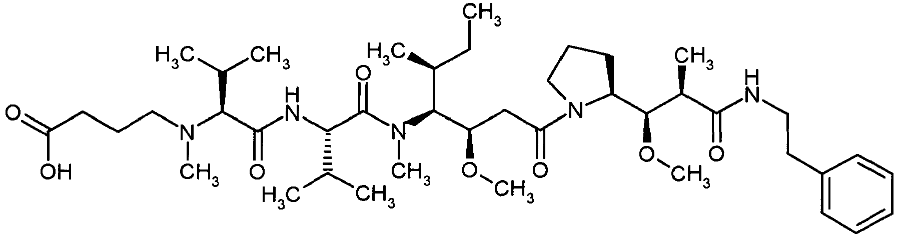

Die Titelverbindung wurde in Analogie zur Synthese von Beispiel 1 durch Umsetzung von 57 mg (71 µmol) *N*-Methyl-L-valyl-*N*-{(3*R*,4*S*,5*S*)-3-methoxy-1-[(2*S*)-2-{(1*R*,2*R*)-1-methoxy-2-methyl-3-oxo-3-[(2-phenylethyl)amino]propyl}pyrrolidin-1-yl]-5-methyl-1-oxoheptan-4-yl}-*N*-methyl-L-valinamid Trifluoressigsäure-Salz (Intermediat 25) mit 15%-iger wässriger Lösung von 4-Oxobutansäure in Gegenwart von Natriumcyanoborhydrid hergestellt.

Ausbeute: 10 mg (19% d. Th.)

LC-MS (Methode 1): Rₜ = 0.85 min; MS (ESIpos): m/z = 775 (M+H)⁺.

### Beispiel 9

### N-(3-Carboxypropyl)-N-methyl-L-valyl-N-[(3R,4S,5S)-1-{(2S)-2-[(1R,2R)-3-{[(1S,2R)-1-hydroxy-1-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

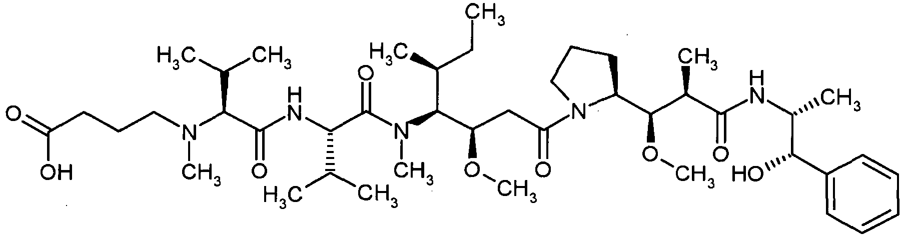

Die Titelverbindung wurde in Analogie zur Synthese von Beispiel 1 durch Umsetzung von 57 mg (71 µmol) *N*-Methyl-L-valyl-*N*-[(3*R*,4*S*,5*S*)-1-{(2*S*)-2-[(1*R*,2*R*)-3-{[(1*S*,2*R*)-1-hydroxy-1-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid Trifluoressigsäure-Salz [Herstellung analog zu Intermediat 17 durch Kupplung von Intermediat 7 mit (1*S*,2*R*)-(+)-Norephedrin und anschließende Entschützung mit Trifluoressigsäure] mit 15%-iger wässriger Lösung von 4-Oxobutansäure in Gegenwart von Natriumcyanoborhydrid hergestellt.

Ausbeute: 94 mg (84% d. Th.)

LC-MS (Methode 1): Rₜ = 0.79 min; MS (ESIpos): m/z = 805 (M+H)⁺.

### Beispiel 10

### N-(3-Carboxypropyl)-N-methyl-L-valyl-N-[(3R,4S,5S)-3-methoxy-1-{(2S)-2-[(1R,2R)-1-methoxy-3-({(2S,3E)-4-[4-(methoxycarbonyl)phenyl]-1-phenylbut-3-en-2-yl}amino)-2-methyl-3-oxopropyl]-pyrrolidin-1-yl}-5-methyl-1-oxoheptan-4-yl]-N-methyl-L-valinamid

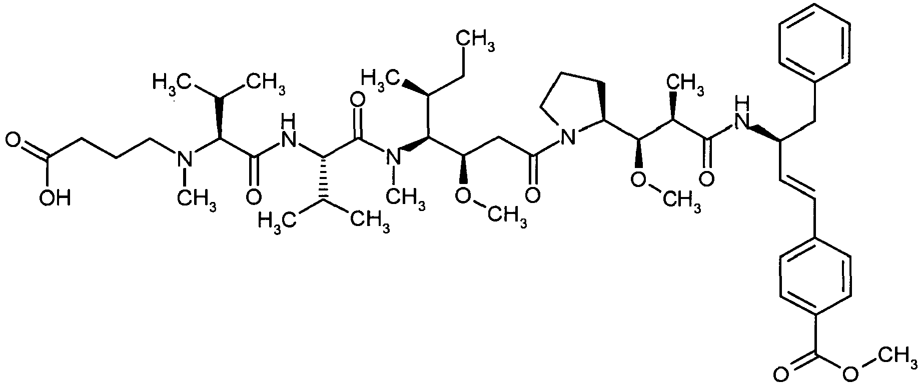

Die Titelverbindung wurde in Analogie zur Synthese von Beispiel 1 durch Umsetzung von 45 mg (47 µmol) *N*-Methyl-L-valyl-*N*-[(3*R*,4*S*,5*S*)-3-methoxy-1-{(2*S*)-2-[(1*R*,2*R*)-1-methoxy-3-({(2*S*,3*E*)-4-[4-(methoxycarbonyl)phenyl]-1-phenylbut-3-en-2-yl}amino)-2-methyl-3-oxopropyl]pyrrolidin-1-yl}-5-methyl-1-oxoheptan-4-yl]-*N*-methyl-L-valinamid Trifluoressigsäuresalz (Intermediat 23) mit 15%-iger wässriger Lösung von 4-Oxobutansäure in Gegenwart von Natriumcyanoborhydrid hergestellt.

Ausbeute: 33.9 mg (78% d. Th.)

LC-MS (Methode 1): Rₜ = 1.02 min; MS (ESIneg): m/z = 933 (M-H)⁻.

### Beispiel 11

### N-(3-Carboxypropyl)-N-methyl-L-valyl-N-[(3R,4S,5S)-1-{(2S)-2-[(1R,2R)-3-{[(2S,3E)-4-(4-carboxyphenyl)-1-phenylbut-3-en-2-yl]amino}-1-methoxy-2-methyl-3-oxopropyl]pyrrolidin-1-yl}-3-methoxy-5-methyl-1-oxoheptan-4-yl]-N-methyl-L-valinamid

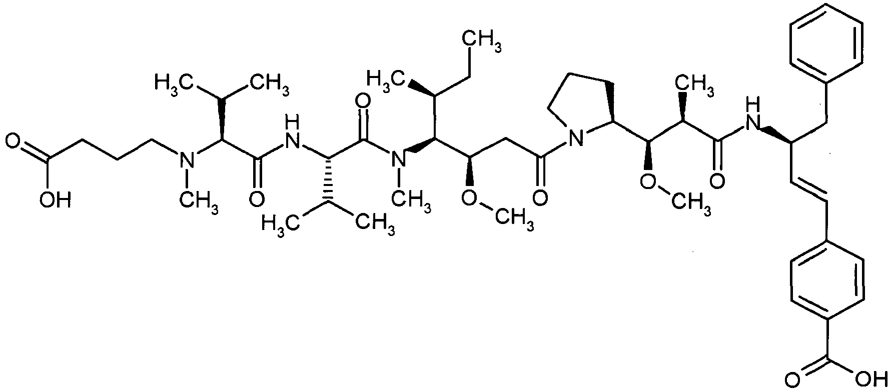

33.9 mg (36 µmol) *N*-(3-Carboxypropyl)-*N*-methyl-L-valyl-*N*-[(3*R*,4*S*,5*S*)-3-methoxy-1-{(2*S*)-2-[(1*R*,2*R*)-1-methoxy-3-({(2*S*,3*E*)-4-[4-(methoxycarbonyl)phenyl]-1-phenylbut-3-en-2-yl}amino)-2-methyl-3-oxopropyl]pyrrolidin-1-yl}-5-methyl-1-oxoheptan-4-yl]-*N*-methyl-L-valinamid (Beispiel 10) wurden in 1.1 ml THF/Wasser (1:1) vorgelegt und mit 3.5 mg (145 µmol) Lithiumhydroxid versetzt. Das Reaktionsgemisch wurde über Nacht bei RT gerührt, anschließend durch Zugabe von 1 N Salzsäure angesäuert und zweimal mit je 10 ml Ethylacetat extrahiert. Die vereinigten organischen Phasen wurden über Magnesiumsulfat getrocknet und eingeengt. Es wurden 18.3 mg (84% Reinheit, 46% d. Th.) der Titelverbindung erhalten.

LC-MS (Methode 9): R, = 4.98 min; MS (ESIneg): m/z = 919 (M-H)⁻.

### B. Bewertung der biologischen Wirksamkeit

Die biologische Aktivität der erfindungsgemäßen Verbindungen kann durch *in vitro-* und *in vivo-*Untersuchungen, wie sie dem Fachmann bekannt sind, nachgewiesen werden. Beispielsweise können die pharmakologischen und pharmakokinetischen Eigenschaften der erfindungsgemäßen Verbindungen mit Hilfe der nachstehend beschriebenen Assays bestimmt werden:

### B-1. Bestimmung der antiproliferativen Wirkung an der HT29wt-Zelllinie:

Eine definierte Zellzahl der humanen Colonkarzinom-Zelllinie HT29wt (Wildtyp) wurde in einer 96-well-Mikrotiterplatte im Vollmedium (10% FCS-RPMI) ausgesät (2 500 Zellen/well) und über Nacht bei 37°C / 5% CO₂ inkubiert. Nach 18 h wurde das Aussaatmedium durch frisches Medium mit 10% FCS ersetzt. Die Behandlung startete mit Zugabe der jeweiligen Testsubstanz. Von den zu untersuchenden Substanzen wurden Dosis-Wirkungskurven in einem Konzentrationsbereich von 10⁻⁵ M bis 10⁻¹⁴ M (1:10-Verdünnungsreihe) bestimmt. Es wurden Inkubationszeiten von 48 h bis 96 h gewählt. Die Detektion der Proliferation erfolgte mit Hilfe des MTT-Assays (ATCC, Manassas, Virginia, USA, Katalog-Nr. 30-1010K). Nach Ablauf der gewählten Inkubationszeit wurde das MTT-Reagens für 4 h mit den Zellen inkubiert, bevor durch Zugabe des Detergens die Lyse der Zellen über Nacht erfolgte. Die Detektion des gebildeten Farbstoffs erfolgte bei 570 nm. Die Proliferation nicht mit Testsubstanz, aber ansonsten identisch behandelter Zellen wurde als 100%-Wert definiert. Die aus diesem Test gewonnenen Daten repräsentieren Dreifachbestimmungen, und es wurden mindestens zwei unabhängige Experimente durchgeführt.

In der folgenden Tabelle 1 sind die IC₅₀-Werte repräsentativer Ausführungsbeispiele aus diesem Assay aufgeführt:

**Tabelle 1**

| **Ausführungsbeispiel** | **IC₅₀ [nM]** |
|---|---|
| 1 | 15 |
| 2 | 3.3 |
| 3 | 0.3 |
| 4 | 1.1 |
| 5 | 0.1 |
| 6 | 0.1 |
| 8 | 0.5 |
| 9 | 6 |
| 11 | 4.5 |

Im Vergleich hierzu weist Monomethylauristatin F (MMAF) in diesem Test einen IC₅₀-Wert von 10 nM auf.

### B-2. Bestimmung des Einflusses auf die Tubulinpolymerisation:

Krebszellen sind entartete Zellen, die häufig auch durch eine gesteigerte Zellteilung zu einer Tumorbildung führen. Microtubuli bilden die Spindelfasern des Spindelapparates und sind ein essentieller Bestandteil des Zellzyklus. Der geregelte Auf- und Abbau von Microtubuli ermöglicht die genaue Aufteilung der Chromosomen auf die Tochterzellen und stellt einen kontinuierlich dynamischen Prozess dar. Eine Störung dieser Dynamik führt zu einer fehlerhaften Zellteilung und letztlich zum Zelltod. Die gesteigerte Zellteilung von Krebszellen macht diese jedoch auch besonders empfänglich gegenüber Spindelfasergiften, die einen festen Bestandteil der Chemotherapie darstellen. Spindelfasergifte wie Paclitaxel oder Epothilon führen zu einer stark erhöhten Polymerisationsgeschwindigkeit der Microtubuli, während Vinca-Alkaloide oder auch Monomethylauristatin E (MMAE) zu einer stark reduzierten Polymerisationsgeschwindigkeit der Microtubuli führen. In beiden Fällen ist die notwendige Dynamik des Zellzyklus empfindlich gestört. Die im Rahmen der vorliegenden Erfindung untersuchten Verbindungen führen zu einer reduzierten Polymerisationsgeschwindigkeit der Microtubuli.

Zur Untersuchung der Tubulinpolymerisation wurde das "Fluorescence-based Microtubule Polymerisation Assay Kit" der Firma Cytoskeleton (Denver, Colorado, USA; Bestellnummer: BK011) verwendet. Bei diesem Assay wird unpolymerisiertem Tubulin GTP zugesetzt, womit die Polymerisation spontan stattfinden kann. Der Assay beruht auf der Bindung des Fluorophors 4',6-Diamidino-2-phenylindol (DAPI) an Tubulin. Freies und gebundenes DAPI können aufgrund unterschiedlicher Emissionsspektra differenziert werden. Da DAPI eine deutlich höhere Affinität gegenüber polymerisiertem im Vergleich zu nicht-polymerisiertem Tubulin zeigt, läßt sich die Tubulinpolymerisation über die Zunahme der Fluoreszenz gebundener DAPI-Fluorophore verfolgen.

Zur Durchführung dieses Assays wurden die in DMSO gelösten Testsubstanzen von ihrer Ausgangskonzentration von 10 mM auf 1 µM in Wasser verdünnt. Neben der Puffer-Kontrolle wurde als Assay-Kontrolle auch polymerisationssteigernd Paclitaxel und andererseits polymerisationshemmend Vinblastin mitgeführt. Für die Messung wurden 96-well-Lochplatten mit halber Bodenfläche verwendet. Die Kinetik der Tubulinpolymerisation wurde 1 h lang bei 37°C in einem Fluorimeter verfolgt. Die Anregungswellenlänge betrug 355 nm, die Emission wurde bei 460 nm verfolgt. Für den Bereich des linearen Anstiegs innerhalb der ersten 10 Minuten wurde die Fluoreszenzänderung pro Minute (ΔF/min) berechnet, welche die Polymerisationsgeschwindigkeit der Microtubuli darstellt. Die Potenz der Testsubstanzen wurde anhand der jeweiligen Reduktion der Polymerisationsgeschwindigkeit quantifiziert.

### B-3. Bestimmung der Plasma-Stabilität in vitro:

### Methode A:

1 mg der jeweiligen Testsubstanz wurde in 0.5 ml Acetonitril/DMSO (9:1) gelöst. Von dieser Lösung wurden 20 µl abgenommen und zu 1 ml 37°C warmen Ratten- bzw. Humanplasma gegeben (Plasma von männlichen Wistar-Ratten mit Li-Heparin, Fa. Harlan & Winkelmann bzw. humanes Leukozyten-depletiertes Frischplasma aus Vollblutentnahme). Aus dieser kräftig geschüttelten Plasmalösung wurden sofort nach Zugabe der Probe (Anfangswert als Bezugsgröße) und dann nach 5, 10, 30, 60, 120, 180 und 240 Minuten sowie gegebenenfalls nach 24 Stunden jeweils 100 µl-Aliquote entnommen und in 300 µl Acetonitril gegeben. Die ausgefällten Plasmaproteine wurden für 10 Minuten bei 5000 U/min abzentrifugiert und 30 µl des Überstandes per HPLC auf seinen Gehalt an unveränderter Testsubstanz analysiert. Quantifiziert wurde über die Flächenprozente der entsprechenden Peaks.

### HPLC-Methode bei Rattenplasma:

Instrument: Agilent 1200 mit DAD, binärer Pumpe, Autosampler, Säulenofen und Thermostat; Säule: Kromasil 100 C18, 250 mm x 4 mm, 5 µm; Säulentemperatur: 45°C; Eluent A: 5 ml Perchlorsäure / L Wasser, Eluent B: Acetonitril; Gradient: 0-8 min 98% A, 2% B; 8-15 min 56% A, 44% B; 15-20 min 10% A, 90% B; 20-21 min 10% A, 90% B; 21-23 min 98% A, 2% B; 23-25 min 98% A, 2% B; Flussrate: 2 ml/min; UV-Detektion: 220 nm.

### HPLC-Methode bei Humanplasma:

Instrument: Agilent 1100 mit DAD, binärer Pumpe, Autosampler, Säulenofen und Thermostat; Säule: Kromasil 100 C18, 250 mm x 4 mm, 5 µm; Säulentemperatur: 45°C; Eluent A: 5 ml Perchlorsäure / L Wasser, Eluent B: Acetonitril; Gradient: 0-3 min 98% A, 2% B; 3-10 min 65% A, 35% B; 10-15 min 40% A, 60% B; 15-21 min 10% A, 90% B; 21-22 min 10% A, 90% B; 22-24 min 98% A, 2% B; 24-26 min 98% A, 2% B; Flussrate: 2 ml/min; UV-Detektion: 220 nm.

### Methode B:

Die jeweilige Testsubstanz wurde in Ratten- bzw. Humanplasma bei 37°C über einen Zeitraum von 5 h unter leichtem Rühren inkubiert. Zu verschiedenen Zeitpunkten (0, 2, 5, 10, 20, 30, 60, 120, 180 und 300 Minuten) wurde jeweils ein 100 µl-Aliquot entnommen. Nach Zugabe von internem Standard (10 µl) wurden die Proteine durch Zugabe von 200 µl Acetonitril gefällt und das Gemisch in einer Eppendorf-Zentrifuge für 5 Minuten zentrifugiert. Nach Zugabe von 150 µl Ammoniumacetat-Puffer pH 3 zu 150 µl des Überstandes wurde der Gehalt an unveränderter Testsubstanz mittels LC/MSMS analysiert.

### B-4. Bestimmung der Zell-Permeabilität:

Die Zell-Permeabilität einer Substanz kann mittels *in vitro*-Testung in einem Flux-Assay unter Verwendung von Caco-2-Zellen untersucht werden [M.D. Troutman und D.R. Thakker, Pharm. Res. 20 (8), 1210-1224 (2003)]. Hierzu wurden die Zellen auf 24-Loch-Filterplatten für 15-16 Tage kultiviert. Zur Bestimmung der Permeation wurde die jeweilige Testsubstanz in einem HEPES-Puffer entweder apikal (A) oder basal (B) auf die Zellen gegeben und für 2 h inkubiert. Nach 0 h und nach 2 h wurden Proben aus den Cis- und Transkompartimenten entnommen. Die Proben wurden mittels HPLC (Agilent 1200, Böblingen, Deutschland) unter Verwendung von reverse phase-Säulen aufgetrennt. Das HPLC-System war über ein Turbo Ion Spray-Interface an ein Triple Quadropol-Massenspektrometer API 4000 (Applied Biosystems Applera, Darmstadt, Deutschland) gekoppelt. Die Permeabilität wurde anhand eines Pₐₚₚ-Wertes bewertet, welcher mittels der von Schwab *et al.* publizierten Formel berechnet wurde [D. Schwab et al., J. Med. Chem. 46, 1716-1725 (2003)]. Eine Substanz wurde als aktiv transportiert klassifiziert, wenn das Verhältnis von Pₐₚₚ (B-A) zu Pₐₚₚ (A-B) > 2 oder <0.5 war.

Von entscheidender Bedeutung für Toxophore, die intrazellulär freigesetzt werden, ist die Permeabilität von B nach A [Pₐₚₚ (B-A)]: Je niedriger diese Permeabilität ist, desto länger ist die Verweilzeit der Substanz in der Zelle nach intrazellulärer Freisetzung und damit auch die Zeit, die für eine Interaktion mit dem biochemischen Target (hier: Tubulin) zur Verfügung steht.

In der folgenden Tabelle 2 sind Permeabilitätsdaten repräsentativer Ausführungsbeispiele aus diesem Assay aufgeführt:

**Tabelle 2**

| **Ausführungsbeispiel** | **Pₐₚₚ (B-A) [nm/s]** |
|---|---|
| 2 | 157 |
| 3 | 179 |
| 4 | 19 |
| 5 | 29 |
| 6 | 45 |
| 7 | 11 |
| 8 | 10 |
| 9 | 2 |
| 11 | 2 |

Im Vergleich hierzu weisen Monomethylauristatin E (MMAE) und Monomethylauristatin F (MMAF) in diesem Test einen Pₐₚₚ (B-A)-Wert von 89 nm/s beziehungsweise 73 nm/s auf.

### B-5. Bestimmung der Substrateigenschaften für P-Glycoprotein (P-gp)

Viele Tumorzellen exprimieren Transporterproteine für Wirkstoffe, was häufig mit einer Resistenzentwicklung gegenüber Cytostatika einhergeht. Substanzen, die keine Substrate von solchen Transporterproteinen wie beispielsweise P-Glycoprotein (P-gp) oder BCRP sind, könnten somit ein verbessertes Wirkprofil aufzeigen.

Die Substrateigenschaften einer Substanz für P-gp (ABCB1) wurden mittels eines Flux-Assays unter Verwendung von LLC-PK1-Zellen, die P-gp überexprimieren (L-MDR1-Zellen), bestimmt [A.H. Schinkel et al., J. Clin. Invest. 96, 1698-1705 (1995)]. Hierzu wurden die LLC-PK1- oder L-MDR1-Zellen auf 96-Loch-Filterplatten für 3-4 Tage kultiviert. Zur Bestimmung der Permeation wurde die jeweilige Testsubstanz allein oder in Gegenwart eines Inhibitors (wie z.B. Ivermectin oder Verapamil) in einem HEPES-Puffer entweder apikal (A) oder basal (B) auf die Zellen gegeben und für 2 h inkubiert. Nach 0 h und nach 2 h wurden Proben aus den Cis- und Transkompartimenten entnommen. Die Proben wurden mittels HPLC unter Verwendung von reverse phase-Säulen aufgetrennt. Das HPLC-System war über ein Turbo Ion Spray-Interface an ein Triple Quadropol-Massenspektrometer API 3000 (Applied Biosystems Applera, Darmstadt, Deutschland) gekoppelt. Die Permeabilität wurde anhand eines Pₐₚₚ-Wertes bewertet, welcher mittels der von Schwab *et al.* publizierten Formel berechnet wurde [D. Schwab et al., J. Med. Chem. 46, 1716-1725 (2003)]. Eine Substanz wurde als P-gp-Substrat klassifiziert, wenn das Efflux-Verhältnis Pₐₚₚ (B-A) zu Pₐₚₚ (A-B) > 2 war.

Als weitere Kriterien zur Bewertung der P-gp-Substrateigenschaften können die Efflux-Verhältnisse in L-MDR1- und LLC-PK1-Zellen oder das Efflux-Verhältnis in An- oder Abwesenheit eines Inhibitors miteinander verglichen werden. Wenn sich diese Werte um mehr als einen Faktor 2 unterscheiden, so handelt es sich bei der betreffenden Substanz um ein P-gp-Substrat.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 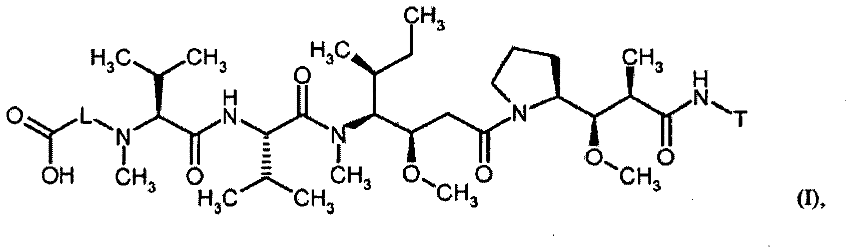 in welcher
L für geradkettiges (C₁-C₁₂)-Alkandiyl steht, das bis zu vierfach mit Methyl substituiert sein kann und in dem (a) zwei Kohlenstoffatome in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können oder (b) bis zu drei zueinander nicht benachbarte CH₂-Gruppen gegen -O- ausgetauscht sein können,
und
T für eine Gruppe der Formel 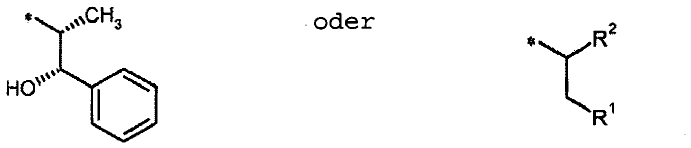 steht, worin
* die Verknüpfüngsstelle mit dem Stickstoffatom kennzeichnet,
R¹ Phenyl oder 1*H*-Indol-3-yl bedeutet,
und
R² Wasserstoff oder eine Gruppe der Formel 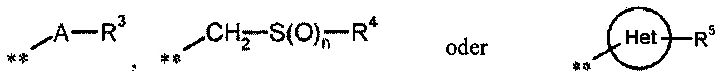 bedeutet, worin
** die jeweilige Verknüpfungsstelle mit dem Rest der betreffenden Gruppe T kennzeichnet,
A geradkettiges (C₁-C₄-Alkandiyl oder geradkettiges (C₂-C₄)-Alkendiyl darstellt,
R³ Phenyl, das mit (C₁-C₄-Alkoxycarbonyl oder Carboxyl substituiert sein kann, darstellt,
n die Zahl 0, 1 oder 2 darstellt,
R⁴ Phenyl, Benzyl oder 2-Phenylethyl, welche jeweils in der Phenylgruppe mit (C₁-C₄-Alkoxycarbonyl oder Carboxyl substituiert sein können, darstellt,
Het einen divalenten 5-gliedrigen Heteroaryl-Ring mit bis zu drei Ring-Heteroatomen aus der Reihe N, O und/oder S darstellt,
und
R⁵ (C₃-C₆)-Cycloalkyl, Phenyl oder (C₁-C₄)-Alkyl, das mit Phenyl substituiert sein kann, darstellt,
wobei die genannten Phenylgruppen ihrerseits mit (C₁-C₄)-Alkoxycarbonyl oder Carboxyl substituiert sein können,
sowie ihre Salze, Solvate und Solvate der Salze.

2. Verbindung der Formel (I) nach Anspruch 1, in welcher
L für geradkettiges (C₁-C₈)-Alkandiyl steht, in dem (a) zwei Kohlenstoffatome in 1,3- oder 1,4-Relation zueinander unter Einbezug des einen beziehungsweise der beiden zwischen ihnen liegenden Kohlenstoffatome zu einem Phenyl-Ring verbrückt sein können oder (b) bis zu zwei zueinander nicht benachbarte CH₂-Gruppen gegen -O-ausgetauscht sein können,
und
T für eine Gruppe der Formel 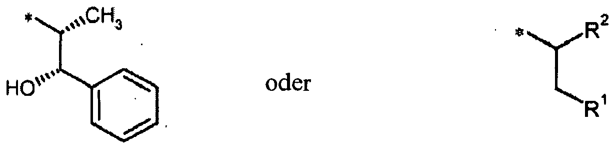 steht, worin
* die Verknüpfungsstelle mit dem Stickstoffatom kennzeichnet,
R¹ Phenyl oder 1*H*-Indol-3-yl bedeutet,
und
R² Wasserstoff oder eine Gruppe der Formel 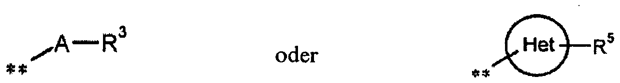 bedeutet, worin
** die Verknüpfungsstelle mit dem Rest der betreffenden Gruppe T kennzeichnet,
A Ethen-1,2-diyl oder Propenl,3-diyl darstellt,
R³ Phenyl, das mit (C₁-C₄)-Alkoxycarbonyl oder Carboxyl substituiert sein kann, darstellt,
Het einen divalenten 5-gliedrigen Heteroaryl-Ring ausgewählt aus der Reihe Pyrazolyl, Imidazolyl, 1,3-Oxazolyl, 1,3-Thiazolyl, 1,2,4- Oxadiazolyl und 1,3,4-Oxadiazolyl darstellt,
und
R⁵ Phenyl, das mit (C₁-C₄)-Alkoxycarbonyl oder Carboxyl substituiert sein kann, darstellt,
sowie ihre Salze, Solvate und Solvate der Salze.

3. Verbindung der Formel (I) nach Anspruch 1 oder 2, in welcher
L für geradkettiges (C₁-C₆)-Alkandiyl steht,
und
T für eine Gruppe der Formel 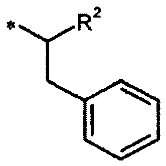 steht, worin
* die Verknüpfungsstelle mit dem Stickstoffatom kennzeichnet
und
R² Wasserstoff oder eine Gruppe der Formel 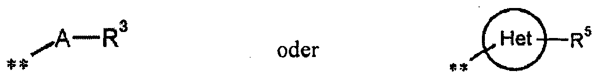 bedeutet, worin
** die Verknüpfungsstelle mit dem Rest der betreffenden Gruppe T kennzeichnet,
A Ethen-1,2-diyl darstellt,
R³ Phenyl, das mit Methoxycarbonyl oder Carboxyl substituiert sein kann, darstellt,
Het 1,3,4-Oxadiazol-2,5-diyl darstellt,
und
R⁵ Phenyl, das mit Methoxycarbonyl oder Carboxyl substituiert sein kann, darstellt,
sowie ihre Salze, Solvate und Solvate der Salze.

4. Verbindung nach Anspruch 1, wobei die Verbindung ausgewählt ist aus der Gruppe bestehend aus: 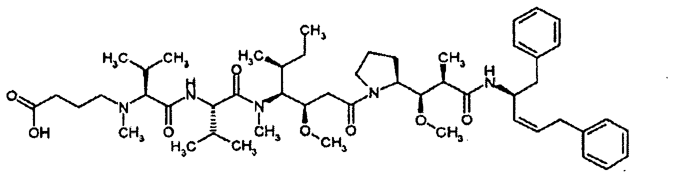 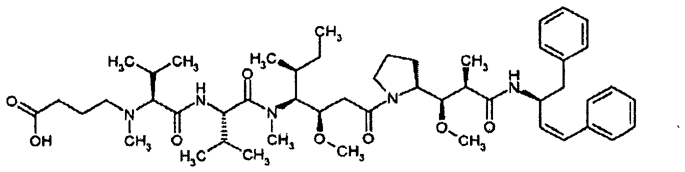 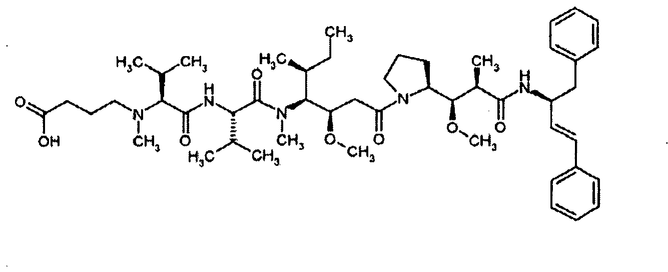 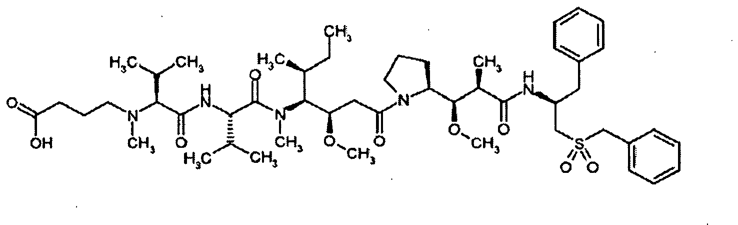 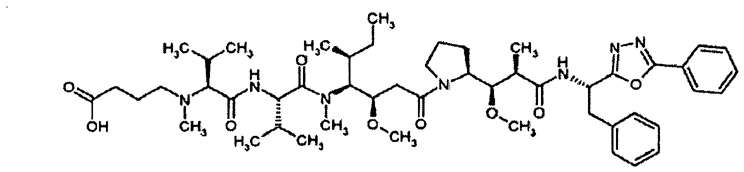 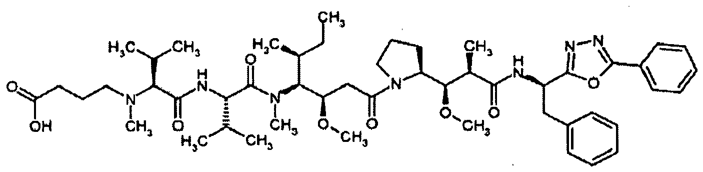 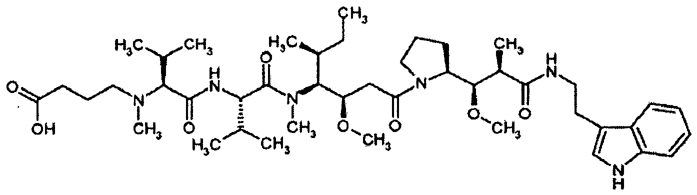 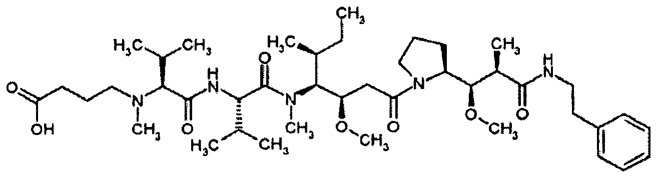 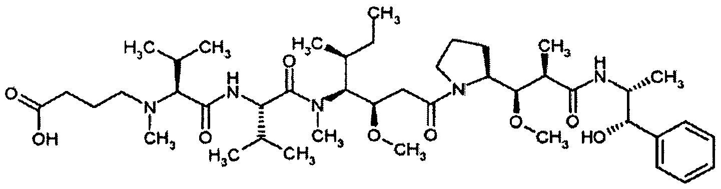 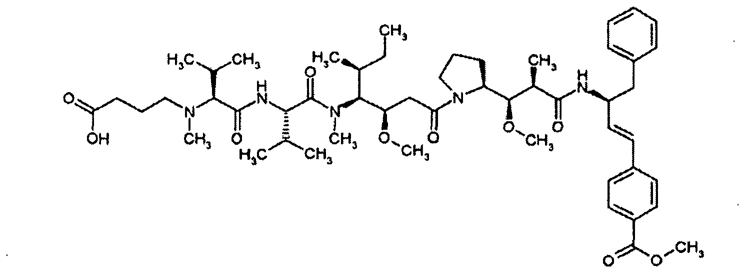 und 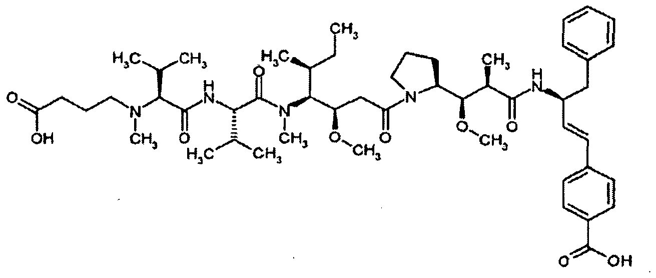 sowie ihre Salze, Solvate und Solvate der Salze.

5. Verfahren zur Herstellung einer Verbindung der Formel (I), wie in den Ansprüchen 1 bis 4 definiert, **dadurch gekennzeichnet, dass** man eine Verbindung der Formel (II) 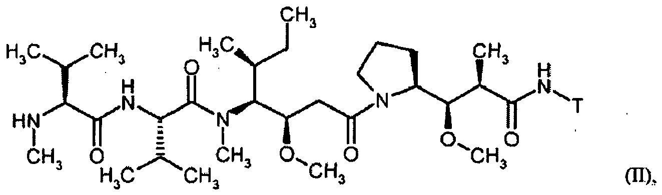 in welcher T die in den Ansprüchen 1 bis 3 angegebenen Bedeutungen hat, in einem inerten Lösungsmittel entweder
[A] durch baseninduzierte Alkylierung mit einer Verbindung der Formel (III) 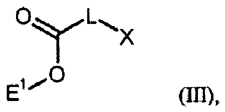 in welcher L die in den Ansprüchen 1 bis 3 angegebene Bedeutung hat,
E¹ für Wasserstoff, (C₁-C₄)-Alkyl oder Benzyl steht,
und
X für eine Fluchtgruppe wie beispielsweise Chlorid, Bromid, Iodid, Mesylat, Triflat oder Tosylat steht, zu einer Verbindung der Formel (IV) 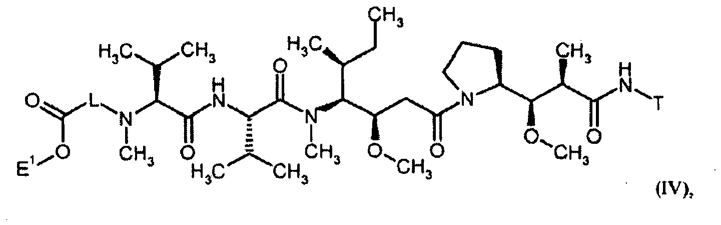
in welcher E¹, L und T die oben angegebenen Bedeutungen haben,
umsetzt und anschließend im Fall, dass E¹ für (C₁-C₄)-Alkyl oder Benzyl steht, diesen Ester-Rest nach üblichen Methoden abspaltet, so dass ebenso wie im Fall, dass E¹ in (III) für Wasserstoff steht, die erfindungsgemäße Carbonsäure der
Formel (I) 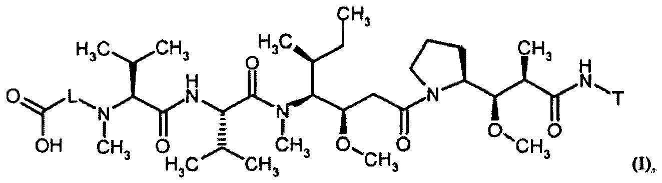 in welcher L und T die oben angegebenen Bedeutungen haben, erhalten wird,
oder
[B] durch Umsetzung mit einer Verbindung der Formel (V) 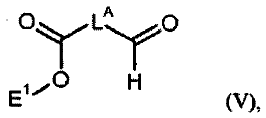 in welcher
E¹ für Wasserstoff, (C₁-C₄)-Alkyl oder Benzyl steht,
und
L^{A} die in den Ansprüchen 1 bis 3 definierte Bedeutung von L hat, jedoch in der Alkyl-Kettenlänge um eine CH₂-Einheit verkürzt ist,
in Gegenwart eines geeigneten Reduktionsmittels in eine Verbindung der Formel (VI) 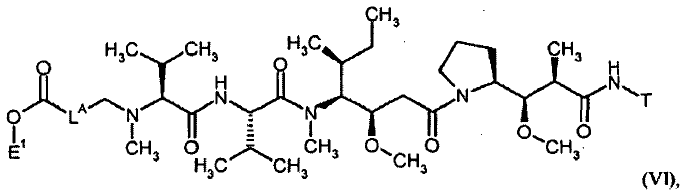 in welcher E¹, L^{A} und T die oben angegebenen Bedeutungen haben,
überführt und anschließend im Fall, dass E¹ für (C₁-C₄)-Alkyl oder Benzyl steht, diesen Ester-Rest nach üblichen Methoden abspaltet, so dass ebenso wie im Fall, dass E¹ in (V) für Wasserstoff steht, die erfindungsgemäße Carbonsäure der Formel (I-A) 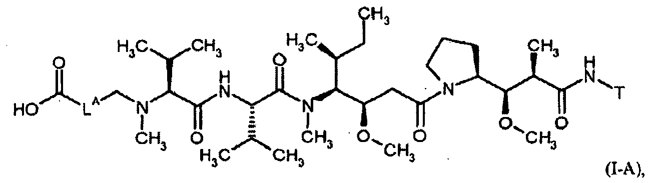 in welcher L^{A} und T die oben angegebenen Bedeutungen haben, erhalten wird,
und die resultierenden Verbindungen der Formel (I) bzw. (I-A) gegebenenfalls in ihre Enantiomere und/oder Diastereomere trennt und/oder mit den entsprechenden (*i*) Lösungsmitteln und/oder (*i*) Basen oder Säuren zu ihren Solvaten, Salzen und/oder Solvaten der Salze umsetzt.

6. Verbindung, wie in einem der Ansprüche 1 bis 4 definiert, zur Verwendung für die Behandlung und/oder Prävention von Krankheiten.

7. Verbindung, wie in einem der Ansprüche 1 bis 4 definiert, zur Verwendung in einem Verfahren zur Behandlung und/oder Prävention von Krebs- und Tumorerkrankungen.

8. Arzneimittel enthaltend eine Verbindung, wie in einem der Ansprüche 1 bis 4 definiert, in Kombination mit einem oder mehreren inerten, nicht-toxischen, pharmazeutisch geeigneten Hilfsstoffen.

9. Arzneimittel enthaltend eine Verbindung, wie in einem der Ansprüche 1 bis 4 definiert, in Kombination mit einem oder mehreren weiteren Wirkstoffen.

10. Arzneimittel nach Anspruch 8 oder 9 zur Verwendung für die Behandlung und/oder Prävention von Krebs- und Tumorerkrankungen.

11. Antiproliferatives Konjugat, in dem eine Verbindung von einem oder mehreren der Ansprüche 1-4 mit einem Protein verbunden ist.

12. Antiproliferatives Konjugat nach Anspruch 12, wobei das Protein ein Antikörper ist.

## Claims

1. Compound of the formula (I) 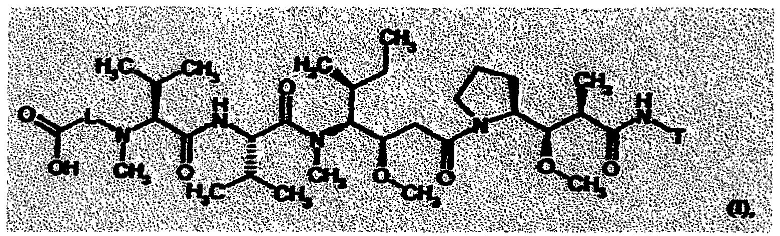 in which
L represents straight-chain (C₁-C₁₂) alkanediyl which may be substituted up to four times with methyl and in which (a) two carbon atoms in 1,2 relation, 1,3 relation or 1,4 relation to one another including the carbon atoms optionally lying between them may be bridged to form a (C₃-C₆) cycloalkyl ring or a phenyl ring or (b) up to three CH₂ groups not adjacent to one another may be exchanged for -O-,
and
T represents a group of the formula 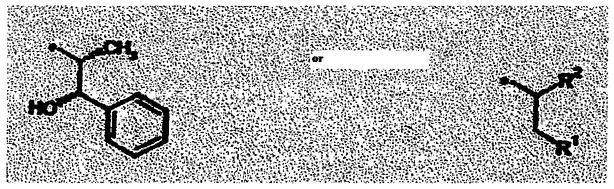 wherein
* indicates the point of attachment with the nitrogen atom,
R¹ denotes phenyl or 1*H*-indol-3-yl,
and
R² denotes hydrogen or a group of the formula 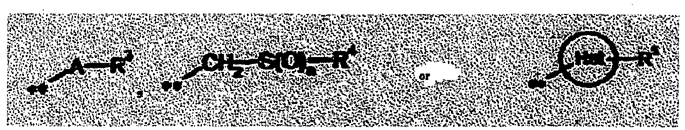 wherein
** indicates the respective point of attachment with the radical of the relevant group T,
A represents straight-chain (C₁-C₄ alkanediyl or straight-chain (C₂-C₄) alkenediyl,
R³ represents phenyl which may be substituted with (C₁-C₄ alkoxycarbonyl or carboxyl,
n represents the number 0, 1 or 2,
R⁴ represents phenyl, benzyl or 2-phenylethyl which may be substituted in each case in the phenyl group with (C₁-C₄ alkoxycarbonyl or carboxyl,
Het represents a divalent 5-membered heteroaryl ring having up to three ring hetero atoms from the series N, O and/or S,
and
R⁵ represents (C₃-C₆) cycloalkyl, phenyl or (C₁-C₄) alkyl which may be substituted with phenyl,
wherein the said phenyl groups in turn may be substituted with (C₁-C₄) alkoxycarbonyl or carboxyl,
as well as its salts, solvates and solvates of the salts.

2. Compound of the formula (I) according to claim 1, in which
L represents straight-chain (C₁-C₆) alkanediyl in which (a) two carbon atoms in 1,3 relation or 1,4 relation to one another including one or both carbon atoms lying between them may be bridged to form a phenyl ring or (b) up to two CH₂ groups not adjacent to one another may be exchanged for -O-,
and
T represents a group of the formula 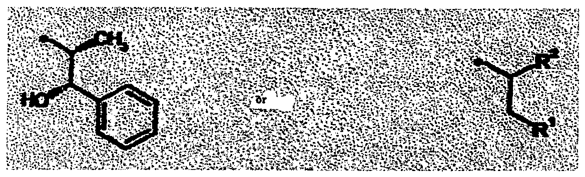 wherein
* indicates the point of attachment with the nitrogen atom,
R¹ denotes phenyl or 1H-indol-3-yl,
and
R² denotes hydrogen or a group of the formula 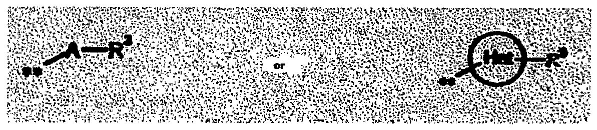 wherein
** indicates the point of attachment with the radical of the relevant group T,
A represents ethene-1,2-diyl or propene-1,3-diyl,
R³ represents phenyl which may be substituted with (C₁-C₄) alkoxycarbonyl or carboxyl,
Het represents a divalent 5-membered heteroaryl ring selected from the series pyrazolyl, imidazolyl, 1,3-oxazolyl, 1,3-thiazolyl, 1,2,4-oxadiazolyl and 1,3,4-oxadiazolyl,
and
R⁵ represents phenyl which may be substituted with (C₁-C₄) alkoxycarbonyl or carboxyl,
as well as its salts, solvates and solvates of the salts.

3. Compound of the formula (I) according to claim 1 or 2, in which
L represents straight-chain (C₁-C₆) alkanediyl,
and
T represents a group of the formula 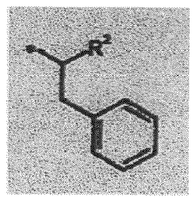 wherein
* indicates the point of attachment with the nitrogen atom,
and
R² denotes hydrogen or a group of the formula 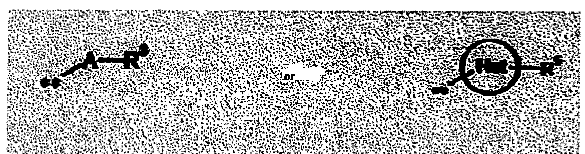 wherein
** indicates the point of attachment with the radical of the relevant group T,
A represents ethene-1,2-diyl,
R³ represents phenyl which may be substituted with methoxycarbonyl or carboxyl,
Het represents 1,3,4-oxadiazol-2,5-diyl,
and
R⁵ represents phenyl which may be substituted with methoxycarbonyl or carboxyl,
as well as its salts, solvates and solvates of the salts.

4. Compound according to claim 1, wherein the compound is selected from the group consisting of: 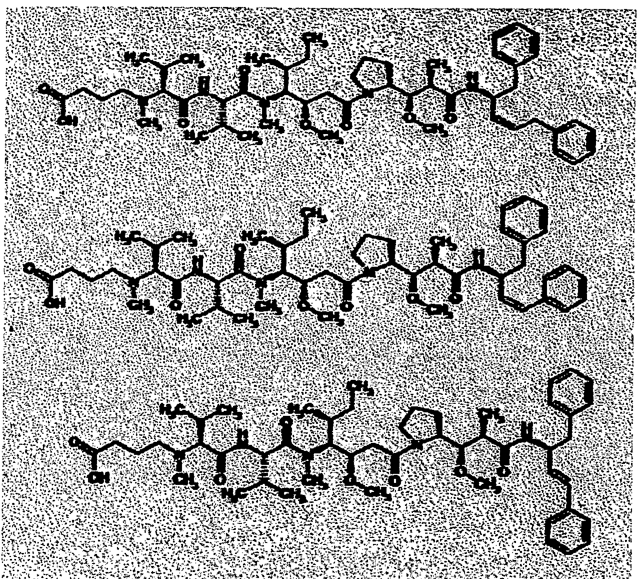 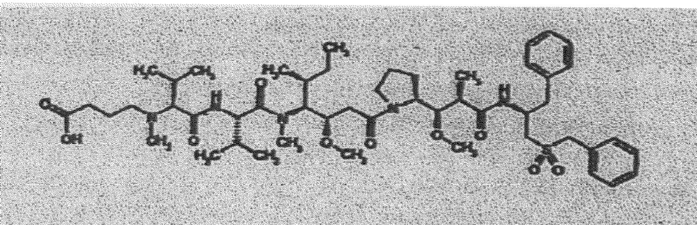 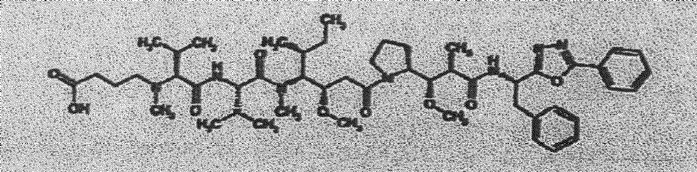 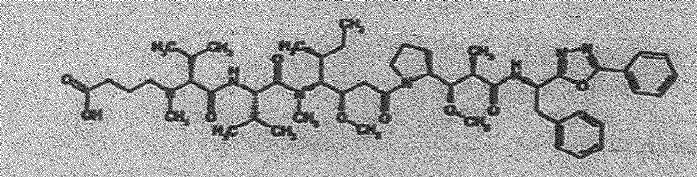 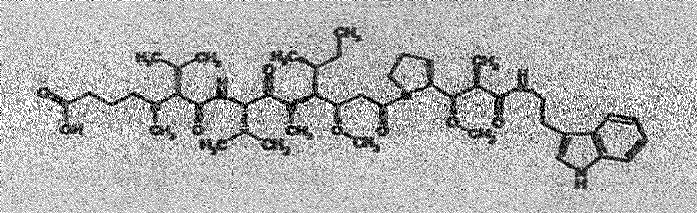 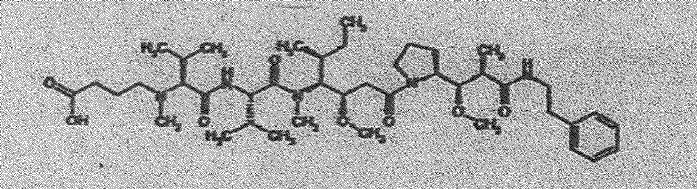 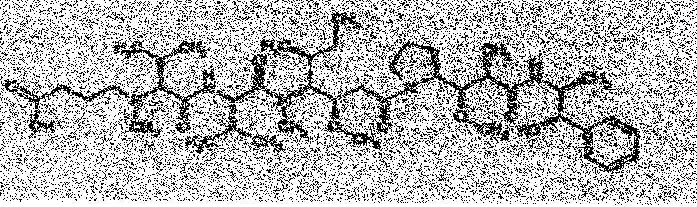 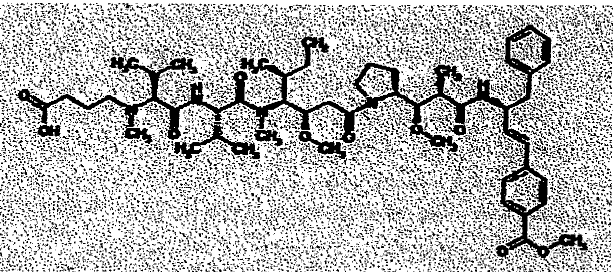 and 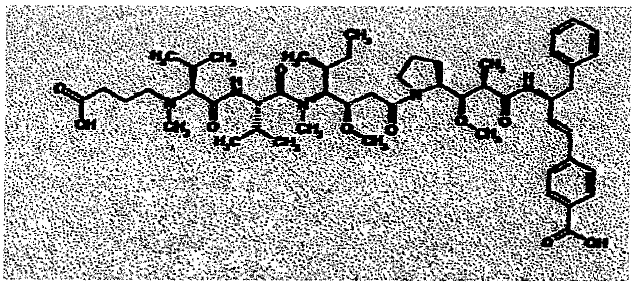 as well as its salts, solvates and solvates of the salts.

5. Method for producing a compound of the formula (I), as defined in claims 1 to 4, **characterised in that** a compound of the formula (II) 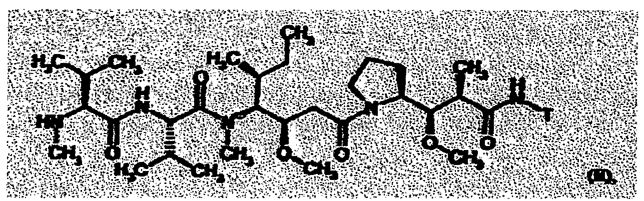 in which T has the meanings indicated in claims 1 to 3, is reacted in an inert solvent either
[A] by base-induced alkylation with a compound of the formula (III) 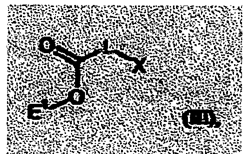 in which L has the meaning indicated in claims 1 to 3,
E¹ represents hydrogen, (C₁-C₄) alkyl or benzyl,
and
X represents a volatile group, such as for example chloride, bromide, iodide, mesylate, triflate or tosylate,
to form a compound of the formula (IV) 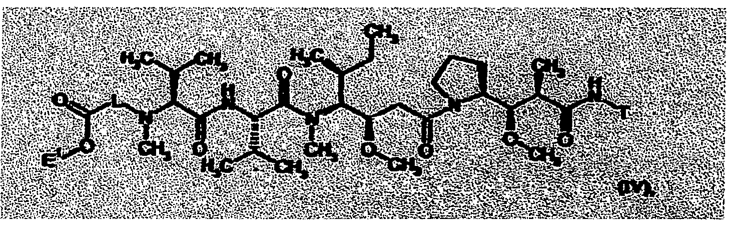 in which E¹, L and T have the meanings indicated above,
and then in the case that E¹ represents (C₁-C₄) alkyl or benzyl, this ester radical is cleaved according to conventional methods so that just as in the case that E¹ in (III) represents hydrogen, the carboxylic acid of the invention of formula (I) 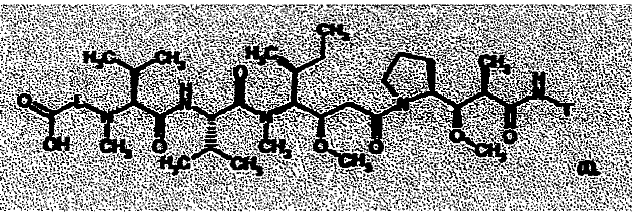 in which L and T have the meanings indicated above, is obtained
or
[B] is converted by reacting with a compound of the formula (V) 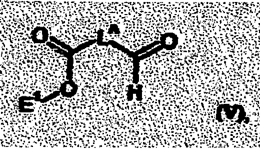 in which
E¹ represents hydrogen, (C₁-C₄) alkyl or benzyl,
and
L^{A} has the meaning of L defined in claims 1 to 3, but is shortened in alkyl chain length by one CH₂ unit,
in the presence of a suitable reducing agent to a compound of the formula (VI) 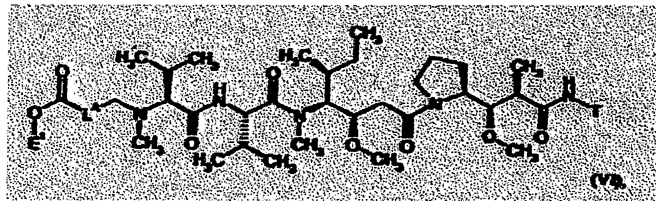 in which E¹, L^{A} and T have the meanings indicated above,
and then in the case that E¹ represents (C₁-C₄) alkyl or benzyl, this ester radical is cleaved according to conventional methods so that just as in the case that E¹ in (V) represents hydrogen, the carboxylic acid of the invention of formula (I-A) 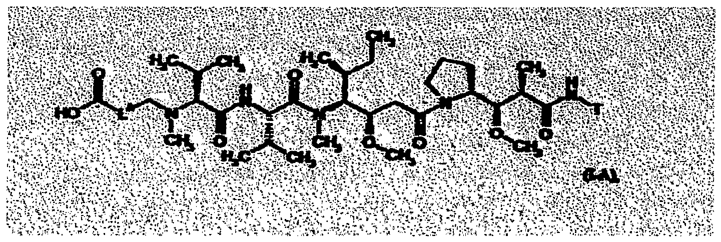 in which L^{A} and T have the meanings indicated above, is obtained,
and the resulting compounds of formula (I) or (I-A) are separated optionally into their enantiomers and/or diastereomers and/or reacted with the corresponding (*i*) solvents and/or (*i*) bases or acids to form their solvates, salts and/or solvates of the salts.

6. Compound, as defined in one of claims 1 to 4, for use for treating and/or preventing diseases.

7. Compound, as defined in one of claims 1 to 4, for use in a method for treating and/or preventing carcinosis and oncosis.

8. Medicament containing a compound, as defined in one of claims 1 to 4, in combination with one or more inert, non-toxic, pharmaceutically suitable adjuvants.

9. Medicament containing a compound, as defined in one of claims 1 to 4, in combination with one or more further active ingredients.

10. Medicament according to claim 8 or 9 for use for treating and/or preventing carcinosis and oncosis.

11. Antiproliferative conjugate in which a compound from one or more of claims 1-4 is linked to a protein.

12. Antiproliferative conjugate according to claim 12, wherein the protein is an antibody.

## Revendications

1. Composé de formule (I) 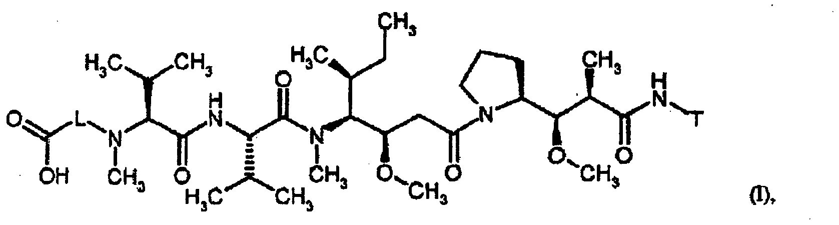 dans laquelle
L représente un alcane (en C₁-C₁₂)-diyle linéaire qui peut être substitué jusqu'à quatre fois par du méthyle et dans lequel (a) deux atomes de carbone en position 1,2, 1,3 ou 1,4 l'un par rapport à l'autre, en incluant les atomes de carbone le cas échéant intercalés peuvent être reliés à un cycle cycloalkyle (en C₃-C₆) ou un cycle phényle pour former un pont ou (*b*) jusqu'à trois groupes CH₂ non adjacents peuvent être remplacés par -O-,
et
T représente un groupe de formule 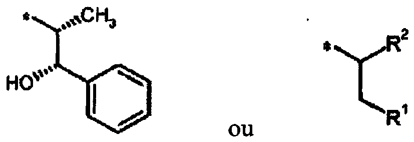 dans laquelle
* caractérise le site de liaison à l'atome d'azote,
R¹ représente le groupe phényle ou le 1*H*-indol-3-yle,
et
R² représente l'hydrogène ou un groupe de formule 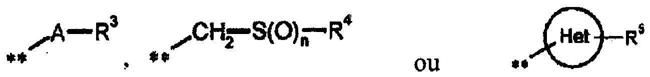 dans laquelle
** caractérise le site de liaison respectif avec le reste du groupe T concerné,
A représente l'alcane(en C₁-C₄)diyle linéaire ou l'alcène(en C₂-C₄)diyle linéaire,
R³ représente le phényle qui peut être substitué par un groupe alcoxy(en C₁-C₄)carbonyle ou carboxyle,
n représente le nombre 0, 1 ou 2,
R⁴ représente le groupe phényle, benzyle ou 2-phényl-éthyle, qui peuvent être substitués respectivement dans le groupe phényle par un groupe alcoxy(en C₁-C₄)carbonyle ou carboxyle,
Het représente un cycle hétéroaryle divalent à 5 membres avec jusqu'à trois hétéroatomes du cycle provenant de la série N, O et/ou S,
et
R⁵ représente un groupe cycloalkyle (en C₃-C₆), phényle ou alkyle (en C₁-C₄), qui peut être substitué par un phényle,
les groupes phényles cités pouvant être à leur tour substitués par un groupe alcoxy(en C₁-C₄)carbonyle ou carboxyle,
et leurs sels, solvates et solvates de leurs sels.

2. Composé de formule (I) selon la revendication 1, dans laquelle
L représente un alcane(en C₁-C₈)-diyle linéaire dans lequel (*a*) deux atomes de carbone en position 1,3 ou 1,4 l'un par rapport à l'autre, en incluant l'un ou les deux atomes de carbone intercalés, peuvent être reliés un cycle phényle pour former un pont ou (*b*) jusqu'à deux groupes CH₂ non adjacents peuvent être remplacés par -O-,
et
T représente un groupe de formule 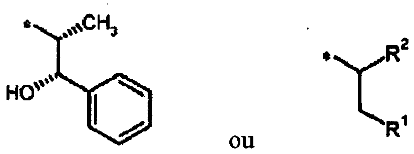 dans laquelle
* caractérise le site de liaison à l'atome d'azote,
R¹ représente le groupe phényle ou le 1*H*-indol-3-yle,
et
R² représente l'hydrogène ou un groupe de formule 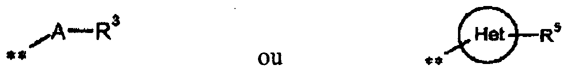 dans laquelle
** caractérise le site de liaison avec le reste du groupe T concerné,
A représente l'éthène-1,2-diyle ou le propène-1,3-diyle,
R³ représente le phényle qui peut être substitué par un groupe alcoxy(en C₁-C₄)carbonyle ou carboxyle,
Het représente un cycle hétéroaryle divalent à 5 membres choisi dans la série constituée par le pyrazolyle, l'imidazolyle, le 1,3-oxazolyle, le 1,3-thiazolyle, le 1,2,4-oxadiazolyle et le 1,3,4-oxadiazolyle,
et
R⁵ représente un groupe phényle, qui peut être substitué par un groupe alcoxy(en C₁-C₄)carbonyle ou carboxyle,
et leurs sels, solvates et solvates de leurs sels.

3. Composé de formule (I) selon la revendication 1 ou 2, dans laquelle
L représente un alcane(en C₁-C₆)-diyle linéaire,
et
T représente un groupe de formule 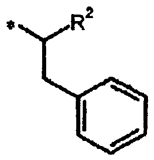 dans laquelle
* caractérise le site de liaison à l'atome d'azote,
et
R² représente l'hydrogène ou un groupe de formule 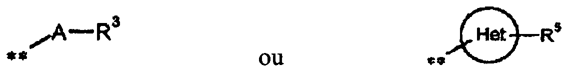 dans laquelle
** caractérise le site de liaison avec le reste du groupe T concerné,
A représente l'éthène-1,2-diyle,
R³ représente le phényle qui peut être substitué par un groupe méthoxycarbonyle ou carboxyle,
Het représente le 1,3,4-oxadiazol-2,5-diyle,
et
R⁵ représente un groupe phényle, qui peut être substitué par un groupe méthoxycarbonyle ou carboxyle,
et leurs sels, solvates et solvates de leurs sels.

4. Composé selon la revendication 1, dans lequel le composé est choisi dans le groupe constitué par : 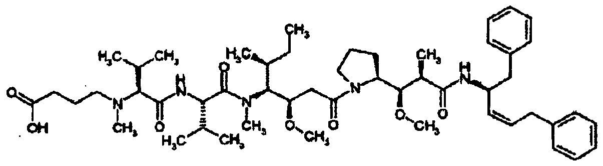 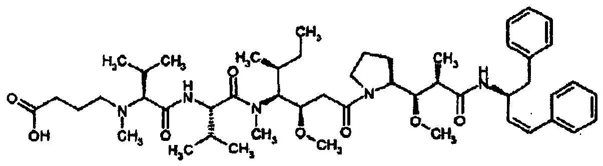 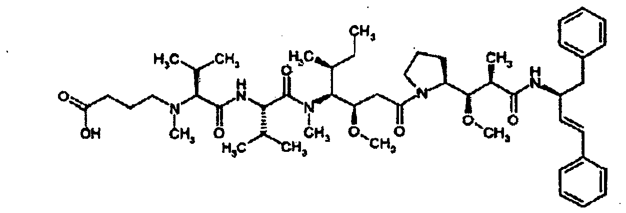 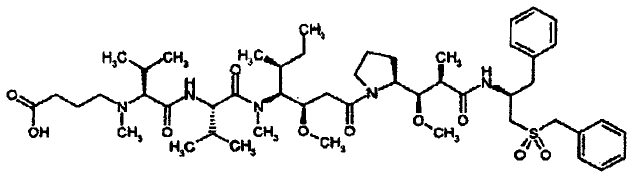 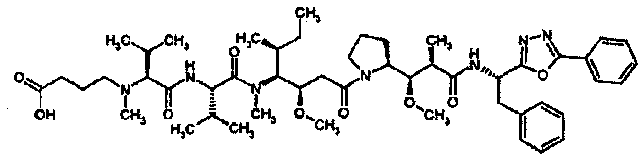 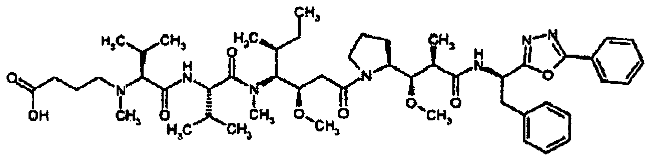 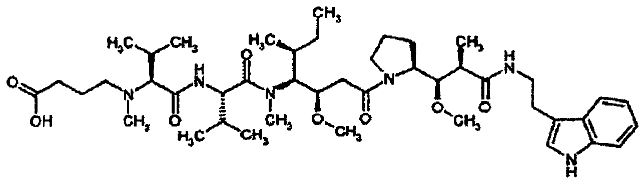 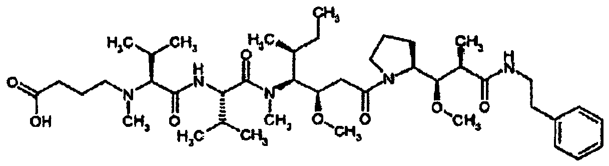 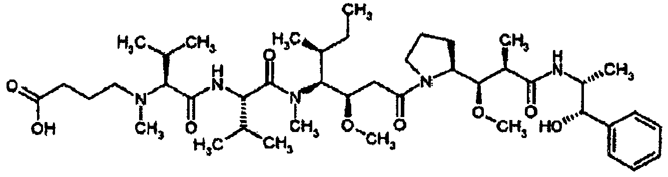 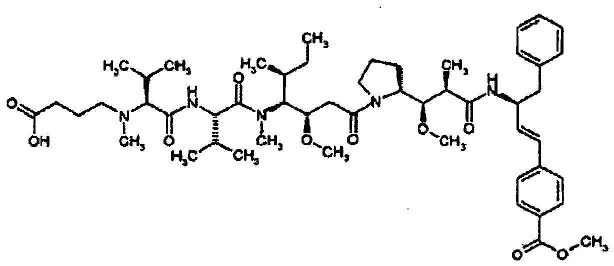 et 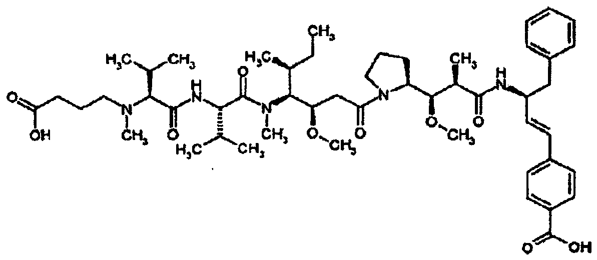 et leurs sels, solvates et solvates de leurs sels.

5. Procédé de préparation d'un composé de formule (I), tel que défini dans les revendications 1 à 4, **caractérisé en ce que** l'on fait réagir un composé de formule (II) 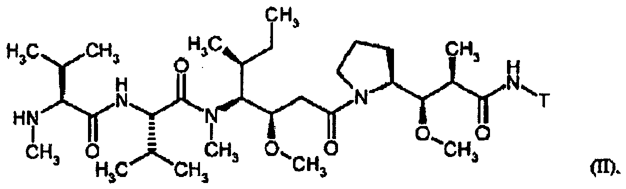 dans laquelle T a les significations indiquées dans les revendications 1 à 3, dans un solvant inerte soit
[A] par alkylation induite par des bases avec un composé de formule (III) 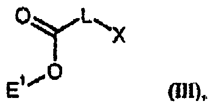 dans laquelle L a la signification indiquée dans les revendications 1 à 3,
E¹ représente l'hydrogène, un groupe alkyle (en C₁-C₄) ou benzyle, et
X représente un groupe sortant comme par exemple le chlorure, le bromure, l'iodure, le mésylate, le triflate ou le tosylate,
pour donner un composé de formule (IV) 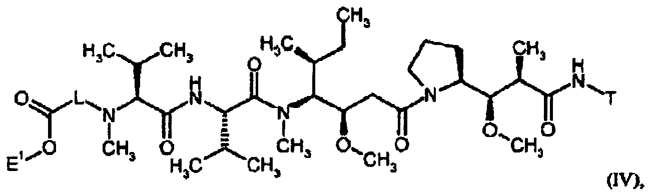 dans laquelle E¹, L et T ont les significations indiquées ci-dessus,
et ensuite dans le cas où E¹ représente un groupe alkyle (en C₁-C₄) ou benzyle, on clive ce radical ester selon les méthodes usuelles afin d'obtenir, comme dans le cas où E¹ représente l'hydrogène dans la formule (III), l'acide carboxylique selon l'invention de formule (I) 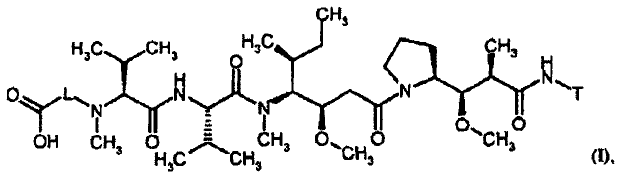 dans laquelle L et T ont les significations indiquées ci-dessus,
soit
[B] par conversion avec un composé de formule (V) 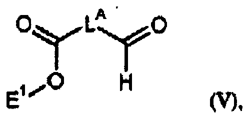 dans laquelle
E¹ représente l'hydrogène, un groupe alkyle (en C₁-C₄) ou benzyle, et
L^{A} a la signification de L définie dans les revendications 1 à 3, mais cependant réduite d'une unité CH₂ dans la longueur de la chaîne alkyle,
en présence d'un agent réducteur approprié pour donner un composé de formule (VI) 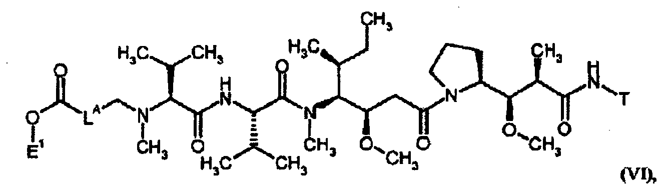 dans laquelle E¹, L^{A} et T ont les significations indiquées ci-dessus,
et ensuite dans le cas où E¹ représente un groupe alkyle (en C₁-C₄) ou benzyle, on clive ce radical ester selon les méthodes usuelles afin d'obtenir, comme dans le cas où E¹ représente l'hydrogène dans la formule (V), l'acide carboxylique selon l'invention de formule (I-A) 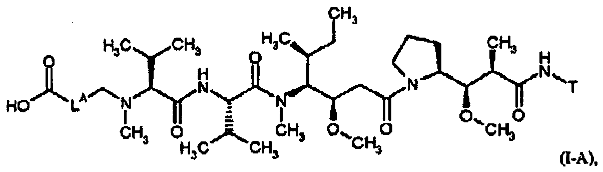 dans laquelle L^{A} et T ont les significations indiquées ci-dessus,
et on sépare les composés résultants de formule (I) ou (I-A) le cas échéant pour donner leurs énantiomères et/ou diastéréomères et/ou on les fait réagir avec leurs (i) solvants et/ou (i) bases ou acides correspondants pour donner leurs solvates, sels et/ou solvates de leurs sels.

6. Composé tel que défini dans l'une des revendications 1 à 4 pour l'utilisation dans le traitement et/ou la prévention des maladies.

7. Composé tel que défini dans l'une des revendications 1 à 4 pour l'utilisation dans un procédé de traitement et/ou de prévention des affections cancéreuses et tumorales.

8. Médicament contenant un composé tel que défini dans l'une des revendications 1 à 4, en combinaison avec un ou plusieurs auxiliaires inertes, non toxiques, pharmaceutiquement appropriés.

9. Médicament contenant un composé tel que défini dans l'une des revendications 1 à 4, en combinaison avec une ou plusieurs autres substances actives.

10. Médicament selon la revendication 8 ou 9 pour l'utilisation dans le traitement et/ou la prévention des affections cancéreuses et tumorales.

11. Conjugué antiprolifératif, dans lequel un composé selon une ou plusieurs des revendications 1 à 4 est lié à une protéine.

12. Conjugué antiprolifératif selon la revendication 12, dans lequel la protéine est un anticorps.
